# EUROPEAN PATENT APPLICATION

(11) **EP 3 540 580 A1**
(43) Date of publication of application: **18.09.2019**
(21) Application number: 17870576.0
(22) Date of filing: 21.08.2017
(51) Int. Cl.: G06F 3/0482, G06F 3/01, G06F 3/0485

(54) **INFORMATION PROCESSING DEVICE, INFORMATION PROCESSING METHOD, AND PROGRAM**

(30) Priority: 08.11.2016 JP 2016217996
(71) Applicant: Sony Corporation, Tokyo 108-0075 (JP)
(72) Inventor: MA, Chao, Tokyo 108-0075 (JP); OKADA, Kazuhira, Tokyo 108-0075 (JP); ENDO, Makoto, Tokyo 108-0075 (JP)
(74) Representative: D Young & Co LLP
(86) International application number: PCT/JP2017/029746
(87) International publication number: WO 2018/087977

(57) **Abstract**

Provided is a user interface suitable for, for example, a non-contact operation or the like. First, an operation direction and an operation distance are calculated from acquired operation information. In addition, an operation space is set in which regions of items to be selected are arranged around an origin position. Then, in a case where a positional relationship between a region of an item and an operation position specified by the operation direction and the operation distance from the origin position as a starting point is changed to a specific state, a process of determining that the item has been selected is performed. In addition, display data corresponding to the determination result is generated such that the user recognizes an operation situation.

## Description

### TECHNICAL FIELD

The present technology relates to an information processing device, an information processing method, and a program and specifically relates to a user interface technology.

### BACKGROUND ART

A non-contact operation has been known which inputs various kinds of operation using the movement of the body of a user or an input device possessed by the user in a space.

The following Patent Document 1 discloses a technique related to a non-contact operation and discloses, for example, a method which recognizes a circular motion.

### CITATION LIST

### PATENT DOCUMENT

Patent Document 1: Japanese Patent Application Laid-Open No. 2013-196391

### SUMMARY OF THE INVENTION

### PROBLEMS TO BE SOLVED BY THE INVENTION

An aspect of a non-contact operation is that, in a case where a user moves a hand or an input device in a space and a specific motion is detected, an electronic device detects the motion and recognizes the motion as a specific operation.

In addition, there is a method in which the movement of the hand of the user or an input device is reflected on a screen and the user moves the hand or the input device in a space while viewing an operation image appearing on a display screen to select a desired item (for example, an operation item on the screen).

However, there is a problem that, in general, there are many uncertain factors in a motion in a space, which makes it difficult to perform a stable operation.

Movement for an operation is reflected as, for example, the movement of a cursor on a screen, which serves as a guide to the operation of the user. However, this is insufficient to stabilize an operability.

For example, even in a case where a cursor corresponding to the movement of the hand or the input device indicates an item on the screen, it is difficult to perform an enter operation, for example, an operation corresponding to a click or the like in this state.

For example, it is considered that, in a state in which a component of two-dimensional (x-y plane direction) movement in a space is reflected in the movement of a cursor on a screen plane, movement in a depth direction (z direction) is set as an enter operation. In general, in the case of movement in a space, a component of a plane direction is also added at the time of movement in the depth direction. Therefore, a position designated by the cursor on a screen deviates, which may cause an erroneous operation. Of course, other motions for an enter operation are also considered. However, in this case, the efficiency of the operation is not very high.

Accordingly, an object of the present disclosure is to provide a user interface that enables a user to stably and easily perform operation input even in a non-contact operation for unstable movement in a space.

### SOLUTIONS TO PROBLEMS

An information processing device according to the present technology includes: an operation detection unit that calculates an operation direction and an operation distance from acquired operation information; a control unit that, in a case where a positional relationship between a region of an item to be selected and an operation position specified by the operation direction and the operation distance calculated by the operation detection unit using an origin position as a starting point is changed to a specific state in a state in which the regions of the items to be selected are arranged around the origin position, performs a process of determining that the item has been selected; and a display data generation unit that generates display data corresponding to a determination result of the control unit.

In the case of this configuration, an operation direction and an operation distance for an operation (non-contact operation) of the user is reflected as a direction and a distance from the origin as a starting point in a virtual operation space. That is, the direction and the distance indicated by the operation of the user are recognized as a direction and a distance from the origin position in the virtual operation space. In the operation space, the items to be selected are arranged around the origin position. Then, in a case where the positional relationship between a region of an item and the operation position specified by the operation direction and the operation distance from the origin position is changed to a specific state indicating a certain item (for example, the operation position reaches or passes through the item), it is recognized that the item has been selected.

In the above-described information processing device according to the present technology, it is considered that the operation detection unit detects non-contact operation information as the operation information and the control unit sets the origin position substantially at a center of an operation region in which the non-contact operation information is effective.

Since the origin position is set at the center of the operation region, the items to be selected are arranged in a circular ring having the center of the operation region as its center.

In the above-described information processing device according to the present technology, it is considered that the display data generation unit generates display data for displaying the operation direction and the operation distance calculated by the operation detection unit on an operation plane.

Not only the origin region and the selected item but also the current operation position determined by the operation direction and the operation distance is displayed on the operation plane displayed on a display screen. For example, the operation position is expressed by an image or the like of an arrow or the like extending from the origin.

In the above-described information processing device according to the present technology, it is considered that the control unit sets an operation plane in which a plurality of items to be selected are arranged around the origin position so as not to overlap each other as viewed from the origin position.

For example, a plurality of items are arranged around the origin position in a circular ring shape or the like. The items are arranged so as not to overlap each other as viewed from the origin position. Therefore, the operation position specified by the operation direction and the operation distance from the origin position as a starting point reaches any item to select the item.

In the above-described information processing device according to the present technology, the following is considered: in a case where the control unit recognizes that a certain item has been selected by an operation position on the operation plane and a lower-layer item is present in the selected item, the control unit resets the operation plane such that the selected item is arranged at the origin position and the lower-layer items are arranged around the origin position so as not to overlap each other as viewed from the origin position; and, in a case where the operation position indicates a certain lower-layer item on the reset operation plane, the control unit performs a process of recognizing that the lower-layer item has been selected.

In a case where the items are arranged around the origin position so as not to overlap each other as viewed from the origin position, each item can be selected by the operation position specified by the operation direction and the operation distance from the origin position as a starting point. In addition, it is possible to perform a similar operation to that in a case where a lower-layer item is present in the selected item. Therefore, the operation plane is switched to an operation plane in which the selected item is arranged at (moved to) the origin position and the lower-layer items are arranged around the origin position. That is, the starting point of the operation regresses to the center.

In the above-described information processing device according to the present technology, it is considered that, in a case where the operation plane is set or reset, the control unit arranges a plurality of items around the origin position along a virtual ring of a true circle having the origin position as a center.

For example, a virtual ring of a true circle having the origin as the center is considered around the origin position and items are arranged along the ring.

In the above-described information processing device according to the present technology, it is considered that, in a case where the operation plane is reset, the control unit controls the display data generation unit such that display presenting movement of the selected item to the origin position is performed.

That is, an aspect of the regression of the selected item to the origin position is presentedto the user on a display.

In the above-described information processing device according to the present technology, it is considered that, in a case where the operation plane is reset, the control unit arranges the lower-layer items and an item for a layer return operation around the origin position so as not to overlap each other as viewed from the origin position.

Since the lower-layer items and the item for returning the layer are arranged around the selected item, it is possible to select an operation of going back to the layer.

In the above-described information processing device according to the present technology, the following is considered: in a case where the item for the layer return operation is selected, the control unit controls the display data generation unit such that display presenting movement of the item for the layer return operation to the origin position is performed; and the control unit resets the operation plane such that upper-layer items are arranged around the origin position so as not to overlap each other as viewed from the origin position.

That is, in a case where an item for a "return" operation is selected, an aspect of the regression of the item for the "return" operation to the origin position is presented to the user on the display and then the upper-layer items are arranged and displayed.

In the above-described information processing device according to the present technology, the following is considered: the control unit sets, as an operation plane for performing an operation involving a plurality of stages for selection or designation of an item, an operation plane in which a plurality of items to be selected in an X-th stage (where X is a natural number equal to or greater than 1) are arranged on an X-th virtual ring-shaped line from the origin position so as not to overlap each other as viewed from the origin position; and, in a case where the control unit recognizes that a certain item among the plurality of items in the X-th stage has been selected by an operation position, the control unit sets an operation plane in which one or a plurality of items to be designated or selected according to the item selected in the X-th stage are arranged on an (X+1)-th virtual ring-shaped line from the origin position.

For example, a plurality of items are arranged around the origin position in a circular ring. However, in a case where a certain item is selected, items to be selected or designated according to the selected item are arranged on the outer circumferential side of the selected item. For example, lower-layer items or an items for an enter operation are arranged.

In the above-described information processing device according to the present technology, the following is considered: in a case where the control unit recognizes that a certain item among the plurality of items in the X-th stage has been selected by the operation position, the control unit sets an operation plane in which a plurality of lower-layer items to be selected in a lower layer of the item selected in the X-th stage are arranged on the (X+1)-th virtual ring-shaped line from the origin position; and, in a case where the operation position indicates a certain lower-layer item, the control unit performs a process of recognizing that the lower-layer item has been selected.

That is, for example, a plurality of virtual ring-shaped lines having the origin position as the center are considered and the operation plane in which the lower-layer items are presented on the outer circumferential side is formed. With this configuration, a lower-layer item can be selected following the selection of an upper-layer item by the extension of the operation position determined by the operation direction and the operation distance.

In the above-described information processing device according to the present technology, the following is considered: in a case where the control unit recognizes that a certain item among the plurality of items in the X-th stage has been selected by the operation position, the control unit sets an operation plane in which an item for an enter operation for the item selected in the X-th stage is arranged on the (X+1)-th virtual ring-shaped line from the origin position; and, in a case where the operation position designates the item for the enter operation, the control unit performs a process of recognizing that the enter operation for the item selected in the X-th stage has been performed.

That is, even in a case where operations are performed in stages, for example, an enter operation is performed after a selection operation, an item for the enter operation is arranged on the outer circumferential side of a plurality of items arranged on a virtual ring-shaped line. Therefore, the enter operation can be performed following the selection of an item by the extension of the operation position determined by the operation direction and the operation distance.

In the above-described information processing device according to the present technology, it is considered that the control unit arranges the item for the enter operation at a position that is on the (X+1) -th virtual ring-shaped line and is adjacent to the item selected in the X-th stage.

That is, the item for the enter operation is arranged adjacent to the immediately outer circumferential side of the selected item.

In the above-described information processing device according to the present technology, the following is considered: the control unit sets the plurality of items to be selected in the X-th stage as items for a scroll operation; and, in a case where an item for the scroll operation is selected, the control unit performs a process of recognizing the scroll operation.

That is, a scroll operation can be performed by the items arranged in a ring shape.

In the above-described information processing device according to the present technology, it is considered that the control unit performs a process of recognizing a direction of the scroll operation according to an order in which a plurality of items for the scroll operation are selected.

That is, an operation of sequentially selecting a plurality of items arranged in a ring shape indicates the direction of the scroll operation.

In the above-described information processing device according to the present technology, it is considered that the control unit arranges an item for an enter operation after scrolling on the (X+1) -th virtual ring-shaped line in the same angular range as an item that is being selected among the items for the scroll operation.

With this configuration, the item for the enter operation is arranged on the outer circumferential side of the scroll item that is being selected and is displayed. In a case where the scroll items are sequentially selected as the scroll operation, the position of the item for the enter operation on the outer circumferential side is changed following the selection. That is, in a case where the item that is being selected is sequentially changed, the item for the enter operation is sequentially displayed at different outer circumferential positions.

In the above-described information processing device according to the present technology, it is considered that, in a case where the control unit recognizes that a certain item among the plurality of items in the X-th stage has been selected by the operation position, the control unit instructs the display data generation unit such that a display aspect of items which have not been selected among the plurality of items in the X-th stage is changed.

In a case where one of the items in the X-th stage is selected, the display aspect of the items which have not been selected on the display is changed. For example, the items are displayed in an inactive state or are not displayed.

In the above-described information processing device according to the present technology, the following is considered: the control unit sets an operation plane in which one or a plurality of items to be designated or selected in an (X+1) -th stage according to the item selected in the X-th stage are arranged; and, in a case where selection of the selected item is canceled, the control unit sets the operation plane such that the items in the (X+1)-th stage are canceled.

In a case where the selected state of the items in the X-th stage is canceled, the menu returns to the operation of selecting the items in the X-th stage. Therefore, the setting of the items in the (X+1)-th stage on the operation plane is canceled and the items are not displayed.

An information processing method according to the present technology is performed by an information processing device and includes : an operation detection step of calculating an operation direction and an operation distance from acquired operation information; a determination step of, in a case where a positional relationship between a region of an item to be selected and an operation position specified by the operation direction and the operation distance calculated in the operation detection step using an origin position as a starting point is changed to a specific state in a state in which the regions of the items to be selected are arranged around the origin position, determining that the item has been selected; and a display data generation step of generating display data corresponding to a determination result in the determination step.

A program according to the present technology causes an information processing device to perform processing steps corresponding to these procedures.

A user interface suitable for an operation, such as a non-contact operation, is achieved by the method or the program.

### EFFECTS OF THE INVENTION

According to the present technology, it is possible to provide a user interface that enables a user to easily and stably perform an operation such as a non-contact operation.

Note that the effects described herein are not necessarily limited and may be any of the effects described in the present disclosure.

### BRIEF DESCRIPTION OF DRAWINGS

Fig. 1 is a diagram illustrating a functional configuration of an information processing device according to an embodiment of the present technology.
Fig. 2 is a diagram illustrating a conversion relationship between an output area and an effective range of an input device according to the embodiment.
Fig. 3 is a diagram illustrating an operation plane according to the embodiment.
Fig. 4 is a diagram illustrating an operation region of a center-regression-type menu according to the embodiment.
Fig. 5 is a diagram illustrating an operation region of a pie-type menu according to the embodiment.
Fig. 6 is a block diagram illustrating a hardware configuration of the information processing device according to the embodiment.
Fig. 7 is a diagram illustrating a menu mode start operation according to the embodiment.
Fig. 8 is a diagram illustrating the display and operation of the center-regression-type menu according to the embodiment.
Fig. 9 is a diagram illustrating the display and operation of the center-regression-type menu according to the embodiment.
Fig. 10 is a diagram illustrating the display and operation of the center-regression-type menu according to the embodiment.
Fig. 11 is a flowchart illustrating a process for the center-regression-type menu according to the embodiment.
Fig. 12 is a diagram illustrating the display transition of the center-regression-type menu according to the embodiment.
Fig. 13 is a diagram illustrating the display and operation of the pie-type menu according to the embodiment.
Fig. 14 is a diagram illustrating the display and operation of the pie-type menu according to the embodiment.
Fig. 15 is a diagram illustrating the display and operation of the pie-type menu according to the embodiment.
Fig. 16 is a flowchart illustrating a first example of a process for the pie-type menu according to the embodiment.
Fig. 17 is a diagram illustrating display transition in the first example of the process for the pie-type menu according to the embodiment.
Fig. 18 is a flowchart illustrating a second example of the process for the pie-type menu according to the embodiment.
Fig. 19 is a diagram illustrating display transition in the second example of the process for the pie-type menu according to the embodiment.
Fig. 20 is a flowchart illustrating a third example of the process for the pie-type menu according to the embodiment.
Fig. 21 is a diagram illustrating display transition in the third example of the process for the pie-type menu according to the embodiment.
Fig. 22 is a flowchart illustrating a fourth example of the process for the pie-type menu according to the embodiment.
Fig. 23 is a diagram schematically illustrating the overall configuration of an operating room system.
Fig. 24 is a diagram illustrating an example of the display of an operation screen in a centralized operation panel.
Fig. 25 is a diagram illustrating an example of an aspect of an operation to which the operating room system is applied.
Fig. 26 is a block diagram illustrating an example of the functional configuration of a camera head and a CCU illustrated in Fig. 25.
Fig. 27 is a diagram illustrating an example of the application of the menu type according to the embodiment to monitor control.
Fig. 28 is a diagram illustrating an example of the application of the menu type according to the embodiment to pan/tilt control.
Fig. 29 is a diagram illustrating an example of the application of the menu type according to the embodiment to focus/zoom control.
Fig. 30 is a diagram illustrating an example of the application of the menu type according to the embodiment to 2D/3D control.
Fig. 31 is a diagram illustrating an example of the application of the menu type according to the embodiment to swap/contrast control.
Fig. 32 is a diagram illustrating an example of the application of the menu type according to the embodiment to color control.

### MODE FOR CARRYING OUT THE INVENTION

Hereinafter, preferred embodiments of the present disclosure will be described in detail with reference to the accompanying drawings. In addition, in the specification and the drawings, components having substantially the same functional configurations are denoted by the same reference numerals and the description thereof will not be repeated.

The description will be made in the following order.
<1. Configuration for Recognizing Operation of Information processing device>
<2. Center-regression-type Menu>
<3. First Example of Pie-type Menu>
<4. Second Example of Pie-type Menu>
<5. Third Example of Pie-type Menu>
<6. Fourth Example of Pie-type Menu>
<7. Example of Application to Operating Room System>
<8. Summary and Modification Examples>

### <1. Configuration for Recognizing Operation of Information processing device>

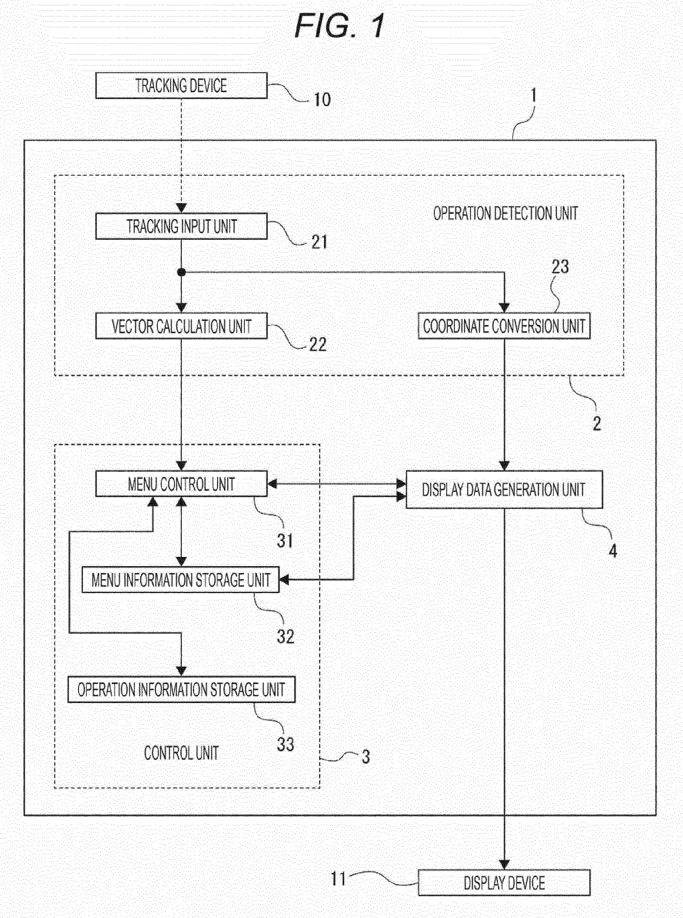

Fig. 1 illustrates the functional configuration of an information processing device 1 according to the present disclosure. The information processing device 1 includes an operation detection unit 2, a control unit 3, and a display data generation unit 4.

The information processing device 1 receives non-contact operation information of a user from a tracking device 10 and displays an operation screen on a screen of a display device 11 to achieve a user interface.

The operation detection unit 2 mainly performs a process that acquires the non-contact operation information of the user detected by the tracking device 10 and calculates an operation direction and an operation distance from the acquired non-contact operation information. For this process, the operation detection unit 2 includes a tracking input unit 21, a vector calculation unit 22, and a coordinate conversion unit 23.

The control unit 3 mainly performs the following process. The control unit 3 sets an operation plane in which a plurality of items to be selected are arranged around an origin position. In a state in which an operation position specified by the operation direction and the operation distance recognized by the operation detection unit using the origin position as a starting point on the operation plane indicates a certain item, the control unit 3 recognizes that the item has been selected. For this process, the control unit 3 includes a menu control unit 31, a menu information storage unit 32, and an operation information storage unit 33.

The display data generation unit 4 performs a process of generating display data for displaying the operation plane set by the control unit 3 on the display device 11.

These functions will be described below.

First, the tracking input unit 21 of the operation detection unit 2 inputs the positional information of the hands of the user or an operation body, such as any input device, in a three-dimensional space which is obtained from the tracking device 10.

It is considered that the tracking device 10 which inputs non-contact operation information to the information processing device 1 (tracking input unit 21) is, specifically, an image recognition device.

For example, an imaging device including an image sensor, such as a charge coupled device (CCD) or a complementary metal oxide semiconductor (CMOS), captures an image of the space in which the user performs a non-contact operation and acquires the image. Then, the imaging device analyzes the image, extracts an image of a portion (the hand or the input device) corresponding to the operating body, and acquires a position (coordinate values in the x, y, and z directions) in an operation space including a depth from the position and size of the extracted image portion.

Alternatively, a method is considered in which the tracking device 10 irradiates the space in which the user performs an operation with infrared light, detects light reflected from an operation body, such as the hand of the user, and determines the positional information of the operation body.

In addition, it is assumed that, in a case where an input device as an operation body is a device including an angular velocity sensor, the tracking device 10 receives angular velocity information from the angular velocity sensor and determines the positional displacement of the operation body.

Furthermore, there are various other detection methods. Any of the detection methods may be used as long as the tracking device 10 can detect three-dimensional information indicating the position of the operation body using the movement of the user in the space.

The tracking input unit 21 acquires the three-dimensional positional information of the operation body from the tracking device 10 at a predetermined time interval. For example, in a case where the tracking device 10 is an imaging device that performs imaging and image analysis at a frame rate of 30 fps, it is possible to acquire three-dimensional positional information at a maximum time interval of 1/30 seconds.

The three-dimensional positional information (coordinate values in the x, y, and z directions) is the non-contact operation information of the user.

The vector calculation unit 22 calculates a vector indicating the displacement of movement as a non-contact operation from the three-dimensional positional information sequentially acquired by the tracking input unit 21. That is, the vector calculation unit 22 calculates an operation direction and an operation distance from the difference between two three-dimensional positional information items at different points of time.

In addition, in this embodiment, a center-regression-type menu and a pie-type menu are given as examples of a graphic user interface type that presents an operation menu. A two-dimensional vector is mainly required during presentation. Therefore, for the operation direction and the operation distance, a two-dimensional vector may be calculated from the x and y coordinate values in each of the three-dimensional positional information items at two points of time.

However, in the embodiment, an example in which an operation in the depth direction is also detected for the start/end of a menu mode is given. This means that an operation in the z direction is separately calculated.

Then, the vector calculation unit 22 sequentially outputs vector information (an operation direction and an operation distance) on the x and y coordinates and vector information on the z coordinate to the menu control unit 31 of the control unit 3.

The coordinate conversion unit 23 converts a two-dimensional coordinate value in the x and y directions in the three-dimensional positional information sequentially acquired by the tracking input unit 21 in order to display the user interface.

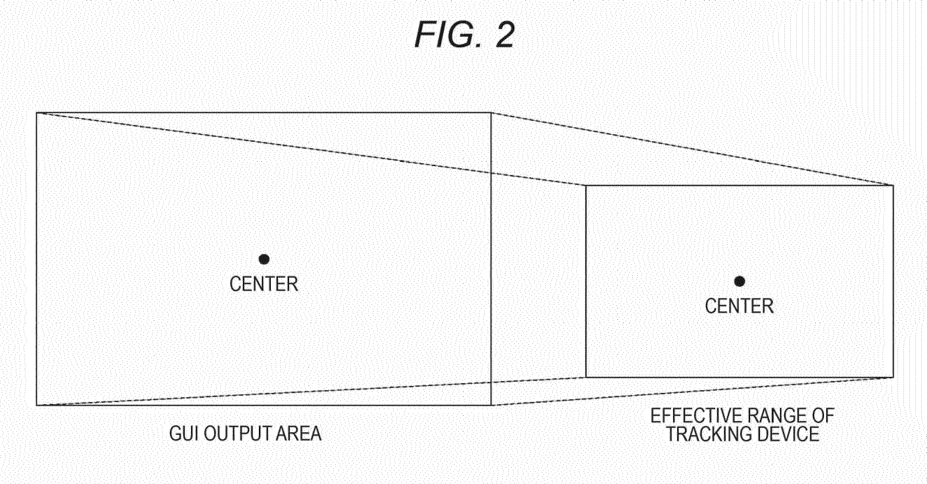

Fig. 2 illustrates an example of a graphic user interface (GUI) output area and an effective range for detecting a non-contact operation in the tracking device 10.

The effective range of the tracking device 10 is not matched with the GUI output area. Therefore, as illustrated in Fig. 2, a two-dimensional coordinate value (the x and y coordinate values in the effective range of the tracking device 10) in the three-dimensional positional information sequentially acquired by the tracking input unit 21 is converted into x and y coordinate values on the plane of an output area for display output as the GUI such that the GUI output area corresponds to a portion or the whole of the effective range of the tracking device 10. Then, the coordinate conversion unit 23 outputs the converted x and y coordinate values as the information of an operation position on an operation screen for displaying the converted x and y coordinate values to the display data generation unit 4. The information is used by the display data generation unit 4 to draw an operation vector Vc which will be described below.

The menu control unit 31 of the control unit 3 sets an operation plane in which items to be selected are arranged, provides the operation plane as information for the user interface to the display data generation unit 4, and recognizes an operation state on the operation plane in response to the detection of the information of the operation direction and the operation distance supplied from the vector calculation unit 22 as described above.

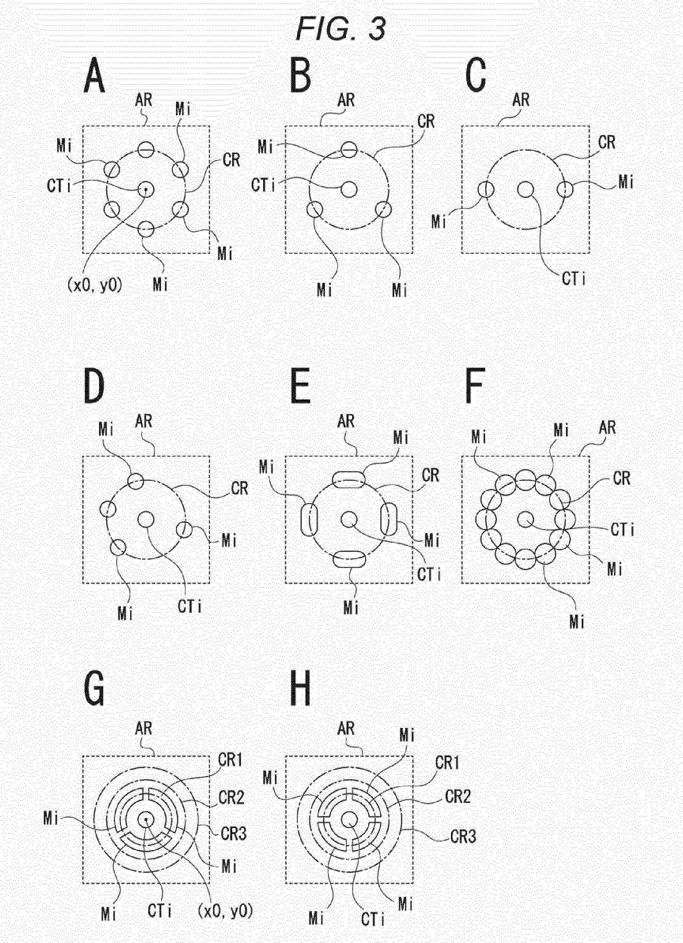

Here, an example of the operation plane will be described with reference to Fig. 3.

For example, Fig. 3A illustrates an operation plane in which items Mi forming a center-regression-type menu, which will be described below, are arranged in a square operation region AR represented by a dashed line.

The center of the operation region AR is an origin position (x0, y0) as a two-dimensional coordinate of the x-y plane and an origin item CTi is arranged at the origin position. Then, a plurality of items Mi to be selected are arranged around the origin item CTi. Here, six items Mi are arranged.

For a non-contact operation of the user, in a case where an operation position specified by the operation direction and the operation distance from the origin position (the origin item CTi as seen by the user) as a starting point reaches a certain item Mi, it is recognized that the item Mi has been selected, which will be described in detail below.

In Fig. 3A, six items Mi arranged around the origin item CTi are arranged at regular intervals along a virtual ring CR (a virtual ring-shaped line represented by a one-dot chain line) which is a true circle having the origin position as the center.

In addition, the virtual ring CR is illustrated in order to describe the arrangement position of the operation plane and is not displayed such that the user recognizes the virtual ring CR as the user interface.

The number of items Mi is six since the number of items selected from the menu is six and the number of items arranged on the operation plane varies depending on choices that can be operated.

Fig. 3B illustrates a case where the number of items Mi is three and Fig. 3C illustrates a case where the number of items Mi is two. In any of the cases, the items Mi are separately arranged at regular intervals along the virtual ring CR.

In addition, the items Mi may not be separately arranged at regular intervals. Fig. 3D illustrates a case where three of four items Mi are arranged so as to be biased to the left.

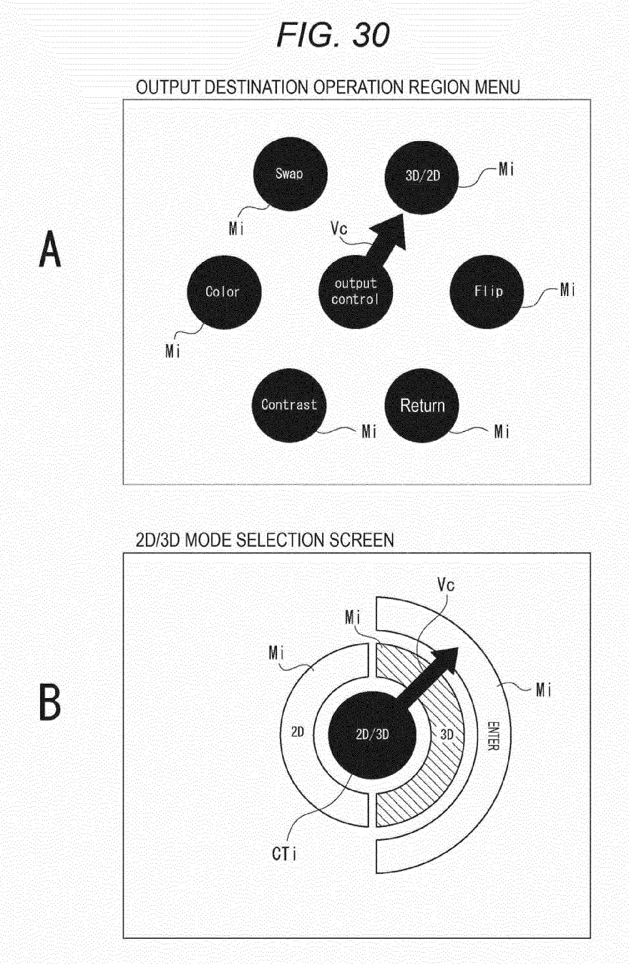

Further, the shape of the item Mi is not limited to a circular shape. Fig. 30 illustrates an example in which elliptical items Mi are arranged. In addition to the above-mentioned shapes, although not shown, for example, items Mi having a square shape, a rectangular shape, and other shapes, triangular items Mi, items Mi having an irregular shape, and items Mi having a specific shape, such as a star shape, are considered.

Furthermore, in the examples illustrated in Figs. 3A to 3E, the items Mi are arranged so as to be separated from each other. However, as illustrated in Fig. 3F, the items Mi may be arranged such that adjacent items come into contact with each other. In addition, the items Mi may be arranged so as to come into contact with each other, regardless of the number of items Mi arranged. For example, in a case where the item Mi has an oval shape curved along the virtual ring CR, a plurality of items Mi can be arranged while coming into contact with each other (that is, with no gap therebetween) even if the number of items is small.

Here, in the operation plane according to any of the examples illustrated in Figs. 3A to 3E, the origin item CTi is arranged at the origin position (x0, y0) and a plurality of items Mi to be selected are arranged around the origin item CTi as illustrated in Fig. 3A.

In particular, in the case of the center-regression-type menu, a plurality of items Mi to be selected are arranged around the origin position so as not to overlap each other as viewed from the origin position.

Note that the example in which a plurality of items Mi are arranged around the origin position along the virtual ring CR which is a virtual true circle having the origin position as the center is given, but the virtual ring CR may not be a true circle. For example, the virtual ring CR having an elliptical shape, a square shape, a rectangular shape, a triangular shape, an irregular shape, or the like may be assumed and the items Mi may be arranged along the virtual ring CR.

Figs. 3G and 3H illustrate examples of the operation plane in which items Mi forming a pie-type menu, which will be described below, are arranged in the square operation region AR represented by a dashed line.

In this case, the center of the operation region AR is the origin position (x0, y0) as a two-dimensional coordinate of the x-y plane and the origin item CTi is arranged at the origin position. Then, a plurality of items Mi to be selected are arranged around the origin item CTi.

However, in this case, virtual rings CR1, CR2, and CR3 are illustrated and the items Mi are arranged along each of the virtual rings. Fig. 3G illustrates a state in which three items Mi are arranged along a first virtual ring and Fig. 3H illustrates a state in which four items Mi are arranged along the first virtual ring.

In a case where an upper-layer item is selected, items are set on a second virtual ring and a third virtual ring. For example, in a case where a certain item Mi on the first virtual ring is selected, lower-layer items, an item for an enter operation, and the like are arranged along the second virtual ring according to the selected item Mi.

The pie-type menu will be described in detail below. For a non-contact operation of the user, in a case where an operation position specified by the operation direction and the operation distance from the origin position (the origin item CTi as seen by the user) as a starting point reaches a certain item Mi, it is recognized that the item Mi has been selected.

In addition, a plurality of items Mi on one virtual ring CR are arranged so as not to overlap each other as viewed from the origin position. For example, a plurality of items Mi on the first virtual ring CR1 in Figs. 3G and 3H do no overlap each other as viewed from the origin position. In addition, in a case where the items Mi are arranged on the second virtual ring CR2, of course, the items Mi may overlap the items Mi on the first virtual ring CR1 as viewed from the origin position. However, a plurality of items Mi on the second virtual ring CR are arranged so as not to overlap each other as viewed from the origin position.

However, the operation region AR forming the operation plane is considered in various ways in relation to the screen of the display device 11.

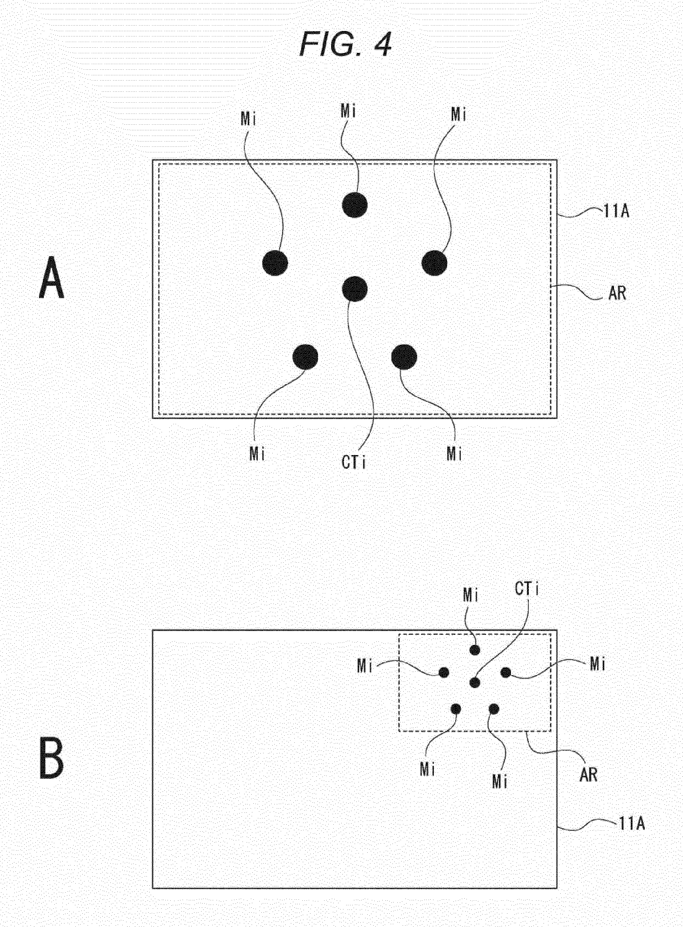

Fig. 4A illustrates an example in which the operation region AR of the center-regression-type menu corresponds to the entire screen 11A of the display device 11. In this configuration, the center-regression-type menu is displayed with a large size on the screen such that the user easily recognizes a menu operation.

Fig. 4B illustrates an example in which the operation region AR of the center-regression-type menu corresponds to a portion of the screen 11A of the display device 11. This configuration makes it possible to perform a menu operation while displaying the center-regression-type menu and other images on the screen.

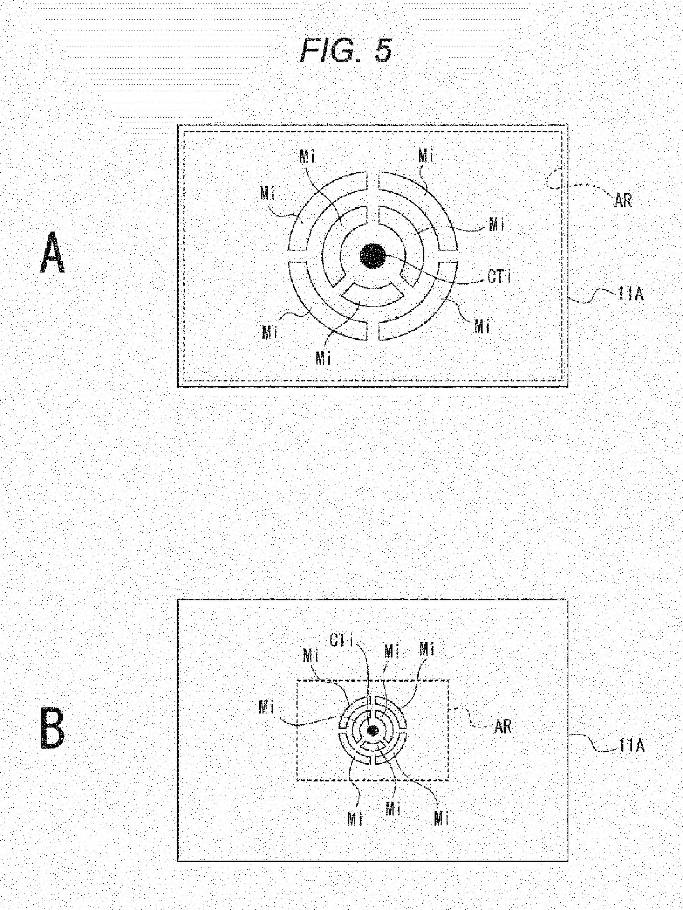

Fig. 5A illustrates an example in which the operation region AR of the pie-type menu corresponds to the entire screen 11A of the display device 11. In this configuration, the pie-type menu is displayed with a large size on the screen such that the user easily recognizes a menu operation.

Fig. 5B illustrates an example in which the operation region AR of the pie-type menu corresponds to a portion of the screen 11A of the display device 11. This configuration makes it possible to perform a menu operation while displaying the pie-type menu and other images on the screen.

The menu control unit 31 illustrated in Fig. 1 sequentially sets the operation plane illustrated in Fig. 3 according to the progress of an operation. Then, the menu control unit 31 provides the information of the operation plane to the display data generation unit 4.

The menu control unit 31 sets the operation plane using the information stored in the menu information storage unit 32 according to the progress of the operation. The menu information storage unit 32 stores detailed menu information, for example, the number of layers, the number of items Mi in each layer, the content of each itemMi, the setting information of an arrangement position (a coordinate region in which the items are arranged), and the like. The menu control unit 31 sequentially sets the operation plane, in which necessary items Mi are arranged, at each point of time with reference to the information of, for example, the items Mi necessary at the point of time.

In addition, the menu control unit 31 associates the information of the operation direction and the operation distance from the vector calculation unit 22 with the coordinates on the set operation plane as a process for monitoring the user's operation. In this case, the menu control unit 31 determines the operation direction from the origin position (x0, y0) at the center of the operation region AR. That is, the menu control unit 31 recognizes the operation direction in an angular range of 360° which has the origin position as the center. In addition, the menu control unit 31 recognizes the operation distance as a length extending form the origin position in the direction of the angle. In this way, the menu control unit 31 specifies the operation position on the operation plane in the operation region AR.

Then, the menu control unit 31 recognizes the selected state of a certain item Mi on the basis of the relationship between the operation position specified by the operation direction and the operation distance and the position where the item Mi is arranged.

For example, in a case where the operation position is in the arrangement region of a certain item Mi, the menu control unit 31 recognizes that the item Mi has been selected. In addition, for example, in a case where the operation position passes through the arrangement region of a certain item Mi, the menu control unit 31 recognizes that the item Mi has been selected.

Further, in a case where a certain item Mi has been selected or an enter operation has been performed, the menu control unit 31 stores the information of the operation in the operation information storage unit 33. That is, the menu control unit 31 stores the information selected or entered by the menu operation.

The menu control unit 31 refers to the operation information stored in the operation information storage unit 33 in order to use the operation information for the subsequent menu progression process or the operation information is referred to by other application programs or an operating system (OS) of the information processing device 1 such that an operation corresponding to the menu operation is performed.

Note that the operation information storage unit 33 described herein corresponds to a unit that outputs the operation information to a program, such as an OS or an application program, which performs a process based on the menu operation. That is, a processing program writes the operation information to a register for detecting an operation and the register may be considered as the operation information storage unit 33.

For example, the information of the operation plane set for a menu operation by the menu control unit 31 and information indicating the recognized state of a selection operation or an enter operation by the relationship between the item Mi and the operation position based on the operation direction and the operation distance are also provided to the display data generation unit 4. In addition, as described above, the operation information subjected to the coordinate conversion is provided from the coordinate conversion unit 23 to the display data generation unit 4.

The display data generation unit 4 generates display data for presenting a screen for the user interface on the basis of these information items.

For example, the display data generation unit 4 generates image data of the operation plane actually displayed on the display device 11 on the basis of, for example, the information of the operation plane set by the menu control unit 31 and character information for displaying the items Mi or the like stored in the menu information storage unit 32.

In this case, the display data generation unit 4 assigns each item Mi to an area defined by a distance range and an angle, on the basis of the information (for example, the numbers of images in the horizontal and vertical directions and the coordinates of the center) of a display area obtained from the coordinate conversion unit 23 and the detailed menu information (the number of layers and items) obtained from the menu information storage unit 32.

In addition, the display data generation unit 4 draws information to be displayed on the basis of the information of the position operation from the coordinate conversion unit 23 or the recognition information of, for example, the selected state from the menu control unit 31.

The display data generation unit 4 outputs the display data generated by these processes to the display device 11 so as to be displayed on the display screen 11a.

In addition, the display data generation unit 4 generates display data corresponding to an instruction from the menu control unit 31.

For example, the information processing device 1 according to the embodiment having the functional configuration illustrated in Fig. 1 can provide the center-regression-type menu or the pie-type menu, which will be described below, to the user, recognize a menu operation corresponding to a non-contact operation of the user, and display the user interface accompanied by the progress of the menu on the display device 11.

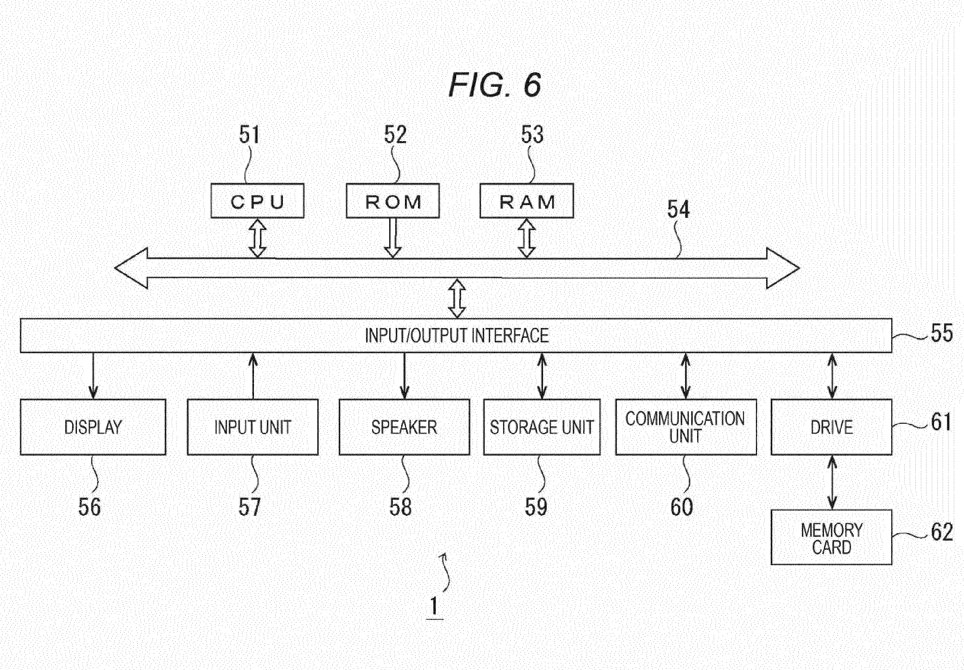

The information processing device 1 is implemented by, for example, a hardware configuration illustrated in Fig. 6.

As illustrated in Fig. 6, the information processing device 1 includes a central processing unit (CPU) 51, a read only memory (ROM) 52, and a random access memory (RAM) 53.

The CPU 51 performs various processes according to a program stored in the ROM 52 or a program loaded from a storage unit 59 to the RAM 53. In addition, the RAM 53 appropriately stores, for example, data necessary for the CPU 51 to perform various processes.

The CPU 51, the ROM 52, and the RAM 53 are connected to each other through a bus 54. In addition, an input/output interface 55 is connected to the bus 54.

For example, a display 56, such as a liquid crystal panel or an organic EL panel, an input unit 57, such as a keyboard or a mouse, a speaker 58, the storage unit 59, such as an HDD, and a communication unit 60 can be connected to the input/output interface 55.

In a case where the display device 11 illustrated in Fig. 1 is integrated with the information processing device 1, the display 56 means the display device 11. Of course, the display device 11 may be provided separately from the information processing device 1. In this case, the display 56 (display device 11) may be a separate device connected to the input/output interface 55.

The input unit 57 illustrated in Fig. 6 means an input device used by the operator who uses the information processing device 1. The tracking device 10 illustrated in Fig. 1 is an example of the input unit 57. The tracking device 10 may be provided integrally with or separately from the information processing device 1.

The communication unit 60 performs a communication process through a network including the Internet or performs communication with peripheral devices.

In addition, a drive 61 is connected to the input/output interface 55 if necessary and a memory card 62 is inserted into the drive 61. A computer program read from the memory card 62 is installed in the storage unit 59 if necessary or data processed by the CPU 51 is stored in the storage unit 59. Of course, the drive 61 may be a recording and reproducing drive for a removable storage medium, such as a magnetic disk, an optical disk, or a magneto-optical disk.

It is possible to perform the processes of the information processing device 1 according to the embodiment, that is, the above-mentioned processes of the operation detection unit 2, the control unit 3, and the display data generation unit 4 illustrated in Fig. 1 in the above-mentioned hardware configuration. That is, these processes are implemented by software run by the CPU 51. A program forming the software is downloaded from a network or is read from a removable storage medium and is then installed in the information processing device 1 illustrated in Fig. 6. Alternatively, the program may be stored in, for example, an HDD as the storage unit 59 in advance.

Then, the CPU 51 runs the program to perform a process of recognizing a non-contact operation associated with the center-regression-type menu or the pie-type menu, which will be described in detail below.

In addition, the information processing device 1 according to the embodiment is not limited to the configuration in which a single device (computer device) having the hardware configuration illustrated in Fig. 6 is provided and may have a configuration in which a plurality of computer devices are systematized. The plurality of computer devices may be systematized by a LAN or the like or may be arranged in remote areas by a VPN or the like using the Internet or the like. The plurality of computer devices may include computer devices that can be used by a cloud computing service.

### <2. Center-regression-type Menu>

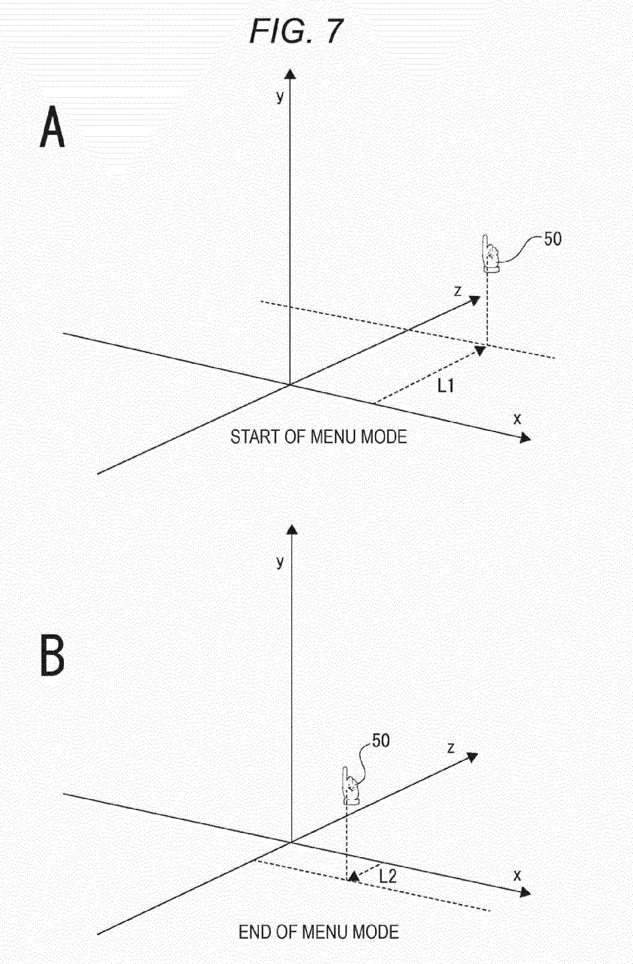

An input operation using the center-regression-type menu will be described. First, the outline of the operation will be described with reference to Figs. 7, 8, 9, and 10.

The user performs an operation of selecting a menu item in a state in which the center-regression-type menu is displayed on the display device 11. First, the user performs an operation of starting a menu mode in order to display the center-regression-type menu.

The start of the menu mode is an example of the non-contact operation. An operation in the z-axis direction (a front-rear direction as viewed from the user) for the x-y plane corresponding to the operation plane is given as an example.

A selection operation on the center-regression-type menu according to the embodiment does not require an operation in the z-axis direction. Therefore, the easiness and stability of an item selection operation are improved. Even in a case where the operation in the z-axis direction is performed to start and end the menu mode, the stability of the operation is not damaged. Therefore, it is assumed that the operation in the z-axis direction is performed to start and end the menu mode.

Fig. 7A illustrates the x-axis, the y-axis, and the z-axis in a space in which the user performs operations.

The movement of an operation body 50 (for example, the hand of the user) in the z-direction is detected assuming that a two-dimensional plane formed by the x-axis and y-axis faces the user.

Fig. 7A illustrates a state in which the user is located on the front side of the plane of Fig. 7A from the x-y plane and the hand (operation body 50) of the user is L1 cm away from the x-y plane in the z direction. For example, L1 cm is 3 cm, or the like.

That is, the user pushes his or her hand forward as illustrated in 7A. The information processing device 1 recognizes this operation as a menu mode start operation.

Fig. 7B illustrates an example of a menu mode end operation. In this case, the user who is located on the front side of the x-y plane performs an operation of pulling back the hand (operation body 50) from the x-y plane in the z-direction by L2 cm as the menu mode end operation. For example, L2 cm is 1 cm, or the like.

That is, once the user pulls back the hand pushed forward, the operation of pulling back the hand from the x-y plane by L2 cm is performed in the process and the information processing device 1 recognizes the operation as the menu mode end operation.

As described above, the tracking device 10 can continuously detect a three-dimensional coordinate as the position of the operation body 50 and the control unit 3 (menu control unit 31) can detect the movement of the operation body 50 in the z direction from the vector information in the z direction (the operation direction and the operation distance in the z direction) from the vector calculation unit 22.

The menu control unit 31 starts to process the center-regression-type menu in response to the detection of the menu mode start operation illustrated in Fig. 7A.

In addition, in a case where the menu mode end operation illustrated in Fig. 7B is detected particularly in a state in which an enter operation is not performed during the processing of the center-regression-type menu, the menu control unit 31 ends the processing of the center-regression-type menu.

In addition, the menu mode start or end operation method is not limited to the above-mentioned example and various methods are considered.

For example, it is considered that the specific movement (for example, movement drawing a circle, or the like) of the operation body 50 is detected as the menu mode start or end operation or a specific gesture of the user is detected.

In addition, voice detection may be performed to recognize, for example, a user's voice or a specific sound as the menu mode start or end operation.

Of course, for example, an operation of pressing a specific operator or an operation by a remote controller corresponding to an electronic device may be detected as the menu mode start/end operations.

In addition, for the end of the menu mode, a menu end item may be prepared as one of selectable items of a menu screen which will be described below.

In a case where the menu mode start operation is recognized, the display of the center-regression-type menu is started by the functions of the control unit 3 and the display data generation unit 4 and menu processing corresponding to the non-contact operation of the user is performed.

That is, the menu control unit 31 sets the operation plane in which various items have been arranged at each point of time while monitoring the operation direction and the operation distance in the x-y plane by the non-contact operation of the user. Then, the display data generation unit 4 generates display data for displaying the operation plane at each point of time or the operation direction and the operation distance according to the operation plane or the operation direction and the operation distance and supplies the display data to the display device 11. In this way, an operation interface screen which will be described below is provided to the user.

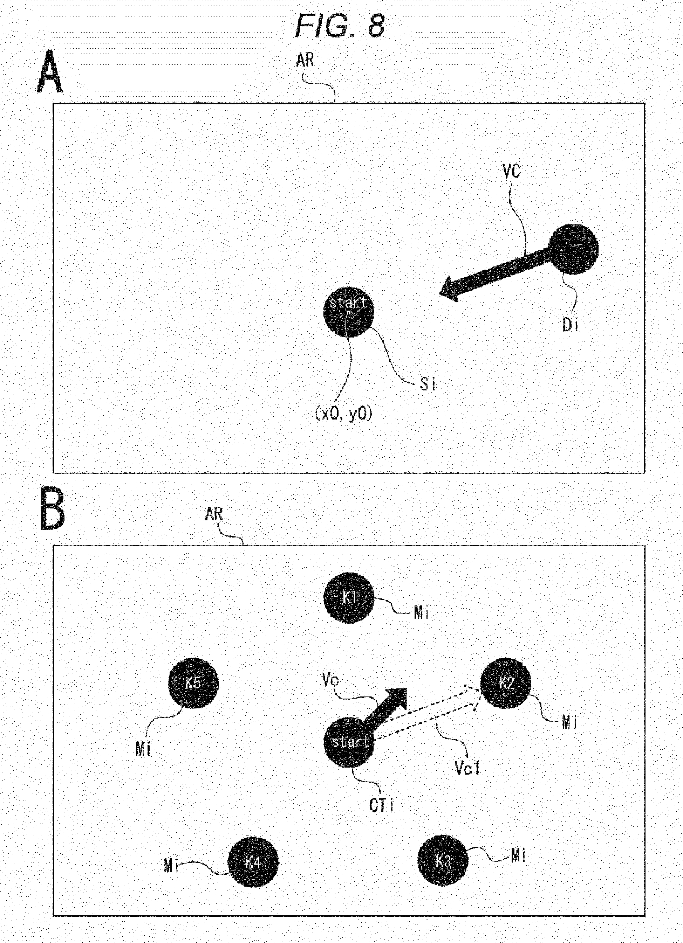

Fig. 8A illustrates an initial screen (initial image) of the center-regression-type menu.

In addition, for the center-regression-type menu, the center of the operation region AR is the origin position (x0, y0) of the x-y plane coordinates forming the operation plane.

For example, a start button Si represented by "start" is displayed at the origin position. In addition, a start operator Di is displayed at a certain position in the operation region AR.

The start button Si and the start operator Di are prepared for the user to perform an operation of starting the display of the center-regression-type menu. The coordinates of the position of the start operator Di may be fixed or the start operator Di may be located at a random position.

In this state, the control unit 3 recognizes the operation of the user as the operation direction and the operation distance from the start operator Di as a starting point and specifies the coordinates of the operation position on the operation plane.

In addition, the display data generation unit 4 acquires the information of the operation position from the coordinate conversion unit 23 and draws the operation vector Vc indicating the operation position such that a display state illustrated in Fig. 8A is obtained.

Therefore, the operation position on the operation plane is recognized according to the non-contact operation of the user and the direction or length of the displayed operation vector Vc is changed.

In a case where the operation position (a coordinate value corresponding to the leading end of the vector Vc) detected according to the non-contact operation of the user reaches the start button Si (for example, a coordinate range in which the circular start button Si is arranged), the control unit 3 recognizes that the user has selected the start of the center-regression-type menu. That is, the leading end of the operation vector Vc reaches the start button Si on the screen.

Then, the control unit 3 sets the operation plane in which items in the highest layer, in which a plurality of items to be selected are arranged, are arranged. The display data generation unit 4 generates display data corresponding to the operation plane and supplies the display data to the display device 11 such that the display data is displayed on the screen. For example, menu screen display illustrated in Fig. 8B is performed.

Note that, in this example, in a case where the start button Si is designated by an operation using the initial image of Fig. 8A following the menu mode start operation, a menu in the highest layer illustrated in Fig. 8B appears. However, the initial image may not be used and the menu illustrated in Fig. 8B may be instantly displayed in response to the menu mode start operation. This method is advantageous in terms of the efficiency of the operation.

A start operation using the initial image screen illustrated in Fig. 8A is suitable for a case where an unnecessary shift to menu display is not desired. In particular, in a case where the menu mode start operation is the operation described in Fig. 7A, the control unit 3 may recognize a user's small gesture as the start operation. Therefore, the image illustrated in Fig. 8A is displayed once and the display of the menu may start only in a case where the user performs the start operation within a predetermined period of time (for example, within 10 seconds, or the like) . In particular, the simple screen illustrated in Fig. 8A is used as the start operation screen. In a case where the start operation is not performed within a predetermined period of time, it is determined that the menu mode has ended and the image illustrated in Fig. 8A is removed, which makes it possible to prevent the occurrence of the situation in which the menu screen in Fig. 8B is unnecessarily displayed without any reason and the user feels complicated.

Hereinafter, the transition of the menu screen after Fig. 8B will be described as a change in the screen recognized by the user. Each screen described herein is an example of a display image obtained by the display data generated by the display data generation unit 4 to present the operation plane sequentially set by the control unit 3 and the operation direction and the operation distance (the operation position determined by the operation direction and the operation distance) recognized by the control unit 3 to the user.

In the menu screen illustrated in Fig. 8B, for example, an origin item CTi represented by "start" is displayed at the origin position. Then, a state in which a plurality of items Mi to be selected are arranged around the origin item CTi at the origin position is displayed. Here, Fig. 8B illustrates a case where there are five choices on the menu. The contents "K1" to "K5" of the items Mi are described in each of the items Mi. Here, "K1" to "K5" are described for convenience. However, in practice, the contents of the operations recognized by the user are described.

In the example, the items Mi are arranged at regular intervals along the virtual ring CR of a true circle having the origin position as the center as described in Fig. 3A.

The starting point of the operation direction and the operation distance by the non-contact operation of the user in the center-regression-type menu is basically the origin position (Fig. 8A is exceptional). The origin item CTi is arranged at the origin position located at the center of the operation region AR as viewed from the user. Therefore, the center can be perceived as the starting point of the operation.

The situation of the non-contact operation of the user is represented by the operation vector Vc extending from the origin item CTi.

The control unit 3 associates the operation direction and the operation distance detected as vector information with the origin position as the starting point to calculate a certain coordinate point on the operation plane in the operation region AR as the operation position.

The display data generation unit 4 calculates the operation position, using the origin position as the starting point, on the basis of information from the coordinate conversion unit 23 and draws the operation vector Vc on the image of the operation plane on the basis of the operation position. Therefore, an image in which the operation vector Vc extends from the origin item CTi to the operation position is presented on the display.

The user may perform a non-contact operation while viewing the operation vector Vc such that the operation vector Vc reaches a desired item Mi . The items Mi are arranged around the origin item CTi so as not to overlap each other as viewed from the origin item CTi (origin position), which makes it possible for the operation vector Vc to directly reach any item Mi (without being disturbed by other items Mi).

For example, in a case where the user performs a non-contact operation such that the operation vector Vc reaches a"K2" item Mi as represented by a dashed line Vc1 in Fig. 8B, "K2" is selected. In addition, the term "select" in the center-regression-type menu indicates not only that an item Mi is simply being selected but also that the item Mi is entered.

Therefore, "K2" is selected and entered only by making the operation vector Vc reaches the item Mi represented by "K2".

Here, in a case where a lower layer is not present in the"K2" item Mi, the control unit 3 stores information indicating that the K2 operation has been selected and entered by the operation in the operation information storage unit 33. The OS or the application program of the information processing device 1 recognizes the operation information and a predetermined process and a predetermined operation are performed.

In contrast, in a case where a menu in the lower layer is present in the "K2" item Mi, an operation interface accompanied by center regression is performed.

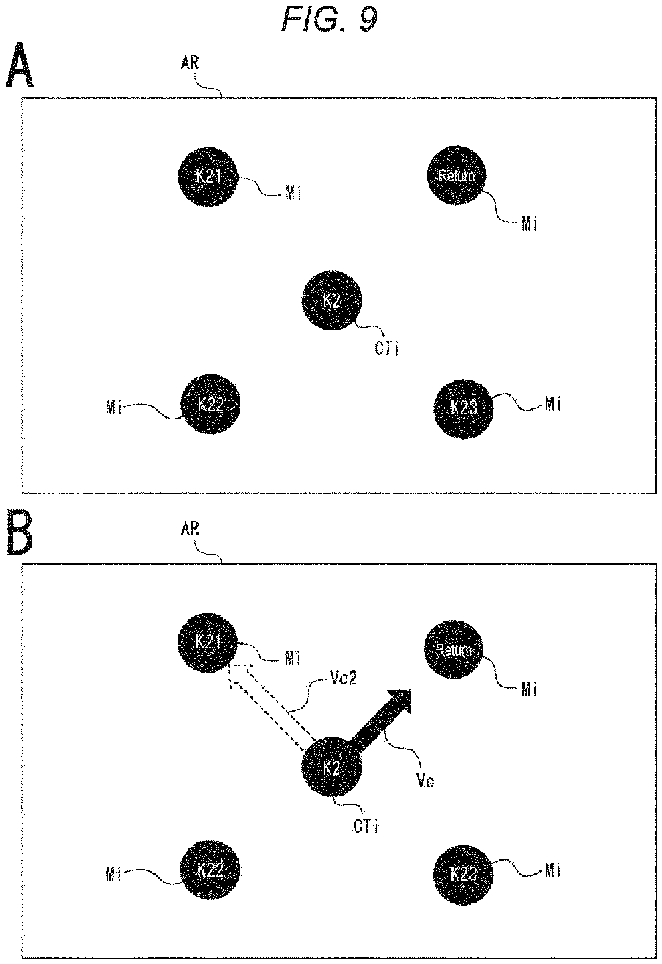

For example, a display screen illustrated in Fig. 9A is displayed.

In this case, the "K2" item Mi is moved to the origin position at the center and becomes the origin item CTi.

Then, a plurality of items Mi (in this case, four items Mi) to be selected are arranged around the "K2" origin item CTi. In this example, similarly to the above-mentioned screen, the four items Mi are arranged at regular intervals along the virtual ring CR (see Fig. 3A) of a true circle having the origin position as the center.

Here, three operation contents "K21", "K22", and "K23" are present as choices in the lower layer of "K2". In this case, items Mi indicating four operation contents including the three operation contents and a "return" operation are arranged.

A "return" item Mi is arranged at the coordinates of the position where the "K2" item has been arranged or at the coordinates of a position in the vicinity of the position on the screen illustrated in Fig. 8B. Therefore, in a case where the operation vector Vc extends to the "K2" item as represented by the dashed line Vc1 in Fig. 8B, the "K2" item Mi is moved to the center and the items Mi in the lower layer are rearranged around the "K2" item as viewed from the user.

In addition, the user perceives that the "start" origin item CTi is interchanged and moved to the vicinity of the "K2" item and becomes the "return" item Mi. In addition, here, the example in which the "start" item is changed to the "return" item and is then displayed has been described. However, "start" may be displayed without any change.

However, in a case where the screen is suddenly switched from the state illustrated in Fig. 8B to the state illustrated in Fig. 9A, it may be difficult for the user to recognize the center regression aspect of the "K2" item. Therefore, an image of a center regression process that represents the switching between the "K2" item and the "start" item may be displayed.

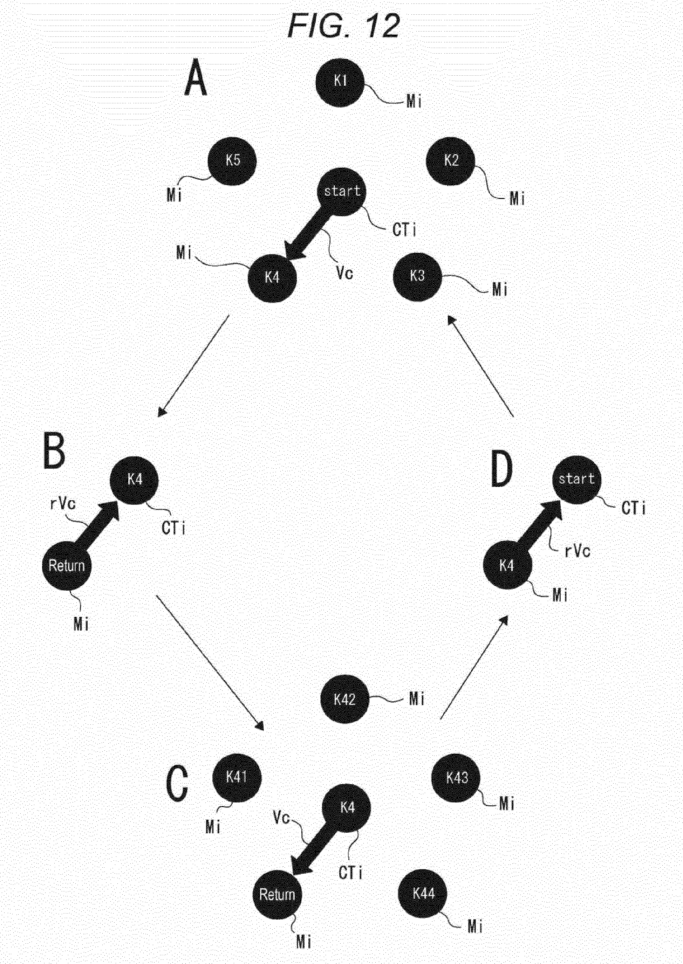

That is, in a case where the operation plane is set again in order to advance to the next menu layer, the control unit 3 controls the display data generation unit 4 such that information presenting the movement of the selected item Mi to the origin position is displayed. This example will be described with reference to Fig. 12.

In the screen illustrated in Fig. 9A, the origin position is a starting point for recognizing the operation position.

That is, as illustrated in Fig. 9B, display is performed such that the operation vector Vc extends from the "K2" item changed to the origin item CTi.

In addition, in this case, the items Mi are arranged around the origin item CTi so as not to overlap each other as viewed from the origin item CTi (origin position) . Therefore, the operation vector Vc can reach any itemMi without being disturbed by other items Mi.

Therefore, the user may perform a non-contact operation such that the operation vector Vc reaches a desired item Mi while viewing the operation vector Vc. Then, the item Mi which the operation vector Vc has reached is selected and entered.

In a case where the "return" item Mi is selected and entered by the non-contact operation of the user, the menu returns to the upper layer, that is, the state illustrated in Fig. 8B. In this case, the "return" item Mi is moved to the origin position and becomes the origin item CTi indicating "start" . In addition, the "K2" item which has been the origin item CTi is moved so as to be changed to the "return" item and returns to the original position illustrated in Fig. 8B.

In addition, for example, in a case where the user performs a non-contact operation such that the operation vector Vc reaches a lower-layer item Mi, such as a "K21" item, as represented by a dashed line Vc2 in Fig. 9B, the "K21" item is selected.

In a case where a lower layer is not present in the "K21" item Mi, the control unit 3 stores information indicating that the "K21" item has been selected and entered by the operation in the operation information storage unit 33. The OS or the application program of the information processing device 1 recognizes the operation information and a predetermined process and a predetermined operation are performed.

In contrast, in a case where a menu in the lower layer is present in the "K21" item Mi, an operation interface accompanied by center regression is performed similarly to the above.

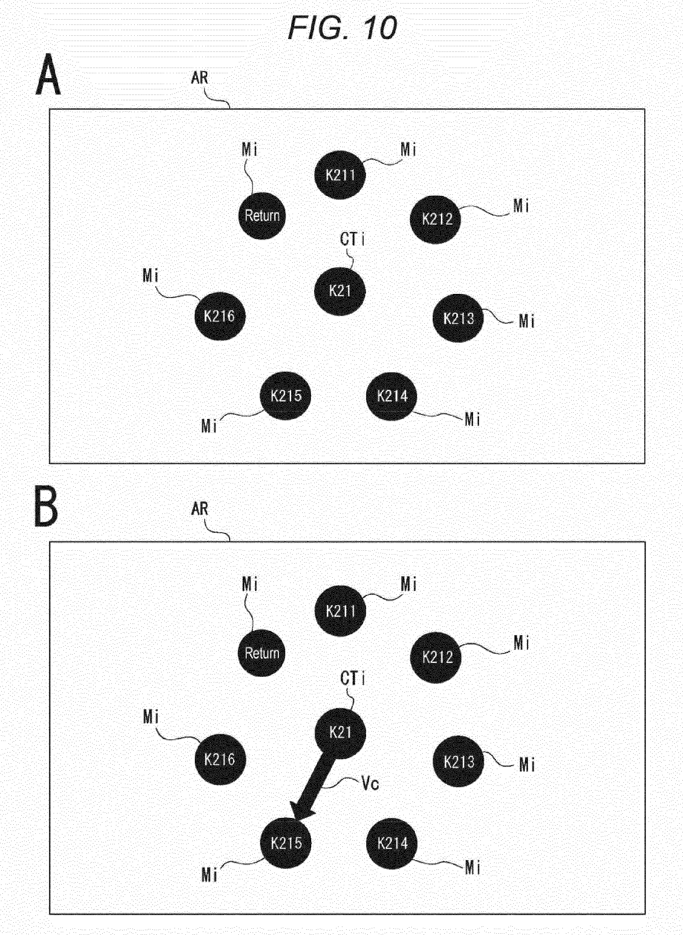

For example, the display screen is as illustrated in Fig. 10A.

In this case, the "K21" item Mi is moved to the origin position at the center and becomes the origin item CTi.

Then, a plurality of items Mi to be selected are arranged around the "K21" origin item CTi similarly to the above-described pattern. In this case, seven items Mi, that is, six operation content items Mi represented by "K211" to "K216" and a "return" operation item Mi are arranged as choices in the lower layer of the "K21" item.

The "return" item Mi is arranged at the coordinates of the position where the item "K21" is originally arranged or the coordinates of a position close to the position on the screen illustrated in Fig. 9B.

Therefore, in a case where the operation vector Vc extends to the "K21" item as represented by the dashed line Vc2 in Fig. 9B, the "K21" item Mi is moved such that the "K21" item Mi and the "K2" origin item CTi are interchanged.

In this example, the"K2" item is changed to the "return" item and is then displayed. However, "K2" may be displayed without any change.

In a case where the screen is suddenly switched from the state illustrated in Fig. 9B to the state illustrated in Fig. 10A, it may be difficult for the user to recognize the center regression aspect of the "K21" item. Therefore, an image of a center regression process representing the interchange between the "K21" item and the "K2" item may be displayed.

In the screen illustrated in Fig. 10A, the origin position is a starting point for recognizing the operation position.

That is, as illustrated in Fig. 10B, display is performed such that the operation vector Vc extends from the "K21" item changed to the origin item CTi.

Similarly to the above, in the operation in the layer illustrated in Fig. 10B, for example, in a case where the operation vector Vc reaches the "K215" item Mi as illustrated in Fig. 10B, the "K215" item Mi is selected and entered.

In addition, in a case where the operation vector Vc reaches the "return" item Mi, the menu returns to the state illustrated in Fig. 9A. At that time, the "return" item Mi returns to the center and becomes the "K2" origin item CTi.

As described above, in the case of the center-regression-type menu, the operation vector Vc extends from the origin item CTi at the center of the operation region AR and reaches the item Mi to perform a selection operation. In addition, the center regression of the item Mi selected with the progress and return of the menu layer is performed. Therefore, the items to be selected are rearranged around the origin position and an operation having the origin position as a starting point is performed.

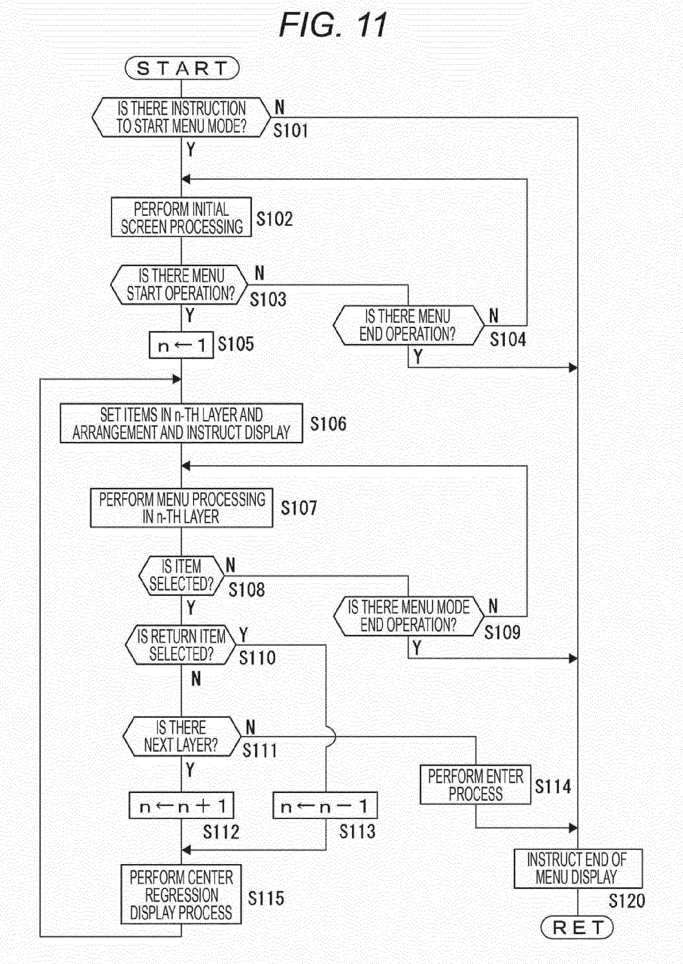

An example of the process of the information processing device 1 for implementing the operation by the center-regression-type menu will be described with reference to Fig. 11. Fig. 11 illustrates an example of the process of the control unit 3. The control unit 3 sequentially inputs vector information corresponding to a non-contact operation from the operation detection unit 2 and performs the process illustrated in Fig. 11. In addition, the control unit 3 transmits, for example, the information of the operation plane or the operation position set according to the progress of the process to the display data generation unit 4 such that the display data generation unit 4 performs, for example, the above-mentioned menu display.

In Step S101 of Fig. 11, the control unit 3 monitors whether or not a menu mode start operation has been performed.

For example, in a case where the menu mode start operation described in Fig. 7A is detected, the control unit 3 proceeds to Step S102 and performs a process of displaying the initial image illustrated in Fig. 8A.

In the state in which the initial screen is displayed, the control unit 3 monitors the menu start operation (the selection of a start button Si) of the user in Step S103 and monitors the end of the menu mode in Step S104.

For example, in a case where the menu mode end operation described in Fig. 7B is detected, the control unit 3 proceeds from Step S104 to Step S120 and transmits the end of the menu mode to the display data generation unit 4 to end the display of the initial image. Then, the control unit 3 ends the processing of the menu mode.

In addition, in a case where the start button Si is not selected from the initial image in Fig. 8A for a predetermined period of time or more, the control unit 3 may determine that the user does not intend to perform a menu operation, end the menu mode, proceed from Step S104 to Step S120 to end the display of the initial image, and end the processing of the menu mode.

In a case where the start button Si is selected in the initial image, the control unit 3 proceeds to Step S105 and sets a variable n indicating the current menu layer to 1.

Then, in Step S106, the control unit 3 sets an operation plane in an n-th layer. That is, since n is 1 at the beginning, the control unit 3 sets an operation plane in the first layer which is the highest layer. Specifically, the menu control unit 31 reads information required to set the operation plane, such as the information of menu selection items in the first layer or the arrangement of each item, from the menu information storage unit and sets the operation plane in which the origin item CTi and the items Mi are arranged in the operation region AR.

In addition, the control unit 3 supplies the information of the set operation plane in the first layer to the display data generation unit 4 and instructs the display data generation unit 4 to display a menu screen in the first layer. The display data generation unit 4 reads the information of items and arrangement on the operation plane in the first layer and character data required to draw, for example, each item Mi and the origin item CTi from the menu information storage unit 32, generates display data for the first layer, and supplies the display data to the display device 11. For example, the display of the menu in the first layer illustrated in Fig. 8B is performed by these processes.

In Step S107, the control unit 3 performs menu processing in the n-th layer.

That is, the control unit 3 reflects the operation direction and the operation distance sequentially recognized as the vector information as a direction and a distance from the origin position of the operation plane set in Step S106 on the operation plane to specify an operation position. Then, the control unit 3 checks whether or not the specified operation position (that is, a position corresponding to the leading end of the operation vector Vc on the display) is matched with the arrangement region (a coordinate range corresponding to a character display range of the item Mi on the operation plane) of a certain item Mi.

The control unit 3 checks whether or not the operation position reaches the arrangement region of any item Mi in Step S107 whenever the vector information is acquired. In a case where the operation position does not reach the arrangement region of any item Mi, the control unit 3 proceeds from Step S108 to Step S109 and monitors a menu mode end operation. In a case where the menu mode end operation is not performed, the control unit 3 returns to Step S107.

Note that, for the period of the monitoring loop process in Steps S107, S108, and S109, the display data generation unit 4 draws the operation vector Vc on the menu image on the basis of the x and y coordinate values converted into display output coordinate values by the coordinate conversion unit 23 and displays the operation vector Vc on the display device 11. In this way, the current operation position recognized by the control unit 3 is presented to the user by the operation vector Vc on the menu image.

In a case where the menu mode end operation of the user has been detected, the control unit 3 proceeds from Step S109 to Step S120 and transmits the end of the menu mode to the display data generation unit 4 to end the display of the menu performed at that time. Then, the control unit 3 ends the processing of the menu mode.

Note that, in a case where the selection of an item is not detected for a predetermined period of time or more in Step S107, the control unit 3 may determine that the menu mode has ended in Step S109.

In a case where it is detected that the operation position reaches the arrangement region of a certain item Mi in the process of Step S107, the control unit 3 determines that an item selection operation has been performed and proceeds from Step S108 to Step S110.

In Step S110, the control unit 3 branches the process on the basis of whether or not the selected item Mi is a "return" item. In a case where the selected item Mi is a "return" item, the control unit 3 decrements the variable n in Step S113 and proceeds to Step S115. This is a case where an operation of returning to an upper layer has been performed.

In a case where the selected item Mi is not a "return" item, the control unit 3 branches the process on the basis of whether or not a lower layer is present in the selected item Mi in Step S111. In a case where a lower layer is present, the control unit 3 increments the variable n in Step S112 and proceeds to Step S115. This is a case where an operation of advancing to a lower layer has been performed.

In a case where a lower layer is not present in the selected item Mi, the control unit 3 performs an enter process in Step S114 . That is, the control unit 3 stores operation information indicating that the content of the item Mi has been operated in the operation information storage unit 33 such that the content of the operation executed by the OS or the application program can be recognized. Then, the control unit 3 directs the display data generation unit 4 to end the display of the menu in Step S120 and ends the processing of the menu.

In a case where the process proceeds to Step S115 according to the return operation or the operation of advancing the lower layer, the control unit 3 instructs the display data generation unit 4 to perform center regression display. Then, the control unit 3 proceeds to Step S106 and performs the setting of the operation plane as a menu in an n-th layer and control for the display of the menu in the layer.

Display transition corresponding to these processes will be described with reference to Fig. 12.

First, the case of advance to a lower layer will be described. Fig. 12A illustrates an aspect in which a "K4" item is selected by an operation for the menu in the first layer, for example, in a case where the variable n is 1. It is assumed that a lower layer is present in the "K4" item.

In a case where the control unit 3 detects this state, that is, the entrance of the operation position into the arrangement region of the "K4" item in Step S107, the control unit 3 proceeds in the order of Step S108→Step S110→Step S111→Step S112→Step S115. In Step S115, the control unit 3 instructs the display data generation unit 4 to perform display illustrated in Fig. 12B.

That is, immediately after the operation vector Vc reaches the "K4" item as illustrated in Fig. 12A, a "start" item is changed to a "return" item as illustrated in Fig. 12B, the "return" item is moved to the original position where the "K4" item has been arranged, and the "K4" item is moved to the center position (origin position) so as to be interchanged with the "return". In addition, a regression arrow rVc indicating the movement (center regression) is displayed as illustrated in Fig. 12B.

Particularly, in a case where only two items whose positions are instantly interchanged in the operation region AR are displayed or information indicating the movement aspect of each of the two items is displayed for about one second, the user can very clearly recognize that the selected item has been moved to the center position. For example, there is a moving image or the like displayed in such a way that two items are moved toward each other, overlap each other at an intermediate point, and are separated from each other to be interchanged.

Then, in Step S106, since the variable n is 2, the setting of the operation plane or display control is performed such that the display of a menu in a second layer under the "K4" item, for example, menu display illustrated in Fig. 12C is performed.

That is, the menu control unit 31 of the control unit 3 reads information required to set the operation plane, such as the information of menu selection items in the second layer for the "K4" item and the arrangement of each item, from the menu information storage unit and sets the operation plane in which the origin item CTi and the items Mi are arranged in the operation region AR.

In addition, the control unit 3 supplies the information of the set operation plane of the second layer to the display data generation unit 4 and instructs the display data generation unit 4 to display a screen of the menu in the second layer. The display data generation unit 4 reads the information of items and arrangement on the operation plane in the second layer and character data required to draw, for example, each item Mi and the origin item CTi from the menu information storage unit 32, generates display data of the second layer under the "K4" item, and supplies the display data to the display device 11. For example, the display of the menu in the second layer illustrated in Fig. 12C is performed by these processes.

Then, in Step S107, an operation for the menu in the second layer is detected by similarly to the above- described method.

Next, a case where the "return" item has been selected will be described. In the lower layers, such as the second layer, the third layer, ···, the "return" item is present as one of the items Mi (see Fig. 12C).

At the time of an operation for the menu in the lower layer illustrated in Fig. 12C, in a case where it is detected in Step S107 that the operation position is within the arrangement region of the "return" item, the control unit 3 proceeds in the order of Step S108→Step S110→Step S113→Step S115. In Step S115, the control unit 3 instructs the display data generation unit 4 to perform display illustrated in Fig. 12D as center regression display.

That is, immediately after the operation vector Vc extending from the "K4" item which is the origin item CTi reaches the "return" item as illustrated in Fig. 12C, the K4" item is moved to the position where the "return" item has been arranged (that is, the original position of the "K4" item arranged in the upper layer) and the "return" item is changed to the "start" item so as to be interchanged with the "K4" item and is moved to the center position (origin position), as illustrated in Fig. 12D. In addition, the regression arrow rVc indicating the movement (center regression) is displayed.

In this case, it is desirable that only two items whose positions are instantly interchanged in the operation region AR are displayed or information indicating the movement aspect of each of the two items is displayed.

Then, in Step S106, since the variable n is 1, the setting of the operation plane or display control is performed such that the display of the menu in the first layer, for example, menu display illustrated in Fig. 12A is performed. The "return" item becomes the "start" item.

Then, in Step S107, a detection of an operation for the menu in the first layer is performed by similarly to the above-described method.

The display of the center-regression-type menu and a process corresponding to an operation are performed by the above-mentioned process illustrated in Fig. 11. Fig. 12 illustrates an example of transition to the second layer. Of course, the variable n may be 3 and advance to a menu in a third layer may be performed. In addition, the layer may advance to the lower layer. Of course, the layer may return in response to a return operation.

Then, in a case where the layer advances or returns, the selected item Mi regresses to the center position and becomes the origin item CTi in the transition layer. Therefore, in any layer, the operation direction and the operation distance from the origin position as the starting point by the non-contact operation of the user are reflected in the display of the operation plane and the menu screen.

### <3. First Example of Pie-type Menu>

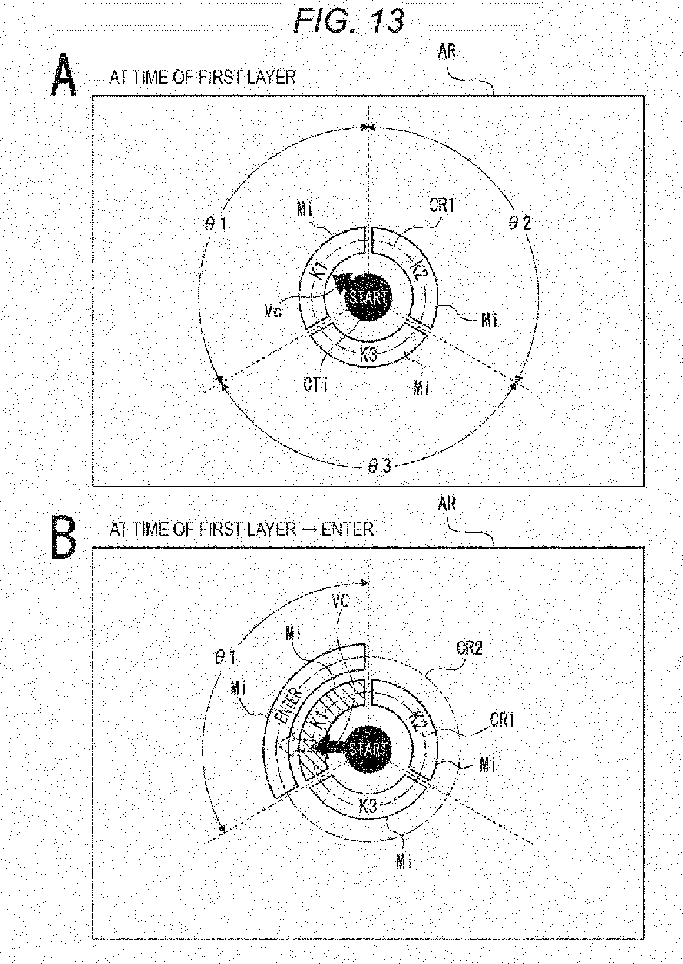

Next, an input operation using the pie-type menu will be described. First, the outline of the operation will be described with reference to Figs. 13, 14, and 15.

Fig. 13A illustrates an initial state indicating the first layer in the pie-type menu.

Note that, first, the user performs an operation of starting the menu mode in order to display the pie-type menu illustrated in Fig. 13A. The menu mode start or end operation may be performed by similarly to the method described in the center-regression-type menu. For example, the control unit 3 may detect a predetermined operation in the z direction and recognize a pie-type menu mode start or end operation.

In addition, after the menu mode starts, display may be changed to the pie-type menu display illustrated in Fig. 13A through a start operation by the initial image described in Fig. 8A or the initial image may be omitted and the pie-type menu display may be started immediately by the menu mode start operation.

Hereinafter, the transition of the menu screen after Fig. 13B will be described as a change in the screen recognized by the user. Each screen is an example of an image displayed on the basis of display data which is generated by the display data generation unit 4 in order to present the operation plane sequentially set by the control unit 3 and the operation direction and the operation distance (an operation position determined by the operation direction and the operation distance) recognized by the control unit 3 to the user.

The process of the control unit 3 sequentially setting the operation plane and the process of specifying the operation position on the basis of the vector information from the vector calculation unit 22 and detecting matching with the arrangement region of the items Mi are similar to those in the case of the center-regression-type menu.

In the menu screen illustrated in Fig. 13A, for example, the origin item CTi represented by "start" is displayed at the origin position. Then, a state in which a plurality of items Mi to be selected are arranged around the origin item CTi at the origin position is displayed. Here, a case where there are three choices on the menu is illustrated. The contents "K1" to "K3" of the items are described in each of the items Mi.

In this example, the items Mi are arranged along the first virtual ring CR1 of the true circle having the origin position as the center which has been described in Fig. 3G.

In order to arrange three items Mi, a circle of 360° having the origin position as the center is divided into three angular ranges, that is, three ranges each of which has an angle of 120° degrees divided by dashed lines. The angular ranges are referred to as angular ranges θ1, θ2, and θ3. Then, the items Mi along a first virtual ring CR1 are arranged so as to substantially cover the angular ranges θ1, θ2, and θ3.

Note that, in this example, the items Mi represented by "K1" to "K3" are slightly separated from each other so as not to come into contact with each other. However, the arrangement regions may be set such that both ends of adjacent items on the first virtual ring come into contact with each other.

The starting point of the operation direction and the operation distance by the non-contact operation of the user in the pie-type menu is basically the origin position similarly to the center-regression-type menu. The origin item CTi is arranged at the origin position at the center of the operation region AR and the operation vector Vc extends from the origin item CTi at the center position as viewed from the user. Therefore, the user can perceive that the center is the starting point of the operation.

The state of the non-contact operation of the user is expressed as the operation vector Vc extending from the origin item CTi similarly to the center-regression-type menu. That is, the control unit 3 associates the operation direction and the operation distance detected as the vector information, using the origin position as the starting point, to calculate certain coordinates in the operation region AR as the operation position. An image in which the operation vector Vc extends from the origin item CTi to the operation position is presented on the display.

The user may perform a non-contact operation while viewing the operation vector Vc such that the operation vector Vc reaches a desired item Mi . The items Mi on the first virtual ring CR1 are arranged around the origin item CTi so as not to overlap each other as viewed from the origin item CTi (origin position). Therefore, for the first layer, the operation vector Vc can directly reach any item Mi (without being disturbed by other items Mi).

For example, in a case where the user performs a non-contact operation such that the operation vector Vc reaches the "K1" item as illustrated in Fig. 13B, the operation content of "K1" is selected. Further, in the pie-type menu, for example, the term "select" indicates that the item Mi is being selected and does not indicate an enter operation.

For example, in a case where a lower layer is not present in the "K1" item, the operation vector Vc reaches the "K1" item and the "K1" item is selected. Then, an "enter" item Mi is displayed on the outer circumferential side of the "K1" item as illustrated in Fig. 13B. The "enter" item is arranged on a second virtual ring CR2 in the same angular range θ1 as the "K1" item that is being selected.

Therefore, the user continuously performs the operation such that the operation vector Vc extends to select the "enter" item (that is, to perform the enter operation).

That is, in a case where the user performed an operation such that the operation vector Vc passes through a certain item Mi that is being selected and reaches the "enter" item on the outer circumferential side of the item Mi, the content of a certain operation is selected and entered.

In addition, selection and entering may be performed at the same time.

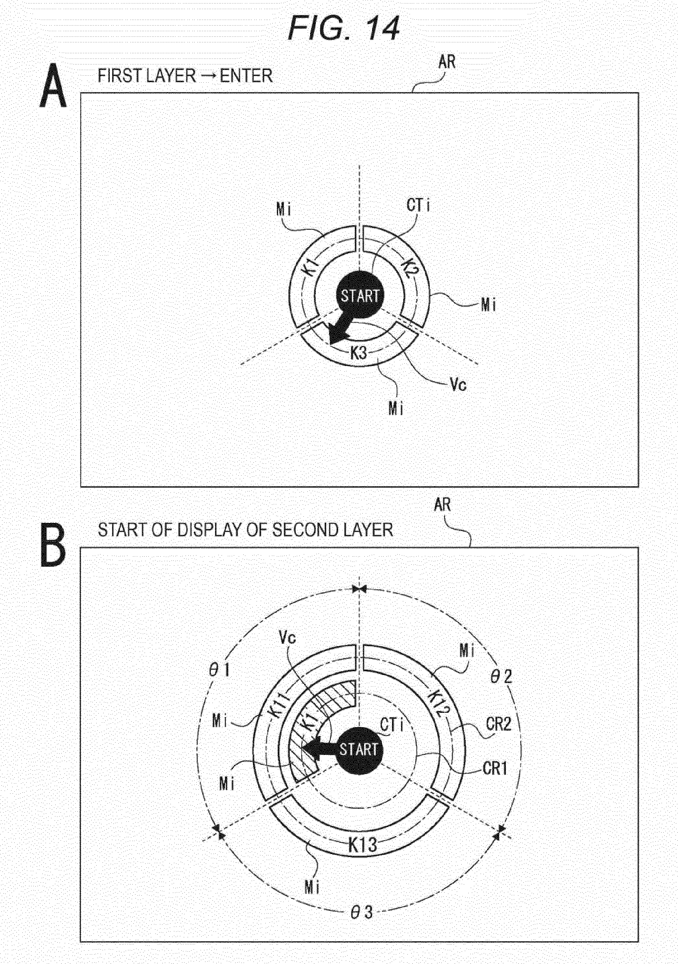

Fig. 14A illustrates a state in which the operation vector Vc reaches a "K3" item. In a case where a lower layer is not present in the "K3" item, selection and entering may be recognized in this state. In this case, it is possible to improve the efficiency of an operation.

In contrast, in a case where the "enter" item is arranged on the outer circumferential side to request an enter operation as illustrated in Fig. 13B, the user clearly recognizes the difference between a case where there is a lower layer, which will be described below, and a case where there is no lower layer. As a result, operability may be improved. In addition, since the "enter" item is arranged immediately on the outer circumferential side of the item Mi that is being selected so as to be adjacent to the item Mi, the enter operation may be performed only by extending the operation distance of the operation body 50. Therefore, the operation is very easy.

It is preferable to select whether to arrange the "enter" item as illustrated in Fig. 13B or to make the selected state also function as the enter operation as illustrated in Fig. 14A in a state in which the item Mi without a lower layer is selected, considering the above.

Alternatively, the user may select any one of the operation methods according to his or her preference.

In a case where an item Mi with a lower layer is selected, items Mi in the lower layer are displayed on the outer circumferential side of the layer.

For example, in a case where the "K1" item is selected from the state illustrated in Fig. 13A, it is assumed that three operation contents "K11", "K12", and "K13" are present as choices in the lower layer of "K1". In this case, as illustrated in Fig. 14B, three items Mi corresponding to the three operation contents are arranged on the outer circumferential side, that is, along the second virtual ring CR2.

Since the angular ranges of the "K11", "K12", and "K13" items Mi are three as in the first layer, the angular range is divided into the angular ranges θ1, θ2, and θ3 and the angular ranges θ1, θ2, and θ3 are arranged. However, the configuration of the lower layer is not necessarily similar to that of the first layer. Of course, in a case where the number of choices is different from that in the first layer, the setting of the angular range is inevitably different from that in the first layer.

Further, in this example, in a case where "K1" is being selected by the operation in the first layer, the "K2" and "K3" items Mi are not displayed on the first virtual ring CR1. This is an illustrative example. The "K2" and "K3" items Mi may be displayed in a light color indicating a so-called inactive portion. Alternatively, the K2" and "K3" items Mi may be displayed without any change.

Since each of the "K2" and "K3" items Mi which have not been selected is not displayed, the user easily performs the operation of selecting each of the "K11", "K12", and "K13" items Mi on the outer circumferential side.

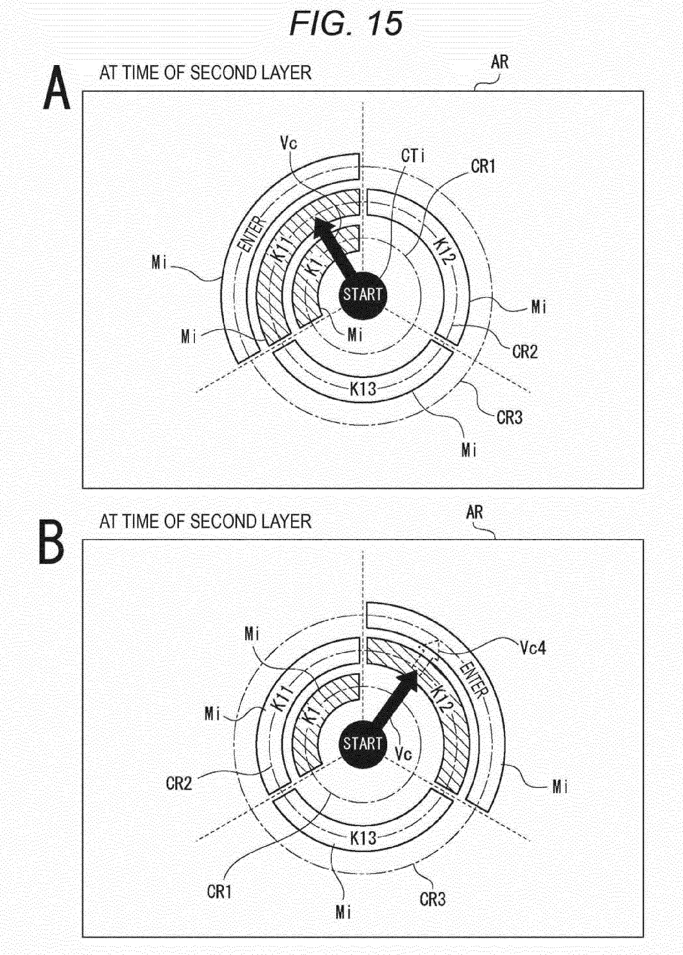

Fig. 15A illustrates a state in which the operation vector Vc further extends from the "K1" item to select the "K11" item. In a case where a lower layer is not present in the "K11" item, the "enter" item Mi is arranged on the outer circumferential side of the "K11" item as illustrated in Fig. 15A. The operation vector Vc extends up to the "enter" item to enter the "K11" item.

Fig. 15B illustrates a state in which the "K1" item is being selected and the operation vector Vc selects the "K12" item. In a case where a lower layer is not present in the "K12" item, the "enter" item Mi is arranged on the outer circumferential side of the "K12" item as illustrated in Fig. 15B. The operation vector Vc extends up to the "enter" item to enter the "K11" item.

In addition, as illustrated in Figs. 13B, 15A, and 15B, the "enter" item Mi may be arranged so as to be adjacent on the outer circumferential side in the same angular range as the item Mi that is being selected. In this case, it is possible to perform the enter operation only by extending the operation vector Vc from the item that is being selected.

Further, in the case of the pie-type menu, the "return" item may not be provided as the item Mi to be selected. For example, the reason is that, in a case where the operation position by the operation vector Vc returns to the inner circumferential side of the selected layer, the selection is canceled and the return of the layer is recognized.

For example, it is assumed that the user performs a non-contact operation such that the operation vector Vc returns to the inner circumferential side and the state illustrated in Fig. 13B returns to the state in which the operation vector Vc does not reach the item Mi in the first layer as illustrated in Fig. 13A. In this case, the state returns to the display state illustrated in Fig. 13A. That is, the selection of the first layer is canceled and the state changes to the display state in which the item Mi in the first layer is required to be selected.

With this configuration, the user can reduce or increase the amount of extension of the operation vector Vc from the origin item CTi at the center to change the layer. Therefore, the efficiency of a menu operation for a plurality of layers is improved.

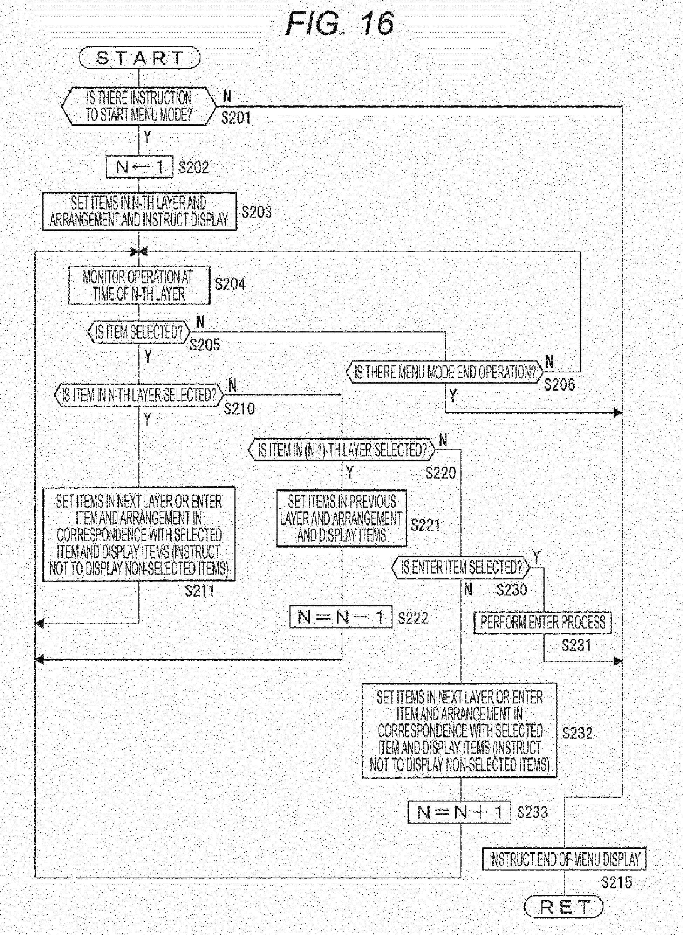

An example of the process of the information processing device 1 for implementing an operation by the pie-type menu will be described with reference to Fig. 16. Fig. 16 illustrates an example of the process of the control unit 3. In addition, for the pie-type menu four process examples will be described. It is assumed that the process example illustrated in Fig. 16 is a first example.

In any of the first to fourth process examples, the control unit 3 sequentially inputs vector information corresponding to a non-contact operation from the operation detection unit 2 and performs menu mode processing (the detection of the operation position and the selected state) . In addition, the control unit 3 transmits, for example, the information of the operation plane set according to the progress of the process to the display data generation unit 4 such that the display data generation unit 4 displays a necessary menu, for example. In addition, the display data generation unit 4 performs a process for displaying the operation vector Vc on the basis of information from the coordinate conversion unit 23.

In Step S201 of Fig. 16, the control unit 3 monitors whether or not a menu mode start operation has been performed.

For example, in a case where the menu mode start operation described in Fig. 7A is detected, the control unit 3 proceeds to Step S202 and sets the variable N indicating the current menu layer to 1.

In addition, the variable N indicates the layer of the item Mi recognized as the current main object to be selected by the control unit 3. Further, the variable N indicates that the operation position (the leading end of the operation vector Vc) is present in an item arrangement region on an N-th virtual ring CR(N) as viewed from the origin position.

Then, in Step S203, the control unit 3 sets an operation plane in an N-th layer. That is, since N is 1 at the beginning, the control unit 3 sets an operation plane in the first layer which is the highest layer.

Specifically, the menu control unit 31 reads information required to set the operation plane, such as the information of menu selection items in the first layer and the arrangement of each item, from the menu information storage unit and sets the operation plane in which the origin item CTi and the items Mi are arranged in the operation region AR.

In addition, the control unit 3 supplies the information of the set operation plane in the first layer to the display data generation unit 4 and instructs the display data generation unit 4 to display a menu screen in the first layer. The display data generation unit 4 reads the information of items and arrangement on the operation plane in the first layer and character data required to draw, for example, each item Mi and the origin item CTi from the menu information storage unit 32, generates display data indicating the items Mi in the first layer, and supplies the display data to the display device 11. For example, the display of the menu in the first layer illustrated in Fig. 13A is performed by these processes .

In Step S204, the control unit 3 processes a menu in the N-th layer.

That is, the control unit 3 reflects the operation direction and the operation distance sequentially recognized as the vector information as a direction and a distance from the origin position of the operation plane set in Step S203 on the operation plane to specify an operation position. Then, the control unit 3 checks whether or not the specified operation position (that is, a position corresponding to the leading end of the operation vector Vc on the display) is matched with the arrangement region (a coordinate range corresponding to a character display range of the item Mi) of a certain item Mi.

In addition, for the period of the monitoring loop process in Steps S204, S205, and S206, the display data generation unit 4 draws the operation vector Vc on the menu image on the basis of the x and y coordinate values converted into display output coordinate values by the coordinate conversion unit 23 and displays the operation vector Vc on the display device 11. In this way, the current operation position recognized by the control unit 3 is presented to the user by the operation vector Vc on the menu image.

The control unit 3 checks whether or not the operation position reaches the arrangement region of any item Mi in Step S204 whenever the vector information is acquired. In a case where the operation position does not reach the arrangement region of any new item Mi, the control unit 3 proceeds from Step S205 to Step S206 and monitors a menu mode end operation. In a case where the menu mode end operation of the user is detected, the control unit 3 proceeds from Step S206 to Step S215 and transmits the end of the menu mode to the display data generation unit 4 to end the display of the menu at that time. Then, the control unit 3 ends the processing of the menu mode.

In addition, in a case where the selection of a new item is not detected for a predetermined period of time or more in Step S204, the control unit 3 may determine that the menu mode has ended in Step S206.

In a case where it is detected that the operation position reaches the arrangement region of a certain item Mi in the process of Step S204, the control unit 3 determines that a new item selection operation has been performed and proceeds from Step S205 to Step S210.

Note that the control unit 3 proceeds from Step S205 to Step S210 in a case where the selected item Mi has been changed. For example, the control unit 3 proceeds from Step S205 to Step S210 in a case where the "K1" item is selected from the state in which no items are selected or in a case where a change from the selected state of the "K1" item to the selected state of the "K2" item is detected. For example, for the period for which the user holds the operation position in the arrangement region of a certain item Mi, a loop process for proceeding from Step S205 to Step S206 is continued.

In Step S210, the control unit 3 checks whether or not a newly selected item Mi is an item in the N-th layer at that time.

In addition, in the case of the pie-type menu, the item having the possibility of being determined to be selected in Steps S204 and S205 is an item Mi in the N-th layer, an item Mi in an (N-1)-th layer, an item Mi in an (N+1)-th layer, or the "enter" item Mi.

Therefore, the control unit 3 branches the process in Steps S210 and S220 in a case where a certain itemMi is selected. That is, in Step S210, the control unit 3 checks whether or not the item is the item Mi in the N-th layer. In addition, in Step S220, the control unit 3 checks whether the item is the item Mi in the (N-1)-th layer or the other items. The other items are the item Mi in the (N+1)-th layer and the "enter" item Mi. This is because the "enter" item Mi is arranged on an (N+1)-th virtual ring CR(N+1) similarly to the item Mi in the (N+1)-th layer as described above.

In a case where the display illustrated in Fig. 13A is performed at the time of N=1, only the items Mi on the first layer are displayed. Therefore, the selected item Mi is an item Mi in the first layer, that is, the N-th layer at that time.

In this case, the control unit 3 proceeds from Step S210 to Step S211 and arranges the items Mi in the next layer or the "enter" item Mi on the operation plane in correspondence with the selected item Mi. In addition, the control unit instructs the display data generation unit 4 to display the items.

That is, in this case, the control unit 3 checks whether or not a lower layer is present in the selected item Mi on the basis of the information of the menu information storage unit 32. In a case where a lower layer is present, the control unit 3 reads the information of lower-layer items (items in the (N+1)-th layer at this time) Mi corresponding to the selected item Mi from the menu information storage 32 and sets the operation plane in which the items are arranged on the (N+1)-th virtual ring CR(N+1). Then, the control unit 3 instructs the display of the operation plane. In this way, for example, the display state illustrated in Fig. 14B is obtained.

In addition, in this case, the control unit 3 may instruct the display data generation unit 4 such that the items Mi which have not been selected in the N-th layer are not displayed as illustrated in Fig. 14B.

Further, in a case where a lower layer is not present in the selected item Mi, the control unit 3 sets the operation plane such that the "enter" item Mi is arranged on the (N+1)-th virtual ring CR(N+1) in the same angular range as the selected item Mi. Then, the control unit 3 instructs the display of the operation plane. In this way, for example, the display state illustrated in Fig. 13B is obtained.

The control unit 3 performs the process in Step S211 as described above, returns to Step S204, and monitors the operation of the user.

For example, it is assumed that, in a state in which the "enter" item Mi is arranged and displayed in response to the selection of the "K1" as illustrated in Fig. 13B, the user performs an operation such that the leading end of the operation vector Vc reaches the "enter" item Mi. In this case, the process of the control unit 3 proceeds in the order of Step S204→Step S205→Step S210→Step S220→Step S230. Since the "enter" item Mi has been selected, an enter process is performed in Step S231.

That is, the control unit 3 stores operation information indicating that an enter operation for the "K1" item has been performed in the operation information storage unit 33 such that the OS or the application program can recognize the content of the operation that has been performed. Then, the control unit 3 instructs the display data generation unit 4 to end the display of the menu in Step S215 and ends the processing of the menu.

Furthermore, for example, it is assumed that, in the state illustrated in Fig. 14B, that is, in a state in which the "K1" item is selected and the "K11", "K12", and "K13" items Mi in the lower layer of the "K1" item are arranged and displayed, the user performs an operation such that the leading end of the operation vector Vc reaches any one of the items Mi of "K11", "K12", and "K13". In this case, the process of the control unit 3 proceeds in the order of Step S204→Step S205→Step S210→Step S220→Step S230. Since the item is not the "enter" item, the control unit 3 proceeds to Step S232.

In Step S232, in response to the selection of an item Mi in a layer (the (N+1)-th layer at the time of N=1) immediately below the current layer, the control unit 3 checks whether or not a lower layer is present in the selected item Mi on the basis of the information of the menu information storage unit 32.

In a case where a lower layer is present, the control unit 3 reads the information of lower-layer items (items in an (N+2) -th layer at the time of N=1) Mi corresponding to the selected item Mi in the (N+1)-th layer from the menu information storage 32 and sets the operation plane in which the items are arranged on an (N+2) -th virtual ring CR(N+2) . Then, the control unit 3 instructs the display of the operation plane. That is, a display state in which choices are further arranged on the outer circumferential side is obtained.

In addition, in this case, the control unit 3 may instruct the display data generation unit 4 such that the items Mi which have not been selected in the N-th layer and the (N+1)-th layer are not displayed.

Further, in a case where a lower layer is not present in the selected item Mi, the control unit 3 sets the operation plane such that the "enter" item Mi is arranged on the (N+2) -th virtual ring CR(N+2) in the same angular range as the selected item Mi. Then, the control unit 3 instructs the display of the operation plane.

In Step S233, the control unit 3 increments the variable N. Then, the control unit 3 returns to Step S204 and monitors the operation of the user.

Since the variable N is incremented, the control unit 3 performs control such that the layer of the item Mi which is being selected at that time is the N-th layer, the (N+1) layer is arranged on the outer circumferential side (that is, the layer of the item Mi arranged on the (N+2) -th virtual ring CR(N+2) in Step S232) of the N-th layer, and the (N-1)-th layer is arranged on the inner circumferential side of the N-th layer and performs the process illustrated in Fig. 16.

Therefore, after the second layer is selected, the user operates the operation vector Vc in a direction in which the operation vector Vc is shortened to select an item Mi in the (N-1)-th layer.

For example, in the state illustrated in Fig. 15A, the user shortens the operation vector Vc such that the leading end of the operation vector Vc is located on the "K1" item.

As such, in a case where the user has selected the item Mi in the (N-1)-th layer at the current variable N, the process of the control unit 3 proceeds in the order of Step S204→Step S205→Step S210→Step S220→Step S221. In Step S221, the control unit 3 sets the operation plane so as to return to the state in which a plurality of items Mi to be selected in the (N-1)-th layer are displayed. For example, the control unit 3 performs a process of returning the state illustrated in Fig. 15A to the state illustrated in Fig. 13A. In addition, in a case where the "K2" item and the "K3" item which have not been selected are not displayed as illustrated in Fig. 15A, the control unit 3 cancels the non-display state and displays the items as choices as illustrated in Fig. 13A.

Then, the control unit 3 decrements the variable N in Step S222 and returns to Step S204. In this way, the control unit 3 returns to the immediately upper layer again and performs the progress.

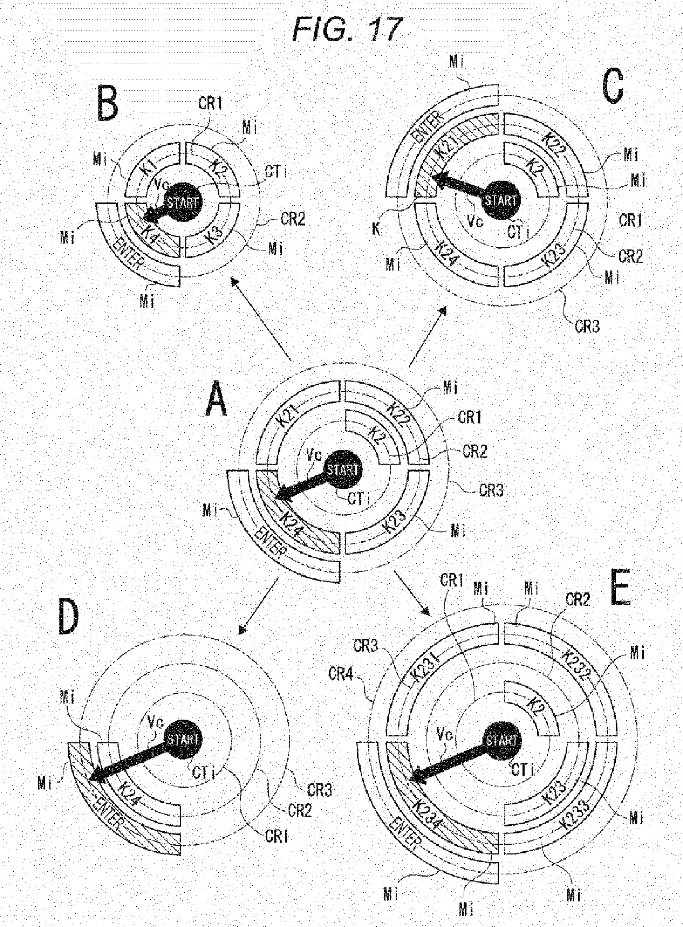

An example of the display transition of the pie-type menu by the above-mentioned process illustrated in Fig. 16 will be described with reference to Fig. 17.

Here, it is assumed that the menu is in a state illustrated in Fig. 17A. This is a case where the control unit 3 sets the variable N to 2 in the process illustrated in Fig. 16. For example, in this state, the "K2" item in the first layer is selected, the "K21", "K22", "K23", and "K24" items Mi in the lower layer of the first layer are displayed, the "K24" item is selected, and the "enter" item Mi is displayed on the outer circumferential side of the "K24" item.

Fig. 17D illustrates a state in which the operation vector Vc further extends from Fig. 17A and reaches the "enter" item Mi. This is a case where the selection of an item is recognized in Step S204 after the state illustrated in Fig. 17A and the process proceeds in the order of Step S204→Step S205→Step S210→Step S220→Step S230→Step S231. The process of an enter operation for the "K24" item selected in Fig. 17A is performed.

Fig. 17C illustrates a case where the operation vector Vc is operated in the state illustrated in Fig. 17A so as to indicate "K21" which is a choice in the same layer. This is a case where the selection of an item is recognized in Step S204 after the state illustrated in Fig. 17A and the process proceeds in the order of Step S204→Step S205→Step S210→Step S211. In a case where a lower layer is not present in the "K21" item, the "enter" item Mi for the "K21" item is displayed as illustrated in Fig. 17C.

Fig. 17B illustrates a state in which the operation vector Vc is operated in the state illustrated in Fig. 17A so as to be shortened and the menu returns to the immediately upper layer. This is a case where the selection of an item in the upper layer is recognized in Step S204 after the state illustrated in Fig. 17A and the process proceeds in the order of Step S204→Step S205→Step S210→Step S220→Step S221.

At that time, the operation vector Vc is shortened and the leading end of the operation vector Vc reaches the range of the "K4" item. In this state, in a case where a lower layer is not present in the"K4" item, the "enter" item Mi for the "K4" item is arranged and displayed.

Note that, for example, in a case where the leading end of the operation vector Vc reaches the inner circumferential side of the item Mi in the first layer, the menu may return to the state illustrated in Fig. 13A, which is not described in detail in the process illustrated in Fig. 16.

Fig. 17E illustrates a state in which the "K23" item is selected from the state illustrated in Fig. 17A, "K231", "K232", "K233", and "K234" items Mi are displayed as choices in the lower layer of the "K23" item, the "K234" item is selected from the state, and the "enter" item Mi for the "K234" item is displayed.

This is a case where the selection of an item is recognized in Step S204 after the state illustrated in Fig. 17A and the process proceeds in the order of Step S204→Step S205→Step S210→Step S220→Step S230→Step S232 and further proceeds in the order of Step S233→Step S204→···→Step S232.

As in the above-mentioned examples, in the case of the pie-type menu, the operation of selecting or entering the item Mi can be performed while the layer is moved up and down according to the operation distance of the operation vector Vc. For these operations, the user may only operate the operation body 50 in the x and y directions.

### <4. Second Example of Pie-type Menu>

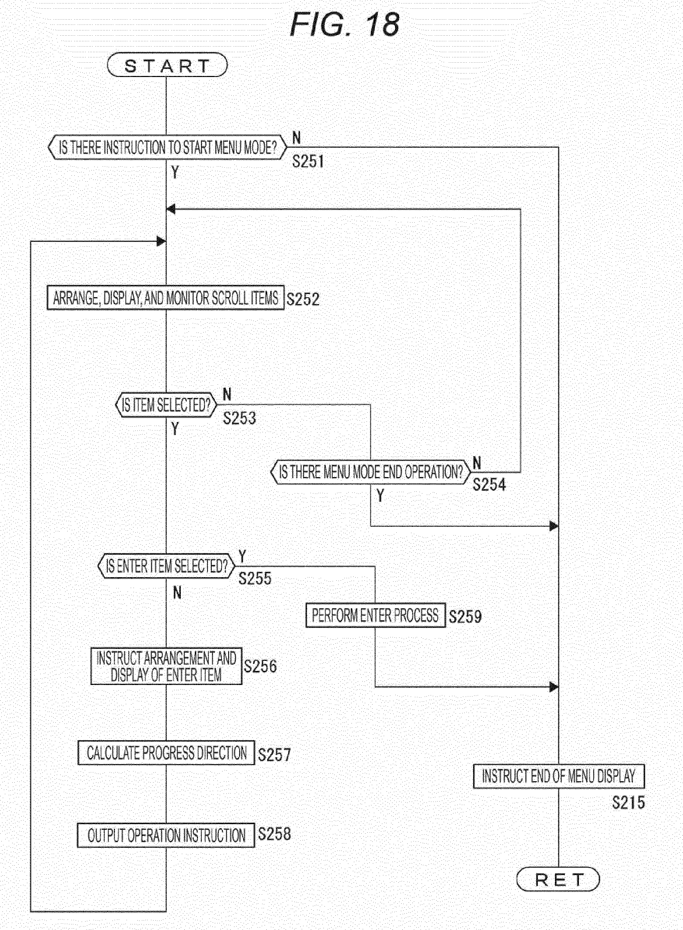

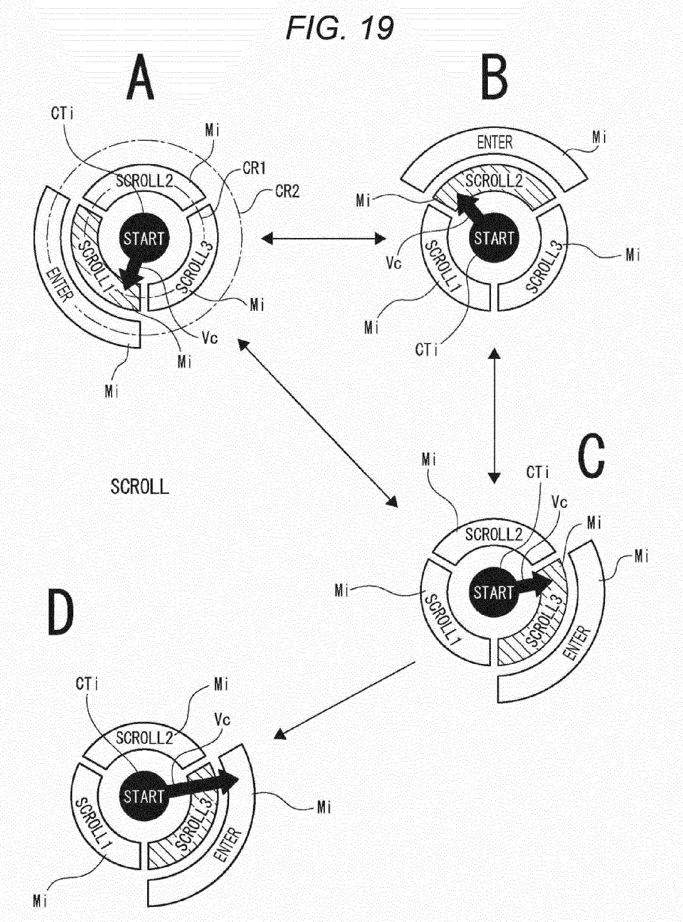

A second example will be described as an example of the processing of the pie-type menu with reference to Figs. 18 and 19. This is an example in which the pie-type menu is used as an interface for a scroll operation.

In Step S251 of Fig. 18, the control unit 3 monitors whether or not a menu mode start operation has been performed.

For example, the control unit 3 proceeds to Step S252 in response to the detection of the menu mode start operation described in Fig. 7A, sets an operation plane for the scroll operation, instructs the display of the operation plane, and monitors a non-contact operation of the user. In addition, the display data generation unit 4 generates display data for displaying the operation vector Vc on the operation plane according to the operation of the user.

Specifically, as illustrated in Fig. 19A, the control unit 3 arranges items Mi represented by "SCROLL" around the origin item CTi. In this example, three "SCROLL" items are arranged. However, for distinction in description, the three "SCROLL" items are referred to as "SCROLL1", "SCROLL2", and "SCROLL3" items.

The description of "SCROLL" in the item Mi is an illustrative example and "SCROLL" may not be described in the item Mi in actual display or other characters or symbols may be described in the item Mi.

The control unit 3 sets the operation plane, instructs the display data generation unit 4 to display the operation plane, and sequentially acquires the operation information of the user.

The user performs a non-contact operation with the user interface for the scroll operation in the example illustrated in Fig. 19 to sequentially select the "SCROLL" items clockwise or counterclockwise in a circular shape, thereby performing a scroll progress/return operation.

For example, the "SCROLL" items are selected in the order of "SCROLL1"→"SCROLL2"→"SCROLL3"→"SCROLL1"··· in the direction of Fig. 19A→Fig. 19B→Fig. 19C→Fig. 19A, that is, in the clockwise direction and scroll progress is instructed step by step.

Conversely, the "SCROLL" items are selected in the order of "SCROLL1"→"SCROLL3"→"SCROLL2"→"SCROLL1"··· in the direction of Fig. 19A→Fig. 19C→Fig. 19B→Fig. 19A, that is, in the counterclockwise direction and scroll return is instructed step by step.

Therefore, in a case where the user performs an operation of slowly rotating the operation position in a circular shape, slow scrolling is performed. In a case where an operation of quickly rotating the operation position in a circular shape is performed, quick scrolling is performed.

In addition, the control unit 3 arranges and displays the "enter" item Mi along the second virtual ring CR2 on the outer circumferential side of a selected "SCROLL" item Mi among a plurality of "SCROLL" items in the same angular range as the selected "SCROLL" item Mi. Therefore, the "enter" item Mi is visually recognized by the user such that the position of the "enter" item Mi is changed according to a scroll operation as illustrated in Figs. 19A, 19B, and 19C.

In a case where an enter operation is performed at the position where scrolling is stopped, the user may perform the operation such that the operation vector Vc reaches the "enter" item Mi as illustrated in Fig. 19D.

In Step S252 of Fig. 18, the control unit 3 determines whether or not the selected item Mi has been changed. Here, for example, the term "change" means that the selected item Mi is changed from the "SCROLL1" item Mi to the "SCROLL2" item Mi, for example.

In addition, with the progress of the process (progress according to the operation of the user whenever the process returns from Step S258 to Step S252), the arrangement and display of the operation plane as illustrated in Figs. 19A, 19B, and 19C are instructed.

Further, the display data generation unit 4 draws the operation vector Vc such that the position of the operation vector Vc is sequentially changed in the display of the operation plane on the basis of the information of the operation position from the coordinate conversion unit 23 to generate display data. Therefore, the direction or length of the operation vector Vc on the screen is changed according to a non-contact operation.

In addition, in a case where the menu mode end operation of the user has been detected, the control unit proceeds from Step S254 to Step S215 and transmits the end of the menu mode to the display data generation unit 4 to end the display of the menu at that time. Then, the control unit ends the processing of the menu mode.

Further, in a case where the selection of a new item is not detected for a predetermined period of time or more in Step S252, the control unit 3 may determine that the menu mode has ended in Step S254.

In a case where a change in the selected item is detected, the control unit 3 proceeds from Step S253 to Step S255 and determines whether or not a newly selected item Mi is the "enter" item Mi.

In a case where the newly selected itemMi is the "SCROLL" item, in Step S256, the control unit 3 instructs the arrangement and display of the "enter" itemMi on the outer circumferential side according to the selected "SCROLL" item.

Then, in Step S257, the control unit 3 calculates a scroll progress direction. The scroll progress direction is calculated by comparison with the previously selected item. That is, in a case where the currently selected item Mi is, for example, "SCROLL2" and the previously selected item Mi is, for example, "SCROLL1", the control unit 3 determines the operation in the progress direction since the operation is a clockwise operation. Conversely, in a case where the currently selected item Mi is, for example, "SCROLL2" and the previously selected item Mi is, for example, "SCROLL3", the control unit 3 determines the operation in the return direction since the operation is a counterclockwise operation.

In Step S258, the control unit 3 outputs an instruction for an operation of "proceeding to the next item" or "returning to the previous item" as the scroll operation according to the determination. In this way, an operation of advancing or returning one step is instructed.

Then, in response to this operation instruction, the OS or the application program performs a scroll operation in the progress direction or the return direction, for example, in the displayed image (an image different from the pie-type menu) .

In a case where the item Mi whose selection has been detected in Step S252 is the "enter" item Mi, the control unit 3 proceeds from Step S255 to Step S259 and performs an enter process.

That is, the control unit 3 stores operation information indicating that an enter operation related to scrolling has been performed in the operation information storage unit 33 such that the content of the operation executed by the OS or the application program can be recognized. For example, as the enter operation related to scrolling, the following are assumed: an operation of entering a choice which is prepared in addition to the pie-type menu and has been selected and determined by scrolling in the pie-type menu (a specific example will be described below in an example of an operating room system) ; an operation of entering a scroll end position; and the like.

Then, in Step S215, the control unit 3 instructs the display data generation unit 4 to end the display of the menu and ends the processing of the menu.

In the process illustrated in Fig. 18, the scroll operation illustrated in Fig. 19 using the pie-type menu can be performed.

Note that, in this example, the "enter" item Mi is arranged such that the enter operation can be performed. However, an example in which the "enter" item Mi is not provided is also considered. That is, a case where only scroll movement is performed is considered.

### <5. Third Example of Pie-type Menu>

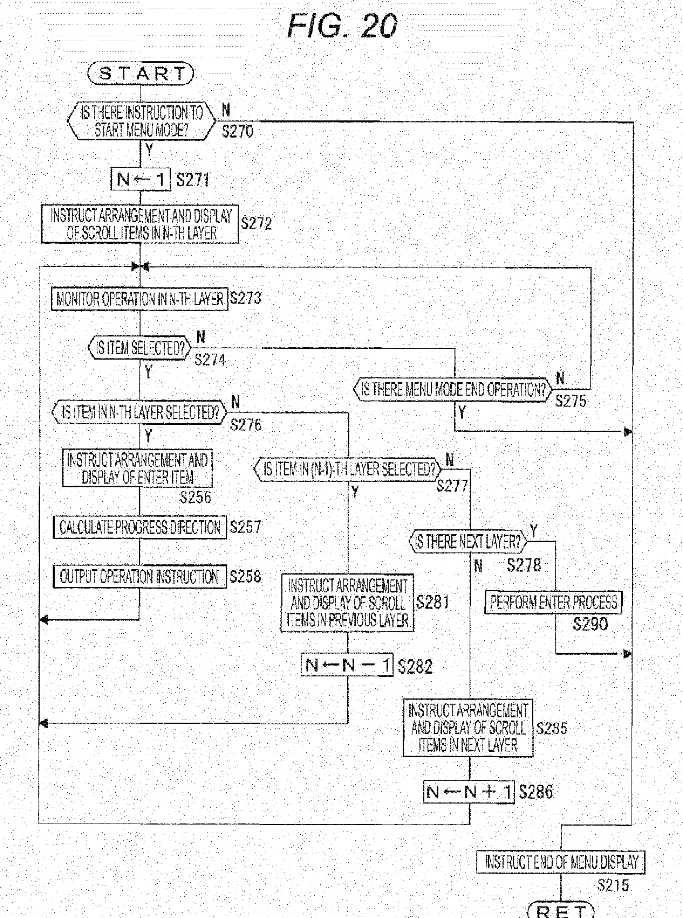

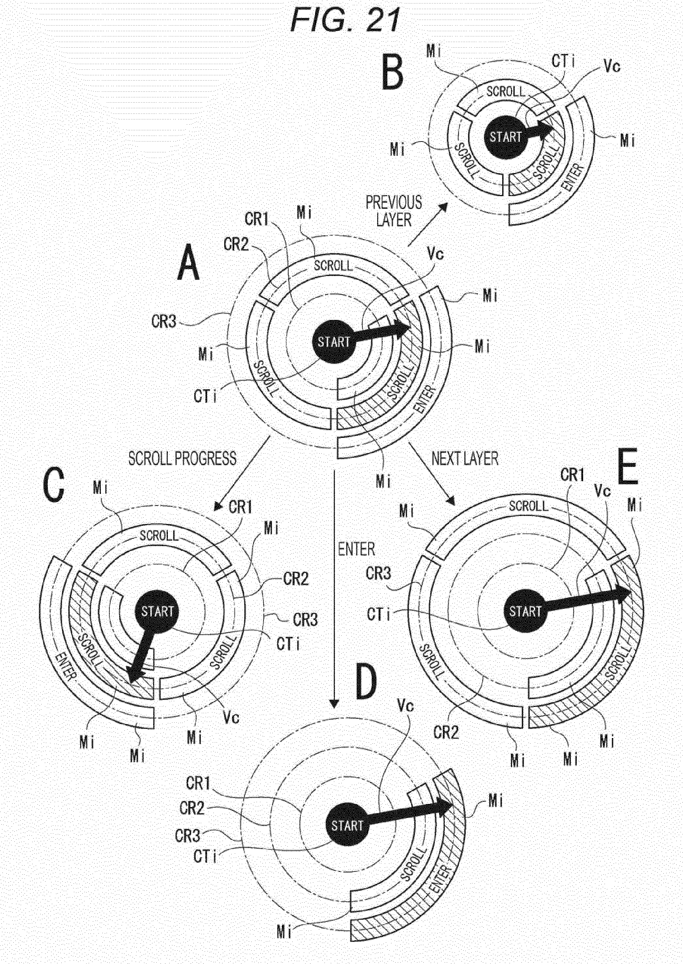

A third example will be described as an example of the processing of the pie-type menu with reference to Figs. 20 and 21. This is an example in which a scroll operation in a plurality of layers is achieved by the pie-type menu.

In Step S270 of Fig. 20, the control unit 3 monitors whether or not a menu mode start operation has been performed.

For example, in a case where the menu mode start operation described in Fig. 7A is detected, the control unit 3 proceeds to Step S271 and sets a variable N indicating the current menu layer (in this case, a scroll operation layer) to 1.

Then, in Step S272, the control unit 3 sets an operation plane in an N-th layer. That is, since N is 1 at the beginning, the control unit 3 sets an operation plane in the first layer which is the highest layer. In addition, the control unit 3 supplies the information of the set operation plane in the first layer to the display data generation unit 4 and instructs the display data generation unit 4 to display a menu screen in the first layer. For example, display for a scroll operation in the first layer illustrated in Fig. 21B is performed by these processes.

In Step S273, the control unit 3 determines whether or not the selected item Mi has been changed. In addition, the control unit 3 instructs the sequential setting and display of the operation plane with the progress of the process.

The direction or length of the operation vector Vc on the screen is changed by the process of the display data generation unit 4 based on the information of the operation position from the coordinate conversion unit 23 according to an operation.

In addition, in a case where the menu mode end operation of the user is detected, the control unit 3 proceeds from Step S275 to Step S215 and transmits the end of the menu mode to the display data generation unit 4 to end the display of the menu at that time. Then, the control unit 3 ends the processing of the menu mode.

In addition, in a case where the selection of a new item is not detected for a predetermined period of time or more in Step S273, the control unit 3 may determine that the menu mode has ended in Step S275.

In a case where the selection of a new item, that is, a change in the selected item is detected, the control unit 3 proceeds from Step S274 to Step S276.

In Step S276, the control unit 3 checks whether or not a newly selected item Mi is an item in the N-th layer at that time.

In the case of the pie-type menu for a scroll operation in multiple layers, the item having the possibility of being determined to be selected in Steps S273 and S274 is an item Mi in the N-th layer, an item Mi in an (N-1)-th layer, or the "enter" item Mi.

Therefore, the control unit 3 branches the process in Steps S276 and S277 in a case where a certain item Mi is selected. That is, in Step S276, the control unit 3 checks whether or not the item is the item Mi in the N-th layer. In addition, in Step S277, the control unit 3 checks whether the item is the item Mi in the (N-1)-th layer or the "enter" item Mi.

In a case where display illustrated in Fig. 21B is performed at the time of N=1 and a new item Mi (that is, an adjacent item Mi) is selected by a scroll operation, an item Mi in the first layer is selected. For example, arrangement and display illustrated in Fig. 21A are performed at the time of N=2. In this case, if a new item Mi (that is, an adjacent "SCROLL" item Mi) is selected by a scroll operation in the second layer, an item Mi in the second layer is selected.

That is, in a case where an adjacent item Mi arranged in a ring shape is selected by a scroll operation, the control unit 3 determines that an item Mi in the N-th layer has been selected and performs the process in Steps S256, S257, and S258.

That is, in Step S256, the control unit 3 instructs the arrangement and display of the "enter" item Mi on the outer circumferential side according to the selected "SCROLL" item.

Then, in Step S257, the control unit 3 calculates a scroll progress direction. Then, in Step S258, the control unit 3 outputs an instruction for an operation of "progressing to the next item" or an operation of "returning to the previous item" as the scroll operation according to whether the scroll progress direction is the clockwise direction or the counterclockwise direction. In this way, an operation of advancing or returning one step is instructed and a scroll operation in the progress direction or the return direction is performed by, for example, an application program.

For example, in a case where the user performs an operation such that the leading end of the operation vector Vc reaches the "enter" item Mi in the display state of the first layer as illustrated in Fig. 21B, the process of the control unit 3 proceeds in the order of Step S273→Step S274→Step S276→Step S277→Step S278 and checks whether or not the next layer is present for a scroll operation.

This is checking whether or not a lower layer is present in another choice which is prepared in addition to the pie-type menu and has been selected by a scroll operation for the pie-type menu.

In a case where a lower layer is not present, the control unit 3 performs an enter process in Step S290. That is, the control unit 3 stores operation information indicating that an enter operation has been performed for a choice which is prepared in addition to the pie-type menu and has been selected and entered in the operation information storage unit 33 such that the content of the operation performed by the OS or the application program can be recognized. Then, in Step S215, the control unit 3 instructs the display data generation unit 4 to end the display of the menu and ends the processing of the menu.

In contrast, in a case where a lower layer is present in the choice subjected to the enter operation, the control unit 3 arranges "SCROLL" items in the next layer on the operation plane in Step S285. In addition, the control unit transmits the information of the rearranged operation plane to the display data generation unit 4 and instructs the display data generation unit 4 to perform a display process. At the time of N=1, for example, a display state is changed from Fig. 21B as illustrated in Fig. 21A.

In addition, in the example illustrated in Fig. 21A, among the "SCROLL" items in the first layer, only a "SCROLL" item in a case where the "enter" itemMi is operated is displayed and the other two "SCROLL" items are not displayed. Therefore, a shift from a scroll operation in the first layer to an operation in the second layer is expressed.

Then, the control unit 3 increments the variable N in response to advance to the lower layer in Step S286 and returns to the monitoring loop of Steps S273, S274, and S275.

In a case where the user performs a scroll operation in the state illustrated in Fig. 21A in which N is 2, the process in Steps S256, S257, and S258 is performed to instruct scrolling step by step while the selected "SCROLL" item is changed as illustrated in Fig. 21C.

In addition, in a case where the user performs an operation of selecting the "enter" item Mi in the state illustrated in Fig. 21A in which N is 2, if a lower layer is present, the process ends in the state illustrated in Fig. 21D and an enter process is performed in Step S290. Further, in a case where a lower layer is present, the operation plane is arranged in Step S285 and a scroll operation screen in the lower layer is displayed as illustrated in Fig. 21E.

In a case where the variable N is equal to or greater than 2, the selected item may be an item in the upper layer. That is, it may be determined that an item in the (N-1)-th layer is selected in Step S277.

In this case, the control unit 3 arranges the "SCROLL" items in the upper layer and the "enter" item on the operation plane in Step S281. That is, the control unit 3 rearranges the items such that an arrangement state returns. Then, the control unit 3 transmits the information of the rearranged operation plane to the display data generation unit 4 and instructs the display data generation unit 4 to perform a display process. At the time of N=2, for example, a display state is changed from Fig. 21A as illustrated in Fig. 21B.

Then, the control unit 3 decrements the variable N in response to return to the upper layer in Step S282 and returns to the monitoring loop of Steps S273, S274, and S275.

A scroll operation can be performed in a plurality of layers using the pie-type menu by the process example illustrated in Fig. 20.

### <6. Fourth Example of Pie-type Menu>

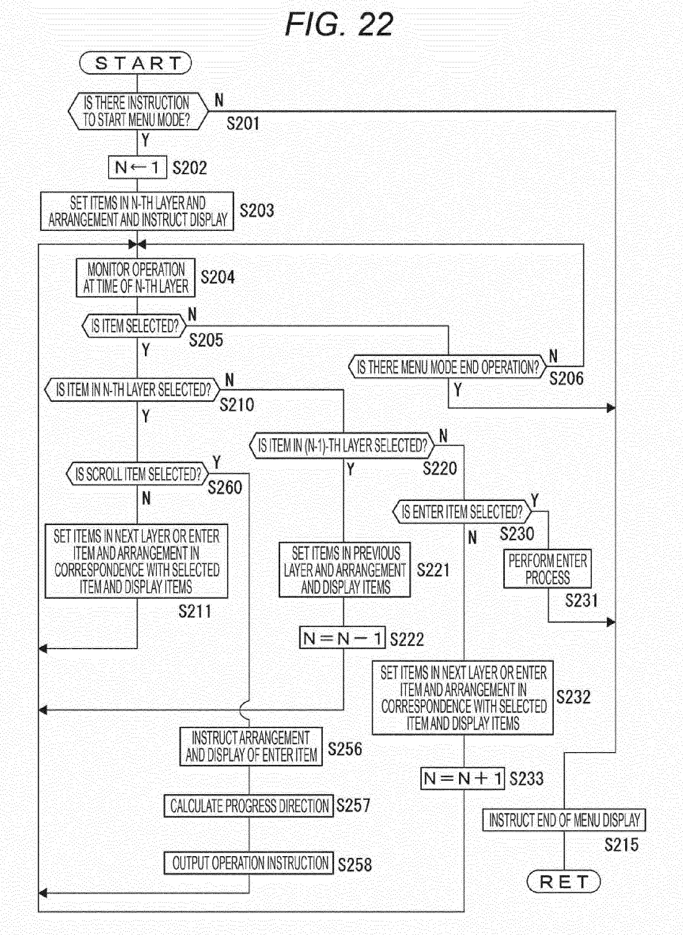

A fourth example will be described as an example of the processing of the pie-type menu with reference to Fig. 22. This is an example in which a scroll operation can be performed in a certain layer within a certain selected item in the item selection operation (first example) using the pie type menu described in Figs. 16 and 17.

In addition, in Fig. 22, the same processes as those in Figs. 16 and 18 are denoted by the same step numbers and the description thereof will not be repeated.

Steps S201 to S233 are the same as those in Fig. 16. However, in a case where the operation plane is arranged in Steps S203, S211, S222, and S232, an item in the layer to be displayed may be the "SCROLL" item.

In Fig. 22, in a case where an item Mi in the N-th layer is determined to be selected in Step S210, the process is branched on the basis of whether or not the selected item is the "SCROLL" item in Step S260.

Then, in a case where the "SCROLL" item is selected, the control unit 3 performs the process (see Fig. 18) in Steps S256, S257, and S258 to instruct scrolling and to progress the screen.

In the process of selecting items in a plurality of layers, a scroll operation can be applied to a certain item by the process illustrated in Fig. 22. A specific example of this configuration will be described in the following application example to the operating room system.

### <7. Example of Application to Operating Room System>

A technique according to the present disclosure can be applied to various products. For example, the technique according to the present disclosure may be applied to the operating room system.

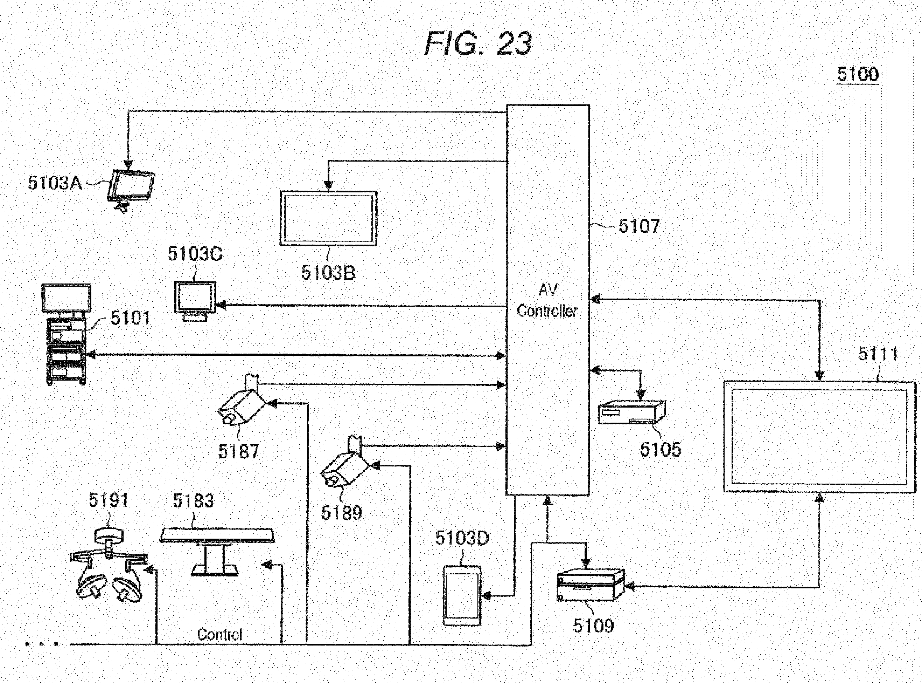

Fig. 23 is a diagram schematically illustrating the overall configuration of an operating room system 5100 to which the technique according to the present disclosure can be applied. Referring to Fig. 23, the operating room system 5100 is configured by connecting device groups installed in an operating room through an audiovisual controller (AV Controller) 5107 and an operating room control device 5109 so as to cooperate with each other.

Various devices are installed in the operating room. For example, Fig. 23 illustrates various device groups 5101 for endoscopic surgery, a ceiling camera 5187 that is provided on the ceiling of the operating room and captures an image of the operator's hands, an operative field camera 5189 that is provided on the ceiling of the operating room and captures an aspect of the entire operating room, a plurality of display devices 5103A to 5103D, a recorder 5105, a patient bed 5183, and a lighting device 5191.

Here, among these devices, the device group 5101 belongs to an endoscopic surgery system 5113 which will be described below and include, for example, an endoscope and a display device that displays images captured by the endoscope. Each device belonging to the endoscopic surgery system 5113 is also referred to as a medical device. The display devices 5103A to 5103D, the recorder 5105, the patient bed 5183, and the lighting device 5191 are provided, for example, in the operating room separately from the endoscopic surgery system 5113. Each device that does not belong to the endoscopic surgery system 5113 is also referred to as a non-medical device . The audiovisual controller 5107 and/or the operating room control device 5109 controls the operations of the medical devices and the non-medical devices so as to cooperate with each other.

The audiovisual controller 5107 controls a process related to the display of images in the medical devices and the non-medical devices in a comprehensive manner. Specifically, among the devices of the operating room system 5100, the device group 5101, the ceiling camera 5187, and the operative field camera 5189 may be devices (hereinafter, also referred to as transmission source devices) having a function of transmitting information to be displayed during the operation (hereinafter, also referred to as display information) . In addition, the display devices 5103A to 5103D may be devices (hereinafter, also referred to as output destination devices) to which the display information is output. Further, the recorder 5105 may be a device corresponding to both the transmission source device and the output destination device. The audiovisual controller 5107 has a function of controlling the operation of the transmission source device and the output destination device such that the display information is acquired from the transmission source device and the display information is transmitted to the output destination device and is displayed or recorded. In addition, examples of the display information include various images captured during the operation and various kinds of information related to the operation (for example, the body information of a patient, past examination results, information related to an operative procedure, and the like).

Specifically, information related to the image of an operative site in the body cavity of a patient which is captured by the endoscope can be transmitted as the display information from the device group 5101 to the audiovisual controller 5107 . In addition, the ceiling camera 5187 can transmit, as the display information, information related to the image of the operator's hands captured by the ceiling camera 5187. Further, the operative field camera 5189 can transmit, as the display information, information related to an image indicating the aspect of the entire operating room captured by the operative field camera 5189. Furthermore, in a case where another device having an imaging function is present in the operating room system 5100, the audiovisual controller 5107 may acquire, as the display information, information related to an image captured by the device from the device.

Alternatively, for example, the audiovisual controller 5107 records information related to the images captured in the past on the recorder 5105. The audiovisual controller 5107 can acquire, as the display information, the information related to the images captured in the past from the recorder 5105. In addition, various kinds of information related to an operation may be recorded on the recorder 5105 in advance.

The audiovisual controller 5107 directs any one of the display devices 5103A to 5103D which are output destination devices to display the acquired display information (that is, the images captured during the operation or various kinds of information related to the operation). In the example illustrated in Fig. 23, the display device 5103A is a display device that is suspended from the ceiling of the operating room, the display device 5103B is a display device that is provided on a wall surface of the operating room, the display device 5103C is a display device that is provided on a desk in the operating room, and the display device 5103D is a mobile device (for example, a tablet personal computer (PC)) having a display function.

In addition, the operating room system 5100 may include a device outside the operating room, which is not illustrated in Fig. 23. The device outside the operating room may be, for example, a server that is connected to a network constructed inside and outside the hospital, a PC that is used by medical staff, a projector that is provided in a conference room of the hospital, or the like. In a case where the external device is provided outside the hospital, the audiovisual controller 5107 may display the display information on a display device of other hospitals through, for example, a video conference system for remote medical care.

The operating room control device 5109 controls processes other than the process related to the display of images in the non-medical device in a comprehensive manner. For example, the operating room control device 5109 controls the driving of the patient bed 5183, the ceiling camera 5187, the operative field camera 5189, and the lighting device 5191.

The operating room system 5100 is provided with a centralized operation panel 5111. The user can input an image display instruction to the audiovisual controller 5107 through the centralized operation panel 5111 or can input an instruction for the operation of the non-medical device to the operating room control device 5109. The centralized operation panel 5111 is configured by providing a touch panel on a display surface of a display device.

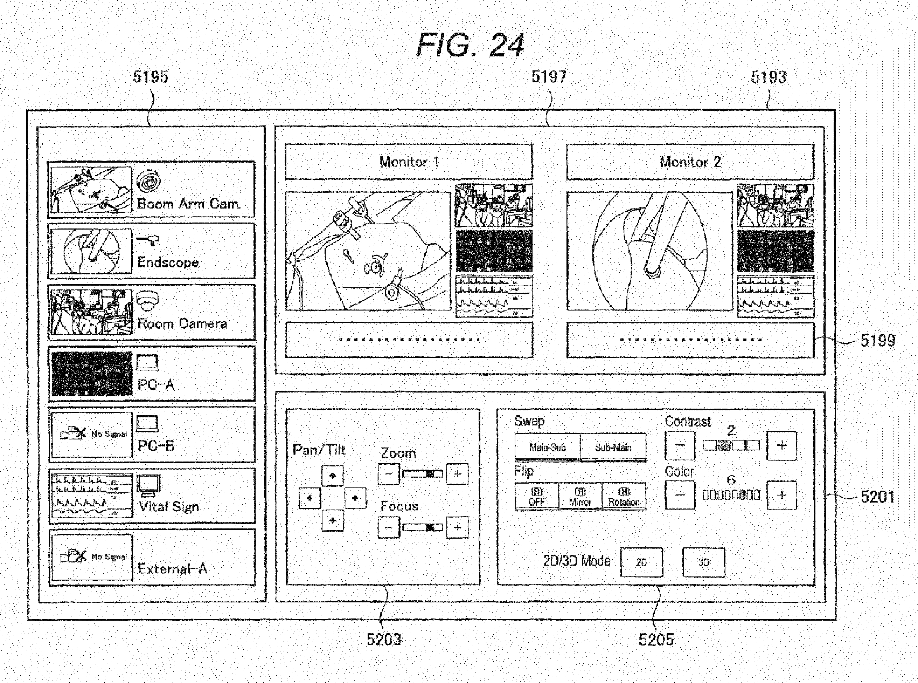

Fig. 24 is a diagram illustrating an example of the display of an operation screen of the centralized operation panel 5111. Fig. 24 illustrates, for example, an operation screen corresponding to a case where two display devices are provided as the output destination devices in the operating room system 5100. Referring to Fig. 24, an operation screen 5193 is provided with a transmission source selection region 5195, a preview region 5197, and a control region 5201.

The transmission source devices provided in the operating room system 5100 and thumbnail screens indicating the display information of the transmission source devices are displayed in transmission source selection region 5195 so as to be associated with each other. The user can select display information desired to be displayed on the display device from any one of the transmission source devices displayed in the transmission source selection region 5195.

Previews of the screens displayed on two display devices (Monitor 1 and Monitor 2) which are the output destination devices are displayed in the preview region 5197. In the example illustrated in Fig. 24, four images are displayed on one display device in a PinP manner . The four images correspond to the display information transmitted from the transmission source devices selected in the transmission source selection region 5195. One of the four images is displayed as a main image with a relatively large size and the remaining three images are displayed as sub-images with a relatively small size. The user can appropriately select the regions in which the four images are displayed to interchange the main image and the sub images. In addition, a status display region 5199 is provided below the regions in which the four images are displayed. A status (for example, the elapsed time of the operation, the body information of the patient, and the like) related to the operation can be appropriately displayed in the region.

The control region 5201 is provided with a transmission source operation region 5203 in which a graphical user interface (GUI) component for performing an operation for the transmission source device and an output destination operation region 5205 in which a GUI component for performing an operation for the output destination device. In the example illustrated in Fig. 24, the transmission source operation region 5203 is provided with GUI components for performing various operations (pan, tilt, and zoom) for a camera in the transmission source device having an imaging function. The user can appropriately select the GUI components to operate the camera in the transmission source device. In addition, in a case where the transmission source device selected in the transmission source selection region 5195 is the recorder (that is, in a case where the image recorded on the recorder in the past is displayed in the preview region 5197), GUI components for performing operations, such as image reproduction, reproduction stop, rewinding, and fast forwarding, can be provided in the transmission source operation region 5203, which is not illustrated in Fig. 24.

In addition, the output destination operation region 5205 is provided with a GUI component for performing various operations (swap, flip, color adjustment, contrast adjustment, and switching between 2D display and 3D display) for the display of the display device which is the output destination device. The user can appropriately select the GUI components to operate the display of the display device.

In addition, the operation screen displayed in the centralized operation panel 5111 is not limited to the example illustrated in Fig. 24. The user can input an operation to each device that is provided in the operating room system 5100 and can be controlled by the audiovisual controller 5107 and the operating room control device 5109 through the centralized operation panel 5111.

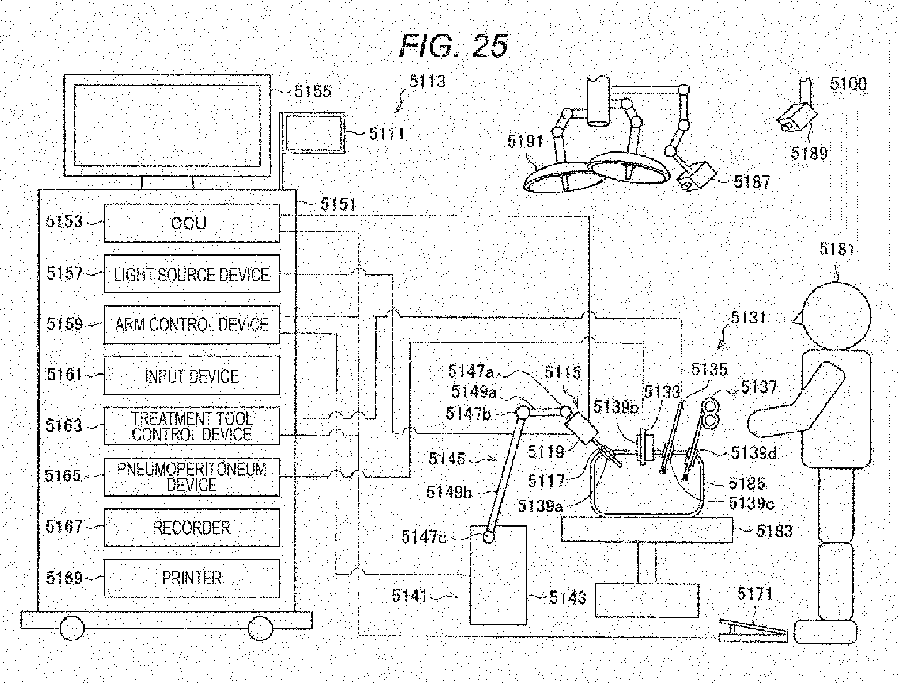

Fig. 25 is a diagram illustrating an example of the aspect of an operation to which the above-mentioned operating room system is applied. The ceiling camera 5187 and the operative field camera 5189 are provided on the ceiling of the operating room and can capture an image of the hands of an operator (doctor) 5181 who performs a treatment on an affected part of a patient 5185 on the patient bed 5183 and an image of the aspect of the entire operating room. The ceiling camera 5187 and the operative field camera 5189 can have, for example, a magnification adjustment function, a focal length adjustment function, and an imaging direction adjustment function. The lighting device 5191 is provided on the ceiling of the operating room and irradiates at least the hands of the operator 5181. The lighting device 5191 may be configured such that, for example, the amount of light emitted from the lighting device 5191, the wavelength (light) of irradiation light, and the irradiation direction of light can be appropriately adjusted.

As illustrated in Fig. 23, the endoscopic surgery system 5113, the patient bed 5183, the ceiling camera 5187, the operative field camera 5189, and the lighting device 5191 are connected through the audiovisual controller 5107 and the operating room control device 5109 (not illustrated in Fig. 25) so as to be associated with each other. The centralized operation panel 5111 is provided in the operating room. As described above, the user can appropriately operate these devices in the operating room through the centralized operation panel 5111.

Hereinafter, the configuration of the endoscopic surgery system 5113 will be described detail. As illustrated in Fig. 25, the endoscopic surgery system 5113 includes an endoscope 5115, other surgical tools 5131, a support arm device 5141 that supports the endoscope 5115, and a cart 5151 provided with various devices for endoscopic surgery.

In endoscopic surgery, a plurality of cylindrical opening tools which are called trocars 5139a to 5139d are inserted into the abdominal wall, instead of cutting the abdominal wall and opening the abdomen. Then, a lens barrel 5117 of the endoscope 5115 and other surgical tools 5131 are inserted into the body cavity of the patient 5185 through the trocars 5139a to 5139d. In the example illustrated in Fig. 25, as other surgical tools 5131, an abdominal tube 5133, an energy treatment tool 5135, and forceps 5137 are inserted into the body cavity of the patient 5185. In addition, the energy treatment tool 5135 is, for example, a treatment tool for incising and peeling tissues or sealing blood vessels using a high-frequency current or ultrasonic vibration. However, the surgical tools 5131 illustrated in Fig. 25 are illustrative examples. Various types of surgical tools generally used in endoscopic surgery, such as tweezers and a retractor, may be used as the surgical tools 5131.

The image of an operative site in the body cavity of the patient 5185 which has been captured by the endoscope 5115 is displayed on a display device 5155. The operator 5181 performs, for example, a treatment for excising the affected part using the energy treatment tool 5135 or the forceps 5137 while viewing the image of the operative site displayed on the display device 5155 in real time. In addition, the abdominal tube 5133, the energy treatment tool 5135, and the forceps 5137 are supported by, for example, the operator 5181 or an assistant during the operation, which is not illustrated in the drawings.

### (Support Arm Device)

The support arm device 5141 includes an arm portion 5145 extending from a base portion 5143. In the example illustrated in Fig. 25, the arm portion 5145 includes joint portions 5147a, 5147b, and 5147c and links 5149a and 5149b and is driven under the control of an arm control device 5159. The endoscope 5115 is supported by the arm portion 5145 and the position and posture of the endoscope 5115 are controlled by the arm portion 5145. In this way, the position of the endoscope 5115 can be stably fixed.

### (Endoscope)

The endoscope 5115 includes the lens barrel 5117 having a region which has a predetermined length from a leading end and is inserted into the body cavity of the patient 5185 and a camera head 5119 connected to a base end of the lens barrel 5117. In the example illustrated in Fig. 25, the endoscope 5115 which is a so-called rigid scope having the hard lens barrel 5117 is illustrated. However, the endoscope 5115 may be a so-called flexible scope having the soft lens barrel 5117.

An opening portion to which an objective lens is fitted is provided at the leading end of the lens barrel 5117. A light source device 5157 is connected to the endoscope 5115. Light generated by the light source device 5157 is guided to the leading end of the lens barrel by a light guide that extends in the lens barrel 5117 and is emitted to an observation target in the body cavity of the patient 5185 through the objective lens. In addition, the endoscope 5115 may be a forward-viewing endoscope, an oblique-viewing endoscope, or a side-viewing endoscope.

An optical system and an imaging element are provided in the camera head 5119. Light (observation light) reflected from the observation target is focused on the imaging element by the optical system. The imaging element performs photoelectric conversion for the observation light to generate an electric signal corresponding to the observation light, that is, an image signal corresponding to an observation image. The image signal is transmitted as RAW data to a camera control unit (CCU) 5153. In addition, the camera head 5119 has a function of appropriately driving the optical system to adjust magnification and a focal length.

Further, for example, the camera head 5119 may be provided with a plurality of imaging elements in order to respond to, for example, a stereoscopic view (3D display). In this case, a plurality of relay optical systems are provided in the lens barrel 5117 in order to guide the observation light to each of the plurality of imaging elements.

### (Various Devices Provided in Cart)

The CCU 5153 is, for example, a central processing unit (CPU) or a graphics processing unit (GPU) and controls the overall operation of the endoscope 5115 and the display device 5155. Specifically, the CCU 5153 performs various types of image processing for displaying an image based on the image signal received from the camera head 5119, such as a development process (demosaicing process), for the image signal. The CCU 5153 provides the image signal subjected to the image processing to the display device 5155. In addition, the audiovisual controller 5107 illustrated in Fig. 23 is connected to the CCU 5153. The CCU 5153 also provides the image signal subjected to the image processing to the audiovisual controller 5107. Further, the CCU 5153 transmits a control signal to the camera head 5119 to control the driving of the camera head 5119. The control signal may include information related to imaging conditions such as magnification and a focal length. The information related to the imaging conditions may be input through an input device 5161 or may be input through the above-described centralized operation panel 5111.

The display device 5155 displays the image based on the image signal subjected to the image processing by the CCU 5153 under the control of the CCU 5153. In a case where the endoscope 5115 corresponds to high-resolution imaging, such as 4K (the number of horizontal pixels 3840 x the number of vertical pixels 2160) or 8K (the number of horizontal pixels 7680 x the number of vertical pixels 4320) imaging and/or in a case where the endoscope 5115 corresponds to 3D display, a display device that can display a high-resolution image and/or a display device that can display a 3D image can be used as the display device 5155 in correspondence with each of the cases. In a case where the display device 5155 corresponds to high-resolution imaging, such as 4K or 8K imaging, a display device having a size of 55 inches or more is used as the display device 5155 to obtain a high sense of immersion. In addition, a plurality of display devices 5155 which are different in resolution and size may be provided according to purposes.

The light source device 5157 is a light source, such as a light emitting diode (LED), and supplies irradiation light to the endoscope 5115 in a case where an image of an operative site is captured.

The arm control device 5159 is a processor, such as a CPU, operates according to a predetermined program, and controls the driving of the arm portion 5145 of the support arm device 5141 according to a predetermined control method.

The input device 5161 is an input interface to the endoscopic surgery system 5113. The user can input various kinds of information or instructions to the endoscopic surgery system 5113 through the input device 5161. For example, the user inputs various kinds of information related to an operation, such as the body information of a patient and information related to an operative procedure, through the input device 5161. In addition, the user inputs, for example, an instruction to drive the arm portion 5145, an instruction to change the imaging conditions (for example, the type of irradiation light, magnification, and a focal length) of the endoscope 5115, an instruction to drive the energy treatment tool 5135 through the input device 5161, and the like.

The type of the input device 5161 is not limited and the input device 5161 may be various known input devices. For example, a mouse, a keyboard, a touch panel, a switch, a foot switch 5171 and/or a lever can be applied as the input device 5161. In a case where a touch panel is used as the input device 5161, the touch panel may be provided on a display surface of the display device 5155.

Alternatively, the input device 5161 is a device worn by the user, such as a glasses-type wearable device or a head mount display (HMD), for example, and various kinds of information are input according to the gesture or viewing direction of the user detected by the device. In addition, the input device 5161 includes a camera that can detect the movement of the user and various kinds of information are input according to the gesture or viewing direction of the user detected from a video captured by the camera. Further, the input device 5161 includes a microphone that can pick up the user's voice and various kinds of information are input by a voice through the microphone. As such, since the input device 5161 is configured to input various kinds of information in a non-contact manner, particularly, the user (for example, the operator 5181) in a clean area can operate devices in an unclean area in a non-contact manner. In addition, since the user can operate the device, without taking his or her hands off the surgical tool being held, the convenience of the user is improved.

A treatment tool control device 5163 controls the driving of the energy treatment tool 5135 for cauterizing and incising tissues or sealing the blood vessels, for example. A pneumoperitoneum device 5165 sends gas into the body cavity of the patient 5185 through the abdominal tube 5133 in order to expand the body cavity of the patient 5185 to ensure the visual field of the endoscope 5115 and the working space of the operator. A recorder 5167 is a device that can record various kinds of information related to an operation. A printer 5169 is a device that can print various kinds of information related to an operation in various formats, such as text, an image, or a graph.

Hereinafter, a particularly characteristic configuration in the endoscopic surgery system 5113 will be described in detail.

### (Support Arm Device)

The support arm device 5141 includes the base portion 5143 which is a base and the arm portion 5145 extending from the base portion 5143. In the example illustrated in Fig. 25, the armportion 5145 includes a plurality of joint portions 5147a, 5147b, and 5147c and a plurality of links 5149a and 5149b connected by the joint portion 5147b. In Fig. 25, for simplicity, the configuration of the armportion 5145 is simply illustrated. In practice, for example, the shape, number, and arrangement of the joint portions 5147a to 5147c and the links 5149a and 5149b and the direction of the rotation axis of the joint portions 5147a to 5147c can be appropriately set such that the arm portion 5145 has a desired degree of freedom. For example, the arm portion 5145 may be preferably configured so as to have six or more degrees of freedom. In this case, it is possible to freely move the endoscope 5115 in the movable range of the arm portion 5145. Therefore, it is possible to insert the lens barrel 5117 of the endoscope 5115 into the body cavity of the patient 5185 from a desired direction.

Actuators are provided in the joint portions 5147a to 5147c and the joint portions 5147a to 5147c are configured so as to be rotatable about a predetermined rotation axis by the driving of the actuators. The arm control device 5159 controls the driving of the actuators to control the rotation angle of each of the joint portions 5147a to 5147c, thereby controlling the driving of the arm portion 5145. In this way, the position and posture of the endoscope 5115 can be controlled. At that time, the arm control device 5159 can control the driving of the arm portion 5145 using various known control methods such as force control or position control.

For example, in a case where the operator 5181 appropriately inputs an operation through the input device 5161 (including a foot switch 5171), the arm control device 5159 may appropriately control the driving of the arm portion 5145 in response to the input of the operation such that the position and posture of the endoscope 5115 are controlled. The control process makes it possible to move the endoscope 5115 at the leading end of the arm portion 5145 from an arbitrary position to an arbitrary position and then to fixedly support the endoscope 5115 at the moved position. In addition, the arm portion 5145 may be operated by a so-called master-slave method. In this case, the arm portion 5145 can be remotely operated by the user through the input device 5161 that is provided in a place separated from the operating room.

Further, in a case where force control is applied, the arm control device 5159 may perform so-called power assist control for driving the actuators of the joint portions 5147a to 5147c such that the arm portion 5145 is smoothly moved by external force received from the user. In this configuration, in a case where the user moves the arm portion 5145 while directly coming into contact with the arm portion 5145, it is possible to move the arm portion 5145 with relatively weak force. Therefore, the endoscope 5115 can be moved more intuitively by a simpler operation and the convenience of the user can be improved.

Here, in endoscopic surgery, the endoscope 5115 is generally supported by a doctor called a scopist. Incontrast, the use of the support arm device 5141 makes it possible to reliably fix the position of the endoscope 5115 without manual intervention. Therefore, it is possible to stably obtain the image of an operative site and to smoothly perform an operation.

In addition, the arm control device 5159 may not be necessarily provided in the cart 5151. Further, the arm control device 5159 may not be necessarily one device. For example, the arm control device 5159 may be provided in each of the joint portions 5147a to 5147c of the arm portion 5145 in the support arm device 5141 and a plurality of arm control devices 5159 may control the driving of the arm portion 5145 in cooperation with each other.

### (Light Source Device)

The light source device 5157 supplies irradiation light to the endoscope 5115 in a case where the image of the operative site is captured. The light source device 5157 is, for example, a white light source configured by an LED, a laser light source, or a combination thereof. In a case where the white light source is configured by a combination of R, G, and B laser light sources, it is possible to control the output intensity and output timing of each color (each wavelength) with high accuracy. Therefore, it is possible to adjust the white balance of a captured image in the light source device 5157. In addition, in this case, an observation target is irradiated with laser light from each of the R, G, and B laser light sources in a time-division manner and the driving of the imaging element in the camera head 5119 is controlled in synchronization with the irradiation timing to capture R, G, and B images in a time-division manner. According to this method, it is possible to obtain a color image, without providing a color filter in the imaging element.

In addition, the driving of the light source device 5157 may be controlled such that the intensity of light output from the light source device 5157 is changed at a predetermined time interval. The driving of the imaging element in the camera head 5119 is controlled in synchronization with the change timing of the intensity of the light to acquire images in a time-division manner and the images are combined to generate an image without so-called blocked-up shadows and blown-out highlights in a high dynamic range.

Further, the light source device 5157 may be configured such that it can supply light in a predetermined wavelength band corresponding to special light observation. In the special light observation, for example, so-called narrow band imaging is performed which emits light in a narrower band than irradiation light (that is, white light) used in normal observation to capture an image of a predetermined tissue, such as the blood vessel in a superficial portion of the mucous membrane, using the wavelength dependency of light absorption in the body tissue. Alternatively, in the special light observation, fluorescence observation may be performed which obtains an image using fluorescence that is generated by the emission of excitation light. In the fluorescence observation, the following can be performed: the body tissue is irradiated with excitation light and fluorescence is observed from the body tissue (self-fluorescence observation) ; or a reagent, such as indocyanine green (ICG), is locally injected into the body tissue and the body tissue is irradiated with excitation light corresponding to the fluorescent wavelength of the reagent to obtain a fluorescent image, for example. The light source device 5157 may be configured so as to supply narrow-band image and/or excitation light corresponding to the special light observation.

### (Camera Head and CCU)

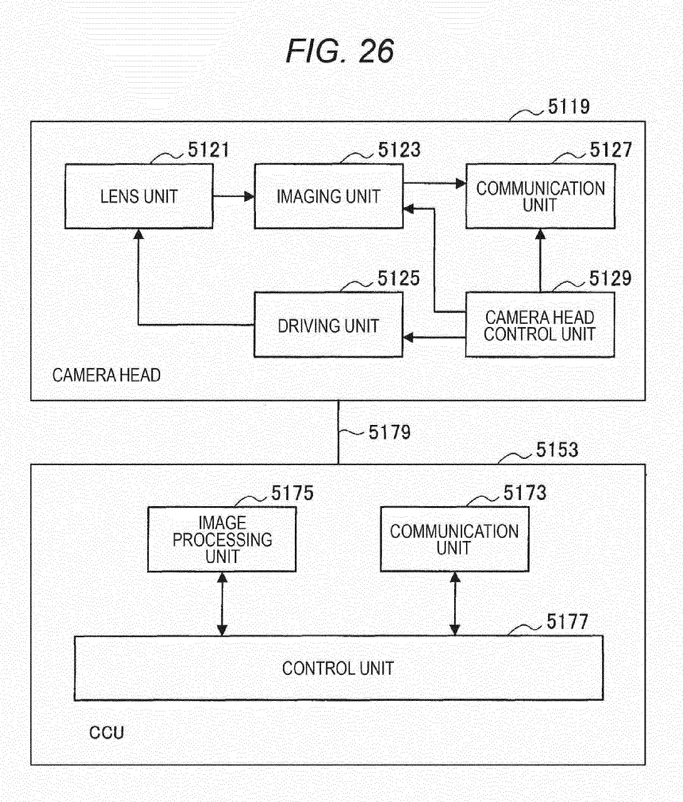

The functions of the CCU 5153 and the camera head 5119 of the endoscope 5115 will be described in more detail with reference to Fig. 26. Fig. 26 is a block diagram illustrating an example of the functional configuration of the camera head 5119 and the CCU 5153 illustrated in Fig. 25.

Referring to Fig. 26, the camera head 5119 includes, as functional units, a lens unit 5121, an imaging unit 5123, a driving unit 5125, a communication unit 5127, and a camera head control unit 5129. In addition, the CCU 5153 includes, as functional units, a communication unit 5173, an image processing unit 5175, and a control unit 5177. The camera head 5119 and the CCU 5153 are connected by a transmission cable 5179 such that they can communicate with each other in both directions.

First, the functional configuration of the camera head 5119 will be described. The lens unit 5121 is an optical system that is provided in a connection portion with the lens barrel 5117. Observation light emitted from the leading end of the lens barrel 5117 is guided to the camera head 5119 and is incident on the lens unit 5121. The lens unit 5121 is configured by a combination of a plurality of lenses including a zoom lens and a focus lens. The optical characteristics of the lens unit 5121 are adjusted such that observation light is focused on a light receiving surface of an imaging element of the imaging unit 5123. In addition, the position of the zoom lens and the focus lens on the optical axis can be moved in order to adjust the magnification and focus of a captured image.

The imaging unit 5123 includes an imaging element and is arranged in a stage behind the lens unit 5121. The observation light transmitted through the lens unit 5121 is focused on the light receiving surface of the imaging element and photoelectric conversion is performed for the observation light to generate an image signal corresponding to an observation image. The image signal generated by the imaging unit 5123 is provided to the communication unit 5127.

For example, a complementary metal oxide semiconductor (CMOS) image sensor which has a Bayer array and can capture a color image is used as the imaging element forming the imaging unit 5123. In addition, for example, the imaging element may be an image element that can respond to the capture of an image with a high resolution of 4K or more. In this case, since an image of an operative site is obtained with high resolution, the operator 5181 can check the aspect of the operative site in more detail and more smoothly progress the operation.

In addition, the imaging element forming the imaging unit 5123 is configured so as to include a pair of imaging elements for acquiring image signals for the right eye and the left eye corresponding to 3D display. The execution of 3D display makes it possible for the operator 5181 to accurately check the depth of the body tissues in the operative site. In addition, in a case where the imaging unit 5123 is a multi-plate type, a plurality of lens units 5121 corresponding to each imaging element are provided.

Further, the imaging unit 5123 may not be necessarily provided in the camera head 5119. For example, the imaging unit 5123 may be provided immediately after the objective lens in the lens barrel 5117.

The driving unit 5125 is an actuator and moves the zoom lens and the focus lens of the lens unit 5121 along the optical axis by a predetermined distance under the control of the camera head control unit 5129. In this way, the magnification and focus of the image captured by the imaging unit 5123 can be appropriately adjusted.

The communication unit 5127 is a communication device for transmitting and receiving various kinds of information to and from the CCU 5153. The communication unit 5127 transmits the image signal obtained from the imaging unit 5123 as RAW data to the CCU 5153 through the transmission cable 5179. At that time, the image signal is preferably transmitted by optical communication in order to display the captured image of the operative site with low latency. At the time of an operation, the operator 5181 performs the operation while observing the state of the affected part using the captured image. Therefore, a moving image of the operative site needs to be displayed in real time as much as possible for a safe and reliable operation. In a case where optical communication is performed, the communication unit 5127 is provided with a photoelectric conversion module that converts an electric signal into an optical signal. The image signal is converted into an optical signal by the photoelectric conversion module and is then transmitted to the CCU 5153 through the transmission cable 5179.

In addition, the communication unit 5127 receives a control signal for controlling the driving of the camera head 5119 from the CCU 5153. The control signal includes information related to imaging conditions, such as information for designating the frame rate of the captured image, information for designating an exposure value during imaging, and/or information for designating the magnification and focus of the captured image. The communication unit 5127 provides the received control signal to the camera head control unit 5129. In addition, a control signal may be transmitted from the CCU 5153 by optical communication. In this case, the communication unit 5127 is provided with a photoelectric conversion module that converts an optical signal into an electric signal. The control signal is converted into an electric signal by the photoelectric conversion module and is then provided to the camera head control unit 5129.

In addition, the control unit 5177 of the CCU 5153 automatically sets the imaging conditions, such as the frame rate, the exposure value, the magnification, and the focus, on the basis of the acquired image signal. That is, the endoscope 5115 has a so-called auto exposure (AE) function, a so-called auto focus (AF) function, and a so-called auto white balance (AWB) function.

The camera head control unit 5129 controls the driving of the camera head 5119 on the basis of the control signal received from the CCU 5153 through the communication unit 5127. For example, the camera head control unit 5129 controls the driving of the imaging element of the imaging unit 5123 on the basis of information for designating the frame rate of the captured image and/or information for designating exposure at the time of imaging. In addition, for example, the camera head control unit 5129 appropriately moves the zoom lens and the focus lens of the lens unit 5121 through the driving unit 5125 on the basis of information for designating the magnification and focus of the captured image. Further, the camera head control unit 5129 may have a function of storing information for identifying the lens barrel 5117 or the camera head 5119.

In addition, the lens unit 5121, the imaging unit 5123, or the like may be configured to be arranged in a closed structure having a high airtight and waterproof performance. In this case, the camera head 5119 can have resistance to an autoclave sterilization process.

Next, the functional configuration of the CCU 5153 will be described. The communication unit 5173 is a communication device for transmitting and receiving various kinds of information to and from the camera head 5119. The communication unit 5173 receives the image signal transmitted from the camera head 5119 through the transmission cable 5179. At that time, as described above, the image signal can be preferably transmitted by optical communication. In this case, the communication unit 5173 is provided with a photoelectric conversion module that converts an optical signal into an electric signal in correspondence with optical communication. The communication unit 5173 provides the image signal converted into an electric signal to the image processing unit 5175.

In addition, the communication unit 5173 transmits a control signal for controlling the driving of the camera head 5119 to the camera head 5119. The control signal may also be transmitted by optical communication.

The image processing unit 5175 performs various kinds of image processing for the image signal which is RAW data transmitted from the camera head 5119. Examples of the image processing include various kinds of known signal processing such as a development process, a process of increasing image quality (a band enhancement process, super resolution processing, a noise reduction (NR) process, and/or a camera shake correction process, for example), and/or an enlargement process (electronic zoom process). In addition, the image processing unit 5175 performs a detection process for the image signal in order to perform AE, AF, and AWB.

The image processing unit 5175 is a processor, such as a CPU or a GPU. The processor operates according to a predetermined program to perform the image processing or the detection process. In addition, in a case where the image processing unit 5175 includes a plurality of GPUs, the image processing unit 5175 appropriately divides information related to the image signal and the plurality of GPUs perform image processing in parallel.

The control unit 5177 performs various types of control related to the capture of the image of the operative site by the endoscope 5115 and the display of the captured image. For example, the control unit 5177 generates a control signal for controlling the driving of the camera head 5119. At that time, in a case where the user inputs imaging conditions, the control unit 5177 generates a control signal on the basis of the imaging conditions input by the user. Alternatively, in a case where the endoscope 5115 has an AE function, an AF function, and an AWB function, the control unit 5177 appropriately calculates an optimum exposure value, an optimum focal length, and an optimum white balance according to the result of the detection process by the image processing unit 5175 and generates a control signal.

In addition, the control unit 5177 displays the image of the operative site on the display device 5155 on the basis of the image signal subjected to the image processing by the image processing unit 5175. At that time, the control unit 5177 recognizes various objects in the image of the operative site using various image recognition techniques. For example, the control unit 5177 can detect the shape, color, and the like of the edge of the object included in the image of the operative site to recognize a surgical tool, such as forceps, a specific living body part, bleeding, mist generated in a case where the energy treatment tool 5135, and the like. In a case where the image of the operative site is displayed on the display device 5155, the control unit 5177 displays various kinds of operation support information so as to be superimposed on the image of the operative site on the basis of the recognition result. Since the operation support information is displayed so as to be superimposed on the image and is presented to the operator 5181, it is possible to stably and reliably progress the operation.

The transmission cable 5179 for connecting the camera head 5119 and the CCU 5153 is an electric signal cable corresponding to the communication of electric signals, an optical fiber corresponding to optical communication, or a composite cable thereof.

Here, in the example illustrated in Fig. 26, wired communication using the transmission cable 5179 is performed. However, wireless communication may be performed between the camera head 5119 and the CCU 5153. In a case where wireless communication is performed between the camera head 5119 and the CCU 5153, it is not necessary to install the transmission cable 5179 in the operating room. Therefore, the situation that the movement of the medical staff in the operating room is disturbed by the transmission cable 5179 can be prevented.

An example of the operating room system 5100 to which the technique according to the present disclosure can be applied has been described above. In addition, here, for example, a case where the medical system to which the operating room system 5100 is applied is the endoscopic surgery system 5113 has been described. However, the configuration of the operating room system 5100 is not limited to the above-mentioned example. For example, the operating room system 5100 may be applied to a flexible endoscope system for examination or a microscopic surgery system, instead of the endoscopic surgery system 5113.

For example, the technique according to the present disclosure can be suitably applied to various operations using the operation screen of the centralized operation panel 5111 illustrated in Fig. 24 among the above-mentioned configurations. Specifically, the doctor or the medical staff can perform a necessary operation in a non-contact manner using the center-regression-type menu or the pie-type menu.

In particular, it is clear that, since various operations are performed in a non-contact manner in the operating room, the above-mentioned configuration is preferable in terms of sanitation. In addition, the use of the center-regression-type menu or the pie-type menu makes it possible to provide a simple and efficient operation environment for various operations required during the operation.

Hereinafter, a specific example of the operation screen will be described. Here, an example in which the center-regression-type menu and the pie-type menu are complexly used is given.

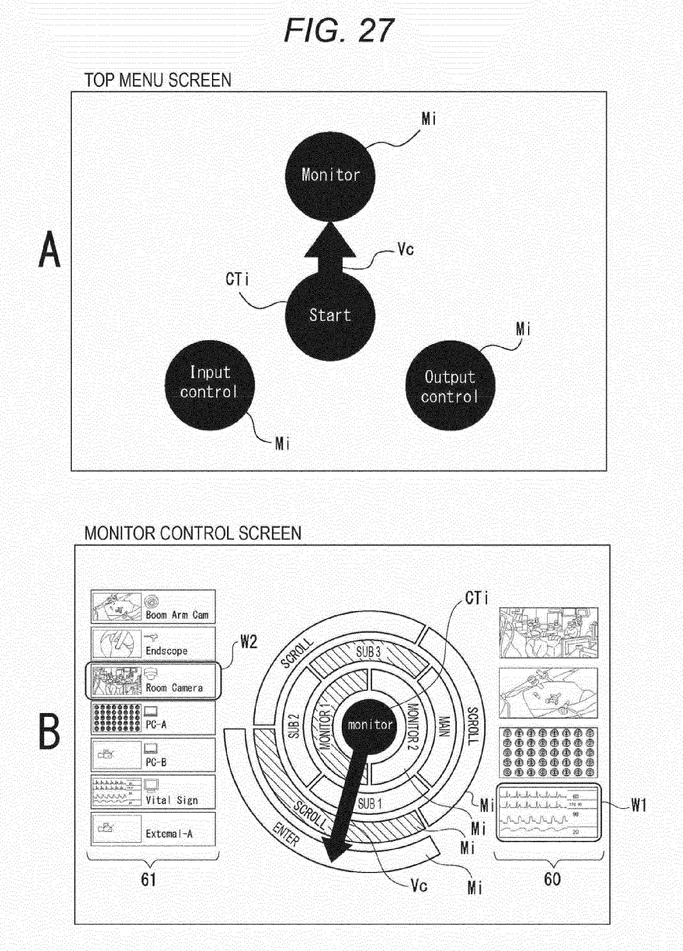

Fig. 27A illustrates a state in which a top menu is displayed by the center-regression-type menu.

A "Monitor" item, an "Input control" item, and an "Output control" item are illustrated as the items Mi that can be selected by the operation vector Vc from the origin item CTi.

The "Monitor" item is an item Mi for selecting an operation (an operation in the transmission source selection region 5195) of selecting an image transmission source displayed on the screen in the preview region 5197 illustrated in Fig. 24.

The "Input control" item is an item Mi for selecting an operation (each of the pan, tilt, and focus operations) in the transmission source operation region 5203 of the control region 5201 illustrated in Fig. 24.

The "Output control" item is an item Mi for selecting an operation (each of the swap, flip, contrast, color, 2D/3D mode operations) in the output destination operation region 5205 of the control region 5201.

It is assumed that the "Monitor" item is selected in the center-regression-type menu illustrated in Fig. 27. In this case, a low-layer item of the center-regression-type menu is not present in the "Monitor" item and the selection of the "Monitor" item is recognized as the entering of an operation for selecting the preview region 5197.

Therefore, as illustrated in Fig. 27B, a monitor control screen including the pie-type menu for an operation of selecting a transmission source in the monitor screen is displayed.

In the pie-type menu, items Mi for selecting two display devices (Monitor1 and Monitor2) which are the output destination devices illustrated in the preview region 5197 of Fig. 24 are arranged in the first layer.

In addition, items Mi for selecting a main screen and three sub-screens of the display device are arranged in the second layer. Fig. 27B illustrates an aspect in which three sub-screens of "Monitor1" have been already selected.

A monitor screen list 60 is displayed on the right side of the pie-type menu and the current images of the main screen and three sub-screens are displayed. In response to the selection of the third sub-screen, a cursor frame W1 is displayed on the image of the third sub-screen (sub3).

In the example, "SCROLL" items are arranged in the third layer. An "enter" item is also arranged on the outer circumferential side according to the "SCROLL" item.

The third layer enables a scroll operation for selecting the source of the third sub-screen of "Monitor1". Specifically, the scroll operation is an operation of selecting each transmission source in the transmission source selection region 5195 illustrated in Fig. 24. A transmission source list 61 corresponding to the transmission source selection region 5195 is displayed on the right side of the pie-type menu illustrated in Fig. 27B. A cursor frame W2 of the transmission source list 61 is moved forward/backward (in this case, up/down) by the scroll operation.

In a case where the user performs an operation of selecting the "enter" item, the transmission source indicated by the cursor frame W2 is selected and entered at that time. The entered transmission source is set to the transmission source of the third sub-screen.

Next, an example of a case where the "Input control" item is selected in the center-regression-type menu illustrated in Fig. 27A will be described.

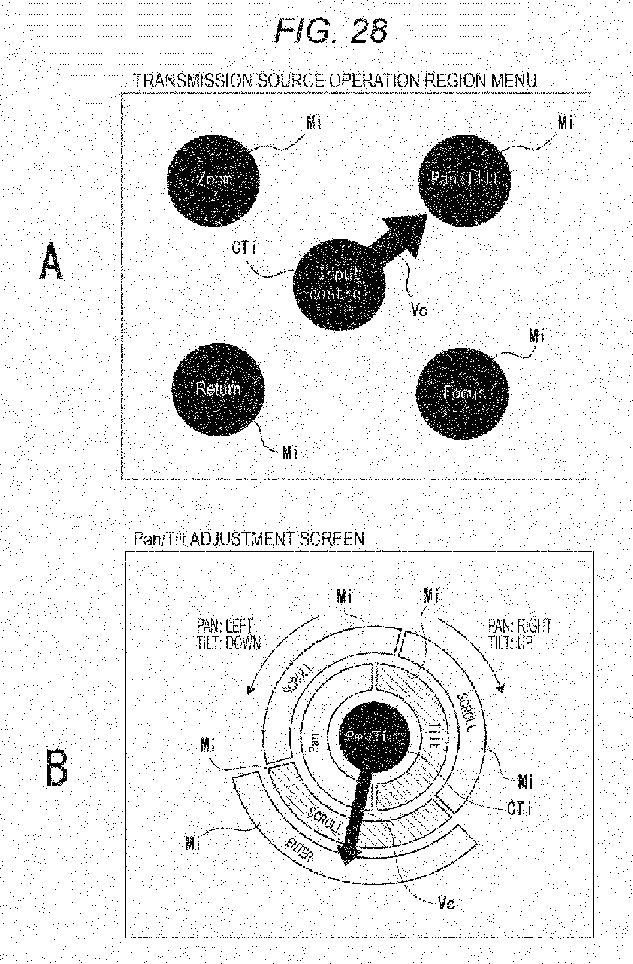

Fig. 28A illustrates a state in which the "Input control" item is selected and then becomes the origin item CTi and four items Mi, that is, a "Pan/Tilt" item, a "Zoom" item, a "Focus" item, and a "return" item are arranged as choices in the lower layer around the "Input control" item.

The "return" item appears by the movement of the "start" item to the position where the "Input control" item has been originally arranged.

Here, assuming that the "Pan/Tilt" item is selected, a pie-type menu for performing a pan or tilt operation using scrolling is displayed as illustrated in Fig. 28B.

In the pie-type menu, items Mi for selecting the pan operation and the tilt operation are arranged in the first layer. Here, Fig. 28B illustrates a state in which the tilt operation has been selected.

In this example, "SCROLL" items are arranged in the second layer. An "enter" item is also arranged on the outer circumferential side according to the "SCROLL" item.

The second layer enables the adjustment of the tilt operation, that is, a tilt angle in the up-down direction by the scroll operation.

In a case where the pan operation is selected, the "SCROLL" items are arranged in the second layer and the pan operation, that is, adjustment in the left-right direction can be performed by the scroll operation.

In a case where the user performs an operation of selecting the "enter" item, adjustment ends at a tilt position (or a pan position) at that time.

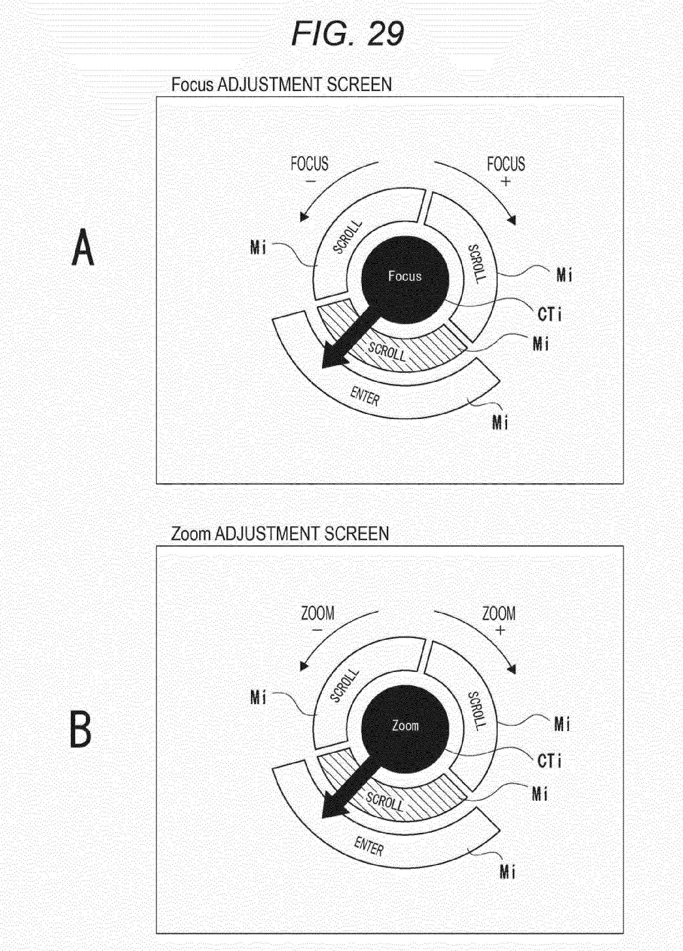

Fig. 29A illustrates a case where the "Focus" item is selected in the center-regression-type menu illustrated in Fig. 28A. In this case, the "SCROLL" items and the corresponding "enter" item are arranged only in the first layer.

The "SCROLL" items are sequentially selected to adjust the focus state in a positive (+) direction or a negative (-) direction.

In a case where the user performs an operation of selecting the "enter" item, adjustment ends in the focus state at that time.

Fig. 29B illustrates a case where the "Zoom" item is selected in the center-regression-type menu illustrated in Fig. 28A. In this case, the "SCROLL" items and the corresponding "enter" item are arranged only in the first layer.

The "SCROLL" items are sequentially selected to adjust the zoom state in the positive direction or the negative direction.

In a case where the user performs an operation of selecting the "enter" item, adjustment ends in the zoom state at that time.

Next, a case where the "Output control" item in the center-regression-type menu illustrated in Fig. 27A, that is, an operation in the output destination operation region 5205 is selected will be described.

Fig. 30A illustrates a state in which the "Output control" item is selected and then becomes the origin item CTi and six items Mi, that is, a "3D/2D" item, a "Swap" item, a "Color" item, a "Contrast" item, a "Flip" item, and a "return" item are arranged as choices in the lower layer around the "Output control" item.

The "return" item appears by the movement of the "start" item to the position where the "Output control" item has been originally arranged.

Here, assuming that the "3D/2D" item is selected, the pie-type menu in which items Mi for selecting a 2D mode or a 3D mode are arranged is displayed as illustrated in Fig. 30B.

In the pie-type menu, only the first layer is given as an example. For example, in a case where the item Mi indicating the 3D mode is selected, the "enter" item is arranged on the outer circumferential side of the selected item Mi. As illustrated in Fig. 30B, the "enter" item is selected to enter the 3D mode.

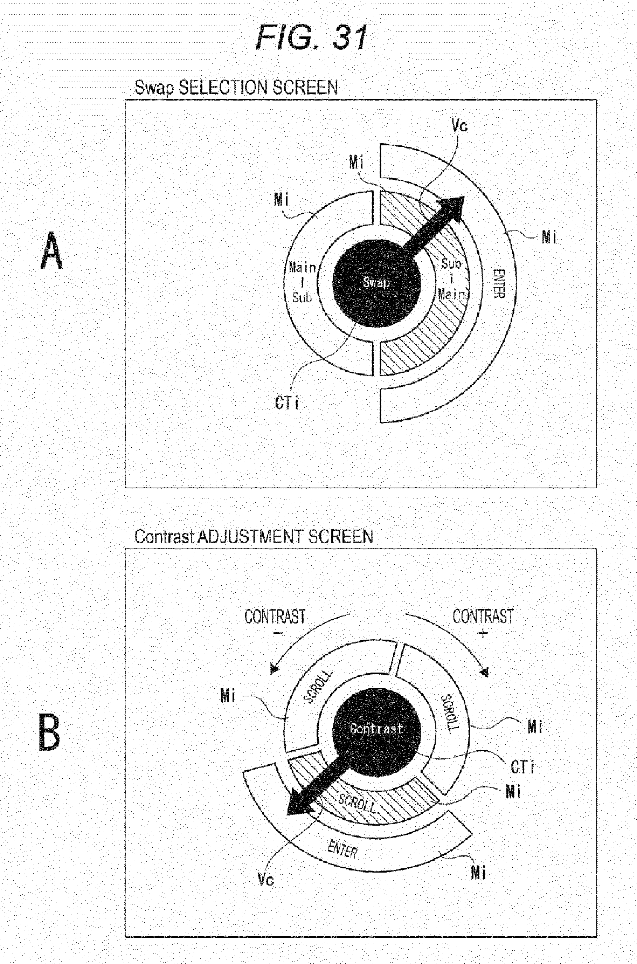

Fig. 31A illustrates a case where the "Swap" item is selected in the center-regression-type menu illustrated in Fig. 30A. In this case, items Mi for a main-sub swap operation and a sub-main swap operation are arranged as selection items only in the first layer.

For example, in a case where the item Mi for the sub-main swap operation is selected, the "enter" item is arranged on the outer circumferential side of the selected item Mi. As illustrated in Fig. 31A, the "enter" item is selected to enter the sub-main swap operation.

Fig. 31B illustrates a case where the "Contrast" item is selected in the center-regression-type menu illustrated in Fig. 30A. This is an example in which the "SCROLL" items only in the first layer and the corresponding "enter" item are arranged.

The "SCROLL" items are sequentially selected to adjust a contrast state in the positive direction or the negative direction.

In a case where the user performs an operation of selecting the "enter" item, adjustment ends in the contrast state at that time.

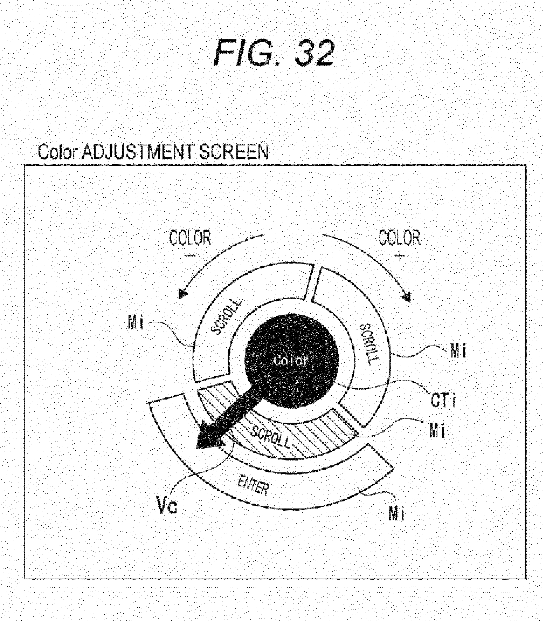

Fig. 32 illustrates a case where the "Color" item is selected in the center-regression-type menu illustrated in Fig. 30A. This is an example in which the "SCROLL" items only in the first layer and the corresponding "enter" item are arranged.

The "SCROLL" items are sequentially selected to adjust a color state in the positive direction or the negative direction.

In a case where the user performs an operation of selecting the "enter" item, adjustment ends in the color state at that time.

An example of the user interface using the center-regression-type menu and the pie-type menu according to the present disclosure to the operating room system has been described above. An information processing device having the functional configuration illustrated in Fig. 1 can combine the processes illustrated in Fig. 11, Fig. 16, Fig. 18, and Fig. 20 to implement the user interface described herein.

### <8. Summary and Modification Examples>

The information processing device 1 according to the above-described embodiment includes the operation detection unit 2 that calculates an operation direction and an operation distance from the acquired non-contact operation information. In addition, the information processing device 1 includes the control unit 3 that performs a process of determining that an item Mi has been selected in a case where a positional relationship between the region of the item Mi to be selected and an operation position which is specified by the operation direction and the operation distance calculated by the operation detection unit 2 using an origin position (x0, y0) as a starting point is changed to a specific state in a state in which the regions of the items Mi to be selected are arranged around the origin position. Specifically, the control unit 3 performs a process that sets an operation plane in which a plurality of items Mi to be selected are arranged around the origin positions (x0, y0) and recognizes that, in a case where the operation position specified by the operation direction and the operation distance from the origin position as the starting point on the operation plane indicates a certain item Mi, recognizes that the item Mi has been selected. In addition, the information processing device 1 includes the display data generation unit 4 that generates display data corresponding to the determination result of the control unit 3. Specifically, the display data generation unit 4 generates display data for displaying the operation plane set by the control unit 3.

That is, the operation direction and the operation distance for a non-contact operation of the user is reflected as a direction and a distance from the origin as a starting point in a virtual operation plane. This means that the direction and the distance indicated by the non-contact operation are recognized as a direction and a distance from the origin position on the operation plane.

The items Mi to be selected are arranged around the origin position in the set operation plane. Then, in a case where the operation position specified by the operation direction and the operation distance from the origin position indicates a certain item Mi (for example, the operation position reaches the item or passes through the item), it is recognized that the item Mi has been selected.

In the center-regression-type menu or the pie-type menu, the items Mi to be selected are arranged around the origin item CTi as the center located at the origin (x0, y0) in a ring shape and the starting point of an operation is the origin (x0, y0). Therefore, for example, in a state in which the position extending from the origin which is specified by the direction and distance of an operation reaches or passes through a certain item Mi, it may be recognized that the item Mi has been selected.

In this configuration, in a case where the operation vector Vc extends, each item Mi (all items Mi in the case of the center regression type and the items Mi in the first round in the case of the pie type) is arranged so as not to be disturbed by other items.

Therefore, for example, in a case where the operation vector Vc reaches an item Mi, the item Mi can be selected. As a result, the operation is very easy.

In addition, with this configuration, other special operations for selecting an item Mi, for example, an operation for expressing a click or button pressing, such as an operation in the depth direction, an operation for maintaining an arrival state for a certain amount of time, and the like are not required.

Further, since other operations corresponding to, for example, a click are not required, an operation error due to other operations does not occur. In particular, in the case of a non-contact operation, the behavior of the user's operation is generally unstable. As the number of necessary extra operations increases, the number of operation errors caused by touch with selection items other than the target (the operation position reaches the selection items other than the target) increases, for example. For example, an operation in the depth direction or an operation of selecting a specific gesture is considered. However, the position on the operation plane is often shifted by the operation in the depth direction and the gesture may not be recognized. In addition, for example, a method may be considered in which the touch of the operation vector Vc with an item Mi is maintained for 3 seconds . However, in this case, a quick operation is hindered.

In contrast, in this embodiment, for example, immediately after the operation vector Vc reaches an item Mi, the item Mi is selected. Therefore, the deviation of the operation position (the position indicated by the operation vector Vc) does not occur. In addition, it does not take time to change to a selected state. As a result, it is easy for the user to perform a stable operation.

Therefore, the user can very easily and stably perform a non-contact operation.

In addition, since this configuration does not require a gesture or an operation in the depth direction, a menu operation can be quickly performed with the minimum operation.

In the embodiment, the origin position is set substantially at the center of the operation region AR in which non-contact operation information is effective as the range of the operation plane.

Since the origin position is set substantially at the center of the operation region AR, the items Mi to be selected are arranged around the center of the operation region AR in a circular ring shape.

Therefore, the items Mi are not arranged extremely close to the edge of the operation region AR. The operation region AR corresponds to the recognition range of the non-contact operation of the user and the center recognizing the non-contact operation corresponds to the center of the operation region AR. Therefore, the configuration in which the items Mi are not arranged extremely close to the edge (a portion in which recognition sensitivity is reduced) is advantageous in recognizing an operation.

In addition, the origin position is not necessarily set at the center position of the operation region AR. For example, the origin position may deviate from an exact center position or may be set at the center of gravity of the operation region AR or the like depending on the shape of the operation region AR. The origin position may not be necessarily the exact center position and may be appropriately set according to, for example, the content of an operation, the aspect of the operation body 50, and the arrangement of items.

In the embodiment, the display data generation unit 4 generates display data for displaying the operation direction and the operation distance calculated by the operation detection unit 2 on the operation plane.

That is, not only the origin region and the items Mi to be selected but also the current operation position specified by the operation direction and the operation distance is displayed on the operation plane displayed on the display screen. For example, the operation position is represented by an image or the like of an arrow or the like extending from the origin.

Specifically, the items Mi and the origin item CTi are displayed as the menu on the screen and the operation vector Vc indicating an operation direction and an operation distance are displayed in response to an operation. The user can receive feedback on the image of a non-contact operation performed in a space and can perform an accurate operation for selecting a desired item Mi.

That is, the operation vector Vc and the extension state of the operation vector Vc are guided, which makes it easy for the user to understand to which item Mi the operation vector Vc extends currently, in which direction a non-contact operation needs to be performed, and how much the non-contact operation needs to be performed.

Note that the current operation position (a position indicated by the operation vector Vc) on the operation plane is represented by an arrow segment such as the operation vector Vc. However, the current operation position may be represented by, for example, a cursor indicating only the current operation position or a pointer display. In addition, the trajectory of the operation may be displayed so as to remain to some extent.

In the embodiment, the control unit 3 sets the operation plane in which a plurality of items Mi to be selected are arranged around the origin position so as not to overlap each other as viewed from the origin position.

For example, the operation plane is a center-regression-type operation plane.

In the case of the center-regression-type menu, all of the plurality of items Mi are arranged so as not to overlap each other as viewed from the origin position. Therefore, the operation vector Vc extending from the origin position as a starting point can reach all of the arranged items Mi to select the items Mi. As a result, the easiness of an operation is improved.

In addition, a case where in the pie-type operation plane, the items Mi exist only on the first virtual ring, no lower layers are present, and the item Mi is entered only by the reaching of the operation vector to the item Mi corresponds to the above-mentioned configuration.

Further, as the processing of the center-regression-type menu, the control unit 3 recognizes that a certain item Mi has been selected by an operation position on the operation plane. In a case where the selected item Mi does not have a lower-layer item Mi, the control unit 3 arranges the selected item Mi at the origin position and resets the operation plane in which the lower-layer items Mi are arranged around the origin position so as not to overlap each other as viewed from the origin position. Then, in a case where an operation position on the reset operation plane indicates a certain lower-layer item Mi, the control unit 3 performs a process of recognizing that the lower-layer item Mi has been selected.

That is, the operation plane setting process and the operation detection process described in Figs. 8 to 12 are performed.

In the case of the center-regression-type menu, a plurality of items Mi are arranged so as not to overlap each other as viewed from the origin position. Therefore, all of the arranged items Mi can be selected by the extension of the operation vector Vc (reaching to the operation position) from the origin position as a starting point. In this case, if lower-layer items Mi are present in the selected item Mi, the operation plane is switched to an operation plane in which the selected item Mi is arranged at (moved to) the origin position and the lower-layer items Mi are arranged around the selected item Mi.

With this configuration, even if there are many lower layers under a certain item, a selection operation for all layers can correspond to an operation of pointing to an item Mi from the origin item CTi (the item Mi which is selected and becomes the origin item) at the origin position as a starting point.

Therefore, a method in which the operation vector reaches or passes through an item Mi to select the item Mi can be applied to a menu operation for a plurality of layers.

In particular, since an operation always starts from the center in each layer, an erroneous operation caused by screen transition is prevented.

In addition, in a case where the layer advances, since the items Mi as the center-regression-type are rearranged, the operation region AR is not expanded by the layer.

For example, in a case where a certain item Mi is selected and lower-layer items Mi are arranged around the item Mi without regressing to the center, the lower-layer items Mi are arranged away from the origin position as the layer advances. As a result, a wide operation region is required. In this case, the region required for an operation is also unnecessarily expanded on the display, which is likely to hinder the display of other items. In addition, in a case where the operation space of the user is excessively widened, it is difficult to recognize an operation.

In the case of the center-regression-type menu according to this embodiment, the selected itemMi regresses to the origin position at the center of the operation region AR and the lower-layer items Mi are arranged around the selected item Mi. Therefore, it is possible to perform an operation for the lower layer in a similar operation region to that in the upper layer.

With this configuration, it is possible to set the operation region AR to a relatively small region and the percentage of a display range on the display is not too high.

In addition, the sensing range of the input device (tracking device 10) may be a relatively narrow range and this configuration can respond to a menu structure having any number of layers.

Further, in many cases, sensitivity is high in the vicinity of the center of the sensing range of the tracking device 10. In any layer, an operation is performed using the center as a starting point, which means that the operation is detected in the vicinity of the center of the sensing range. That is, a region having high operation sensitivity is used as much as possible, which is preferable. As a result, the operation of the user is smoothly performed.

For the center-regression-type menu according to the embodiment, in a case where the operation plane is set or reset, the control unit 3 arranges a plurality of items Mi around the origin position along the virtual ring (virtual ring CR) of a true circle having the origin position as the center.

With this configuration, it is easy for each item Mi to be equally visibly recognized as a selection target and the distances from the origin position to all of the items Mi are equal to each other. There is no difference in the easiness of a selection. Therefore, it is possible to create an operation plane suitable for any selection operation of the user.

For the center-regression-type menu according to the embodiment, in a case where the control unit 3 resets the operation plane for center regression, the control unit 3 controls the display data generation unit 4 such that display for presenting the movement of the selected item Mi to the origin position is performed (see Step S115 in Fig. 11 and Figs. 12B and 12D).

That is, an aspect in which the selected itemMi regresses to the origin position is presented to the user on the display.

Therefore, it is easy for the user to recognize a state in which the selected item Mi is moved to the center and the lower-layer items Mi are arranged around the selected item Mi.

Thereafter, the selected item Mi becomes the origin item CTi and an operation is performed again using the center as a starting point. The user is prevented from being confused by display clearly showing the aspect of center regression.

For the center-regression-type menu according to the embodiment, in a case where the operation plane is reset, the control unit 3 arranges the lower-layer items Mi and the "return" item for returning the layer around the origin position so as not to overlap each other as viewed from the origin position.

That is, since the lower-layer items and the layer-return item are arranged around the selected item, it is possible to select an operation of going back to the upper layer.

Therefore, it is possible to easily select the "return" operation as in the selection of the lower-layer item Mi.

In particular, as described in Fig. 12A→Fig. 12B→Fig. 12C, the "return" item is arranged at the position of the selected item Mi. In this case, the user can see the aspect in which the selected item Mi and the "return" item (previous origin item CTi) are interchanged with each other and can easily recognize the advance of the layer.

In addition, the user can easily check a position for the return operation.

However, the "return" item may not be necessarily arranged at the position of the previously selected item Mi and may be matched with the number of choices in the layer that advances.

Furthermore, the "return" item may be arranged at a specific coordinate position regardless of how the layer advances. For example, the "return" item may be arranged immediately below the origin item CTi. In a case where the "return" item is arranged at a specific coordinate position, the user can always recognize the "return" operation as an operation in the direction and it is easy to perform an operation.

Further, the item Mi for the return operation is the item Mi represented by "return". However, the item Mi for the return operation may be represented by the word in the previous layer. For example, the item Mi for the return operation may be represented by "start" in Fig. 9A and may be represented by "K2" in Fig. 10.

In addition, the item Mi for the return operation may not be provided and the return operation may be performed by other methods.

For the center-regression-type menu according to the embodiment, in a case where the "return" item for returning the layer is selected, the control unit 3 controls the display data generation unit such that display for presenting the movement of the "return" item to the origin position is performed (see Step S113→Step S115 in Fig. 11 and Fig. 12D) . Then, the control unit 3 resets the operation plane such that the upper-layer items Mi are arranged around the origin position so as not to overlap each other as viewed from the origin position.

That is, in a case where the "return" item is selected, the control unit 3 represents the aspect in which the "return" item regresses to the origin position to the user on the display, arranges the items Mi in the upper layer, and displays the items Mi.

This configuration makes it easy for the user to recognize the changed state in which the "return" item is moved to the center and the items Mi in the immediately upper layer are arranged around the "return" item. Then, the menu returns to the immediately upper layer and an operation having the center as a starting point is performed. Therefore, the user recognizes the return of the layer. As a result, it is easy for the user to correctly recognize the operation situation without confusing the operation.

In the embodiment, the example in which the control unit 3 sets an operation plane of the pie-type menu as the operation plane for performing an operation involving a plurality of stages for selecting or designating an itemhas been described.

In this case, the control unit 3 sets an operation plane in which a plurality of items Mi to be selected in an X-th stage (X is a layer value) are arranged on an X-th virtual ring-shaped line (an X-th virtual ring CR(X)) from the origin position so as not to overlap each other as viewed from the origin position.

In addition, in a case where the control unit 3 recognizes that a certain item Mi among the plurality of items Mi in the X-th stage has been selected by an operation position, the control unit 3 sets an operation plane in which one or a plurality of items Mi to be designated or selected according to the item selected in the X-th stage are arranged on an (X+1)-th virtual ring-shaped line from the origin position.

That is, a pie-type operation plane is formed and a selection/designation operation in a plurality of stages is recognized.

In the case of the pie-type menu, in response to the selection of one of the items Mi arranged in a ring shape, for example, a plurality of items Mi in the lower layer and an item Mi for an enter operation are arranged on the outer circumferential side of the selected item Mi. Therefore, an operation can be performed in a plurality of stages by the extension of the operation vector Vc. Therefore, it is possible to very quickly perform, for example, an operation for a layered menu or an operation of continuously designating a plurality of items.

For the pie-type menu according to the embodiment, in a case where the control unit 3 recognizes that a certain item Mi among the plurality of items Mi in the X-th stage has been selected by an operation position, the control unit 3 sets an operation plane in which a plurality of lower-layer items Mi to be selected in the lower layer of the item selected in the X-th stage are arranged on the (X+1) -th virtual ring-shaped line from the origin position (see Fig. 14B and Step S211 in Fig. 16) . Then, in a case where the operation position indicates a certain lower-layer item Mi, the control unit 3 performs a process of recognizing that the lower-layer item Mi has been selected (Fig. 15A, Fig. 15B, and Steps S204 and S205 in Fig. 16).

That is, the operation plane is formed in which the items Mi in the lower layer are presented on the outer circumferential side, considering, for example, a plurality of virtual ring-shaped lines (CR1, CR2, ···) having the origin position as the center. Therefore, a lower-layer item Mi can be selected following the selection of an upper-layer item Mi by the extension of an operation position determined by an operation direction and an operation distance.

In this method, a pie-type menu having a layer structure can be used.

The user can perform an operation of extending the operation vector Vc in any direction to quickly and easily perform an operation of sequentially selecting the items Mi from the upper layer to the lower layer.

For the pie-type menu according to the embodiment, in a case where the control unit 3 recognizes that a certain item Mi among a plurality of items Mi in the X-th stage has been selected by an operation position, the control unit 3 sets an operation plane in which an "enter" item for the item selected in the X-th stage is arranged on the (X+1)-th virtual ring-shaped line from the origin position (see Fig. 13B and Step S211 in Fig. 16). Then, in a case where the operation position designates the "enter" item, the control unit 3 performs a process (S230, S231) of recognizing that the enter operation for the item selected in the X-th stage has been performed.

That is, in a case where the operations are performed in stages, that is, the enter operation is performed after the selection operation, an item Mi for the enter operation is arranged on the outer circumferential side of a plurality of items Mi arranged on the virtual ring CR. Therefore, the enter operation can be performed following the selection of an item Mi by the extension of an operation position determined by an operation direction and an operation distance.

In this method, the selection and entering of an item in the pie-type menu can be performed by a series of operations . That is, the user can perform an operation of extending the operation vector Vc in any direction to quickly and easily perform an operation of selecting and entering a certain item Mi.

For the pie-type menu according to the embodiment, the control unit 3 arranges the "enter" item at a position that is on the (X+1)-th virtual ring-shaped line and is adjacent to the item selected in the X-th stage as illustrated in Figs. 13B, 15A, and 15B.

With this configuration, in a case where the operation distance extends in the operation direction immediately after the selection of an item Mi, the operation vector reaches the "enter" item. Therefore, the enter operation is very easily and quickly performed.

In addition, the "enter" item is arranged so as to be adjacent to the selected item Mi in the same angular range as the selected item Mi. Therefore, the user intuitively understands the enter operation.

Further, the "enter" item may not be necessarily arranged in the same angular range as the selected item Mi. The angular ranges may partially overlap each other or the angular range of the "enter" item may be wider than that of the selected item. Furthermore, it is considered that the "enter" item is set on the entire circumference of the (X+1)-th virtual ring CR in a ring shape.

In addition, in a case where an item Mi without a lower layer is selected as described in Fig. 14A, a configuration may be used in which the "enter" item is not arranged and the user immediately recognizes that an enter operation has been performed. In this case, it is possible to improve the efficiency of an operation.

For the pie-type menu according to the embodiment, the control unit 3 sets a plurality of items Mi to be selected in the X-th stage as items for a scroll operation and performs a process of recognizing the scroll operation in a case where the item for the scroll operation is selected (see Figs. 18, 19, 20, and Fig. 21).

That is, the scroll operation can be performed by the "SCROLL" items arranged in a ring shape.

Since the scroll operation using the pie-type menu can be performed, it is possible to effectively perform, for example, a menu operation in a case where there are many choices, an operation of moving a display range, and various other operations using the pie-type menu.

In addition, the item for the scroll operation is not limited to the "SCROLL" item. For example, the following operation method is considered: for the "advance" and "return" items, while the operation vector Vc is being positioned at the items, the "advance" operation continues or the "return" operation continues.

For the pie-type menu according to the embodiment, the control unit 3 performs a process of recognizing the direction of the scroll operation according to the order in which a plurality of "SCROLL" items for the scroll operation are selected (see Fig. 18, Fig. 20, and Step S257 in Fig. 22).

That is, an operation of sequentially selecting a plurality of items arranged in a ring shape indicates the direction of the scroll operation.

A scroll operation that is easy to perform and understand can be performed by this method. In a case where the user sequentially selects a plurality of items Mi arranged in a ring shape clockwise or counterclockwise, this becomes the scroll operation. For example, the user may perform a clockwise or counterclockwise circular operation as the non-contact operation. Therefore, the operation is intuitively easy to understand.

In addition, the example in which three "SCROLL" items are arranged in a circular ring shape has been described. However, two or more "SCROLL" items may be arranged. In a case where two "SCROLL" items are arranged, it is difficult to distinguish the operation directions. However, in a case where the scroll operation is performed in one direction, two "SCROLL" items are enough. In a case where the scroll operation is performed in two operation directions, three or more "SCROLL" items are required.

Further, as a larger number of "SCROLL" items, for example, four or five "SCROLL" items are arranged in a circumferential direction, the scroll operation can be performed at a higher speed. The reason is that, as the number of "SCROLL" items becomes larger, the interval until the operating position reaches the next "SCROLL" item in response to the user's operation becomes shorter.

For the pie-type menu according to the embodiment, the control unit 3 arranges an item for an enter operation after scrolling on the (X+1)-th virtual ring-shaped line (CR) in the same angular range as the item that is being selected among the items for the scroll operation (see the "enter" item in Fig. 19 and Fig. 21 and Step S256 in Fig. 18, Fig. 20, and Fig. 22).

With this configuration, the "enter" item is arranged on the outer circumferential side of the "SCROLL" item that is being selected and the position of the "enter" item on the outer circumferential side is also changed with the progress of the scroll operation. Therefore, in a case where the scroll operation is performed, the aspect in which the "enter" item on the outer circumferential side is moved clockwise or counterclockwise is clearly displayed. As a result, the user performs an operation while easily recognizing the situation of the scroll operation.

Further, in a case where the scroll operation is stopped, since the "enter" item is located on the immediately outer circumferential side (same angle range) of the "SCROLL" item that is being selected at that time, the enter operation can be performed only by moving the operation position to the outer circumferential side. Therefore, it is possible to perform an operation with very high efficiency.

For the pie-type menu according to the embodiment, in a case where the control unit 3 recognizes that a certain item Mi among a plurality of items Mi in the X-th stage has been selected by an operation position, the control unit 3 instructs the display data generation unit 4 such that the display aspect of items Mi which have not been selected among the plurality of items Mi in the X-th stage is changed (see Fig. 14B and Steps S211 and S232 in Fig. 16).

That is, in a case where one of the items Mi in the X-th stage is selected, the display aspect of the items Mi which have not been selected on the display is changed. For example, the items are displayed in an inactive state or are not displayed.

In the pie-type menu, in a case where one of the items Mi in the X-th stage is selected, items Mi in the (X+1)-th stage are displayed on the outer circumferential side of the selected item. In a case where the non-selected items Mi in the X-th stage are displayed on the inner circumferential side of the items Mi in the (X+1)-th stage so as to be operable, it may be difficult to perform an operation. For this reason, the non-selected items Mi are not displayed or are displayed in an inactive state to ensure the easiness of an operation.

For the pie-type menu according to the embodiment, the control unit 3 sets an operation plane in which one or a plurality of items Mi to be designated or selected in the (X+1)-th stage according to the item Mi selected in the X-th stage are arranged. Then, in a case where the selection of the selected item Mi is canceled, the control unit 3 sets the operation plane such that the items Mi in the (X+1)-th stage are canceled (see Steps S221 and S222 in Fig. 16).

That is, in a case where the selected state of the item Mi in the X-th stage is canceled, the menu returns from the state in which the selection of the item in the (X+1)-th stage is detected to the state in which the control unit waits for the selection of a plurality of items Mi to be selected in the X-th stage (the menu returns to the upper layer) . Therefore, the setting of the items in the (X+1)-th stage on the operation plane is canceled and the items are not displayed.

This configuration enables the user to clearly recognize the return of the operation layer to the upper layer.

Note that the example in which the center-regression-type menu or the pie-type menu according to the embodiment is displayed on the display device 11 has been described. The display device 11 may be different from a display device that displays, for example, a content image or an image of a control panel to be operated.

In addition, the display data generation unit 4 may supply display data of the center-regression-type menu or the pie-type menu to an image projection device such that the user visibly recognizes the menu image from the projection display.

Further, in the above-described embodiment, a non-contact operation is assumed. The technique according to the present disclosure can be widely applied to contact operations, such as an operation of touching a screen with fingers or the like and an operation using a device such as a mouse, other than the non-contact operation, for example. For example, in the contact operation, a situation is considered in which it is difficult to stably perform an operation depending on a contact portion or an operator. In this case, the technique according to the present disclosure is preferable as a method for stabilizing an operation.

Further, the technique according to the present disclosure can be applied to a case where a three-dimensional operation space is assumed and items are arranged around the origin position in a three-dimensional direction, in addition to the example in which a two-dimensional operation plane is assumed and items are arranged around the origin position. In this case, it is suitable to display a three-dimensional operation space as the user interface.

In addition, the arrangement of the items around the origin position has been described using the virtual ring CR which is a virtual ring-shaped line, but of course, the virtual ring CR is not a true circle and an elliptical ring, a square ring, a rectangular ring, an irregular ring, or the like may be assumed. That is, it is assumed that a plurality of items Mi are arranged around the origin position along an elliptical or rectangular orbit.

In the above-described embodiment, in a case where the positional relationship between the region of an item Mi and the operation position specified by the operation direction and the operation distance recognized by the operation detection unit using the origin position as a starting point changes to a specific state, the control unit 3 of the information processing device 1 determines that the item has been selected. As an example of the configuration, in a case where the leading end (operation position) of the operation vector Vc reaches the region of an item Mi, the control unit 3 determines that the item has been selected.

However, the present disclosure is not limited thereto. For example, in a case where the operation position passes through the region of an item Mi, the information processing device 1 may recognize that the item Mi has been selected.

In addition, in a case where the operation position approaches an item Mi (the edge of the region of the item Mi) until the distance of the operation position from the item Mi is less than a predetermined value, the information processing device 1 may recognize that the item Mi has been selected.

A program according to the embodiment of the invention causes the information processing device to perform an operation detection step, a determination step, and a display data generation step.

In the operation detection step, the program causes the information processing device 1 to perform a process of calculating an operation direction and an operation distance from operation information acquired by the information processing device (for example, S107 and S204).

In the determination step, the program causes the information processing device 1 to perform a process that, in a case where the positional relationship between the region of an item Mi to be selected and the operation position specified by the operation direction and the operation distance calculated in the operation detection step using the origin position as a starting point is changed to a specific state in a state in which the regions of the items Mi to be selected are arranged around the origin position, determines that the item Mi has been selected (for example, S106, S108, S203, S205, S211, S221, and S232).

In the display data generation step, the program causes the information processing device 1 to perform a process of generating display data corresponding to the determination result in the determination step, that is, a process of generating display data for displaying an operation plane (for example, S106, S203, S211, S221, and S232 and the process of the display data generation unit 4).

The program makes it easy to achieve the information processing device 1 according to this embodiment.

Then, the program may be stored in a recording medium provided in a device, such as a computer device, a ROM of a microcomputer having a CPU, or the like in advance. Alternatively, the program may be temporarily or permanently stored in a removable recording medium, such as a semiconductor memory, a memory card, an optical disk, a magneto-optical disk, or a magnetic disk. In addition, the removable recording medium can be provided as so-called packaged software.

In addition, the program may be installed in, for example, a personal computer from the removable recording medium or may be downloaded from a download site through a network such as a LAN or the Internet.

Further, the effects described in the specification are illustrative examples and are not limited thereto. Other effects may be obtained.

Note that the present technology can have the following configurations.
(1) There is provided an information processing device including: an operation detection unit that calculates an operation direction and an operation distance from acquired operation information; a control unit that, in a case where a positional relationship between a region of an item to be selected and an operation position specified by the operation direction and the operation distance calculated by the operation detection unit using an origin position as a starting point is changed to a specific state in a state in which the regions of the items to be selected are arranged around the origin position, performs a process of determining that the item has been selected; and a display data generation unit that generates display data corresponding to a determination result of the control unit.
(2) In the information processing device according to (1), the operation detection unit detects non-contact operation information as the operation information and the control unit sets the origin position substantially at a center of an operation region in which the non-contact operation information is effective.
(3) In the information processing device according to (1) or (2), the display data generation unit generates display data for displaying the operation direction and the operation distance calculated by the operation detection unit on an operation plane.
(4) In the information processing device according to any one of (1) to (3), the control unit sets an operation plane in which a plurality of items to be selected are arranged around the origin position so as not to overlap each other as viewed from the origin position.
(5) In the information processing device according to (4), in a case where the control unit recognizes that a certain item has been selected by an operation position on the operation plane and a lower-layer item is present in the selected item, the control unit resets the operation plane such that the selected item is arranged at the origin position and the lower-layer items are arranged around the origin position so as not to overlap each other as viewed from the origin position. In a case where the operation position indicates a certain lower-layer item on the reset operation plane, the control unit performs a process of recognizing that the lower-layer item has been selected.
(6) In the information processing device according to (5), in a case where the operation plane is set or reset, the control unit arranges a plurality of items around the origin position along a virtual ring of a true circle having the origin position as a center.
(7) In the information processing device according to (5) or (6), in a case where the operation plane is reset, the control unit controls the display data generation unit such that display presenting movement of the selected item to the origin position is performed.
(8) In the information processing device according to any one of (5) to (7), in a case where the operation plane is reset, the control unit arranges the lower-layer items and an item for a layer return operation around the origin position so as not to overlap each other as viewed from the origin position.
(9) In the information processing device according to (8), in a case where the item for the layer return operation is selected, the control unit controls the display data generation unit such that display presenting movement of the item for the layer return operation to the origin position is performed. The control unit resets the operation plane such that upper-layer items are arranged around the origin position so as not to overlap each other as viewed from the origin position.
(10) In the information processing device according to any one of (1) to (3), the control unit sets, as an operation plane for performing an operation involving a plurality of stages for selection or designation of an item, an operation plane in which a plurality of items to be selected in an X-th stage are arranged on an X-th virtual ring-shaped line from the origin position so as not to overlap each other as viewed from the origin position. In a case where the control unit recognizes that a certain item among the plurality of items in the X-th stage has been selected by an operation position, the control unit sets an operation plane in which one or a plurality of items to be designated or selected according to the item selected in the X-th stage are arranged on an (X+1)-th virtual ring-shaped line from the origin position (where X is a natural number equal to or greater than 1).
(11) In the information processing device according to (10), in a case where the control unit recognizes that a certain item among the plurality of items in the X-th stage has been selected by the operation position, the control unit sets an operation plane in which a plurality of lower-layer items to be selected in a lower layer of the item selected in the X-th stage are arranged on the (X+1)-th virtual ring-shaped line from the origin position. In a case where the operation position indicates a lower-layer item, the control unit performs a process of recognizing that the lower-layer item has been selected.
(12) In the information processing device according to (10), in a case where the control unit recognizes that a certain item among the plurality of items in the X-th stage has been selected by the operation position, the control unit sets an operation plane in which an item for an enter operation for the item selected in the X-th stage is arranged on the (X+1)-th virtual ring-shaped line from the origin position. In a case where the operation position designates the item for the enter operation, the control unit performs a process of recognizing that the enter operation for the item selected in the X-th stage has been performed.
(13) In the information processing device according to (12), the control unit arranges the item for the enter operation at a position that is on the (X+1)-th virtual ring-shaped line and is adjacent to the item selected in the X-th stage.
(14) In the information processing device according to (10), the control unit sets the plurality of items to be selected in the X-th stage as items for a scroll operation. In a case where an item for the scroll operation is selected, the control unit performs a process of recognizing the scroll operation.
(15) In the information processing device according to (14), the control unit performs a process of recognizing a direction of the scroll operation according to an order in which a plurality of items for the scroll operation are selected.
(16) In the information processing device according to (15), the control unit arranges an item for an enter operation after scrolling on the (X+1)-th virtual ring-shaped line in the same angular range as an item that is being selected among the items for the scroll operation.
(17) In the information processing device according to any one of (10) to (13), in a case where the control unit recognizes that a certain item among the plurality of items in the X-th stage has been selected by the operation position, the control unit instructs the display data generation unit such that a display aspect of items which have not been selected among the plurality of items in the X-th stage is changed.
(18) In the information processing device according to any one of (10), (11), (12), (13), and (17), the control unit sets an operation plane in which one or a plurality of items to be designated or selected in an (X+1)-th stage according to the item selected in the X-th stage are arranged. In a case where selection of the selected item is canceled, the control unit sets the operation plane such that the items in the (X+1)-th stage are canceled.
(19) There is provided an information processing method performed by an information processing device. The information processing method includes: an operation detection procedure of calculating an operation direction and an operation distance from acquired operation information; a determination procedure of, in a case where a positional relationship between a region of an item to be selected and an operation position specified by the operation direction and the operation distance calculated in the operation detection procedure using an origin position as a starting point is changed to a specific state in a state in which the regions of the items to be selected are arranged around the origin position, determining that the item has been selected; and a display data generation procedure of generating display data corresponding to a determination result in the determination procedure.
(20) There is provided a program that causes an information processing device to perform: an operation detection step of calculating an operation direction and an operation distance from acquired operation information; a determination step of, in a case where a positional relationship between a region of an item to be selected and an operation position specified by the operation direction and the operation distance calculated in the operation detection step using an origin position as a starting point is changed to a specific state in a state in which the regions of the items to be selected are arranged around the origin position, determining that the item has been selected; and a display data generation step of generating display data corresponding to a determination result in the determination step.

### REFERENCE SIGNS LIST

- 1: Information processing device
- 2: Operation detection unit
- 3: Control unit
- 4: Display data generation unit
- 10: Tracking device
- 11: Display device
- 21: Tracking input unit
- 22: Vector calculation unit
- 23: Coordinate conversion unit
- 31: Menu control unit
- 32: Menu information storage unit
- 33: Operation information storage unit
- 50: Operator
- 60: Monitor screen list
- 61: Transmission source list
- AR: Operation region
- CR: Virtual ring
- CR1: First virtual ring
- CR2: Second virtual ring
- Mi: Item
- CTi: Origin item
- Di: Start operator
- Si: Start button
- Vc: Operation vector

## Claims

1. An information processing device comprising:
an operation detection unit that calculates an operation direction and an operation distance from acquired operation information;
a control unit that, in a case where a positional relationship between a region of an item to be selected and an operation position specified by the operation direction and the operation distance calculated by the operation detection unit using an origin position as a starting point is changed to a specific state in a state in which the regions of the items to be selected are arranged around the origin position, performs a process of determining that the item has been selected; and
a display data generation unit that generates display data corresponding to a determination result of the control unit.

2. The information processing device according to claim 1,
wherein the operation detection unit detects non-contact operation information as the operation information, and
the control unit sets the origin position substantially at a center of an operation region in which the non-contact operation information is effective.

3. The information processing device according to claim 1,
wherein the display data generation unit generates display data for displaying the operation direction and the operation distance calculated by the operation detection unit on an operation plane.

4. The information processing device according to claim 1,
wherein the control unit sets an operation plane in which a plurality of items to be selected are arranged around the origin position so as not to overlap each other as viewed from the origin position.

5. The information processing device according to claim 4,
wherein, in a case where the control unit recognizes that a certain item has been selected by an operation position on the operation plane and a lower-layer item is present in the selected item, the control unit resets the operation plane such that the selected item is arranged at the origin position and the lower-layer items are arranged around the origin position so as not to overlap each other as viewed from the origin position, and
in a case where the operation position indicates a certain lower-layer item on the reset operation plane, the control unit performs a process of recognizing that the lower-layer item has been selected.

6. The information processing device according to claim 5,
wherein, in a case where the operation plane is set or reset, the control unit arranges a plurality of items around the origin position along a virtual ring of a true circle having the origin position as a center.

7. The information processing device according to claim 5,
wherein, in a case where the operation plane is reset, the control unit controls the display data generation unit such that display presenting movement of the selected item to the origin position is performed.

8. The information processing device according to claim 5,
wherein, in a case where the operation plane is reset, the control unit arranges the lower-layer items and an item for a layer return operation around the origin position so as not to overlap each other as viewed from the origin position.

9. The information processing device according to claim 8,
wherein, in a case where the item for the layer return operation is selected, the control unit controls the display data generation unit such that display presenting movement of the item for the layer return operation to the origin position is performed, and
the control unit resets the operation plane such that upper-layer items are arranged around the origin position so as not to overlap each other as viewed from the origin position.

10. The information processing device according to claim 1,
wherein the control unit sets, as an operation plane for performing an operation involving a plurality of stages for selection or designation of an item, an operation plane in which a plurality of items to be selected in an X-th stage are arranged on an X-th virtual ring-shaped line from the origin position so as not to overlap each other as viewed from the origin position, and
in a case where the control unit recognizes that a certain item among the plurality of items in the X-th stage has been selected by an operation position, the control unit sets an operation plane in which one or a plurality of items to be designated or selected according to the item selected in the X-th stage are arranged on an (X+1)-th virtual ring-shaped line from the origin position (where X is a natural number equal to or greater than 1).

11. The information processing device according to claim 10,
wherein, in a case where the control unit recognizes that a certain item among the plurality of items in the X-th stage has been selected by the operation position, the control unit sets an operation plane in which a plurality of lower-layer items to be selected in a lower layer of the item selected in the X-th stage are arranged on the (X+1)-th virtual ring-shaped line from the origin position, and
in a case where the operation position indicates a lower-layer item, the control unit performs a process of recognizing that the lower-layer item has been selected.

12. The information processing device according to claim 10,
wherein, in a case where the control unit recognizes that a certain item among the plurality of items in the X-th stage has been selected by the operation position, the control unit sets an operation plane in which an item for an enter operation for the item selected in the X-th stage is arranged on the (X+1)-th virtual ring-shaped line from the origin position, and
in a case where the operation position designates the item for the enter operation, the control unit performs a process of recognizing that the enter operation for the item selected in the X-th stage has been performed.

13. The information processing device according to claim 12,
wherein the control unit arranges the item for the enter operation at a position that is on the (X+1)-th virtual ring-shaped line and is adjacent to the item selected in the X-th stage.

14. The information processing device according to claim 10,
wherein the control unit sets the plurality of items to be selected in the X-th stage as items for a scroll operation, and
in a case where an item for the scroll operation is selected, the control unit performs a process of recognizing the scroll operation.

15. The information processing device according to claim 14,
wherein the control unit performs a process of recognizing a direction of the scroll operation according to an order in which a plurality of items for the scroll operation are selected.

16. The information processing device according to claim 15,
wherein the control unit arranges an item for an enter operation after scrolling on the (X+1)-th virtual ring-shaped line in the same angular range as an item that is being selected among the items for the scroll operation.

17. The information processing device according to claim 10,
wherein, in a case where the control unit recognizes that a certain item among the plurality of items in the X-th stage has been selected by the operation position, the control unit instructs the display data generation unit such that a display aspect of items which have not been selected among the plurality of items in the X-th stage is changed.

18. The information processing device according to claim 10,
wherein the control unit sets an operation plane in which one or a plurality of items to be designated or selected in an (X+1)-th stage according to the item selected in the X-th stage are arranged, and
in a case where selection of the selected item is canceled, the control unit sets the operation plane such that the items in the (X+1)-th stage are canceled.

19. An information processing method performed by an information processing device, the method comprising:
an operation detection procedure of calculating an operation direction and an operation distance from acquired operation information;
a determination procedure of, in a case where a positional relationship between a region of an item to be selected and an operation position specified by the operation direction and the operation distance calculated in the operation detection procedure using an origin position as a starting point is changed to a specific state in a state in which the regions of the items to be selected are arranged around the origin position, determining that the item has been selected; and
a display data generation procedure of generating display data corresponding to a determination result in the determination procedure.

20. A program that causes an information processing device to perform:
an operation detection step of calculating an operation direction and an operation distance from acquired operation information;
a determination step of, in a case where a positional relationship between a region of an item to be selected and an operation position specified by the operation direction and the operation distance calculated in the operation detection step using an origin position as a starting point is changed to a specific state in a state in which the regions of the items to be selected are arranged around the origin position, determining that the item has been selected; and
a display data generation step of generating display data corresponding to a determination result in the determination step.
